(19) Europäisches Patentamt
European Patent Office
Office européen des brevets

(11) **EP 2 093 293 A2**

(12) **EUROPEAN PATENT APPLICATION**


(43) Date of publication:
**26.08.2009 Bulletin 2009/35**

(21) Application number: **09004715.0**

(22) Date of filing: **15.06.2001**

(51) Int Cl.:
***C12N 15/12*** *(2006.01)* ***C12N 5/10*** *(2006.01)*
***C07K 14/705*** *(2006.01)* ***C07K 16/28*** *(2006.01)*
***C12Q 1/68*** *(2006.01)* ***G01N 33/68*** *(2006.01)*
***A01K 67/027*** *(2006.01)*

(84) Designated Contracting States:
**AT BE CH CY DE DK ES FI FR GB GR IE IT LI LU MC NL PT SE TR**

(30) Priority:
**16.06.2000 US 212483 P**
**23.06.2000 US 213950 P**
**26.06.2000 US 214062 P**
**07.07.2000 US 216595 P**
**14.07.2000 US 218936 P**
**19.07.2000 US 219154 P**

(62) Document number(s) of the earlier application(s) in accordance with Art. 76 EPC:
**01944579.0 / 1 292 678**



(71) Applicant: **Incyte Corporation**
**Wilmington, DE 19880 (US)**

(72) Inventors:
- **Lal, Preeti**
  **Santa Clara,**
  **CA 95056 (US)**
- **Graul, Richard**
  **San Francisco,**
  **CA 94121 (US)**
- **Hafalia, April J. A.**
  **Santa Clara,**
  **CA 95054 (US)**
- **Walia, Narinder K.**
  **San Leandro,**
  **CA 94577 (US)**
- **Thornton, Michael**
  **Woodside, (US)**
- **Nguyen, Danniel B.**
  **San Jose,**
  **CA 95118 (US)**
- **Lu, Yan**
  **Palo Alto,**
  **CA 94303 (US)**
- **Gandhi, Ameena R.**
  **San Francisco, CA 94118 (US)**
- **Patterson, Chandra**
  **Menlo Park,**
  **CA 94025 (US)**
- **Kallilck, Deborah A.**
  **Portola Valley, CA 94028 (US)**
- **Baughn, Mariah R.**
  **San Leandro,**
  **CA 94577 (US)**
- **Ramkumar, Jayalaxmi**
  **Fremont,**
  **CA 94555 (US)**
- **Tribouley, Catherine M.**
  **San Francisco,**
  **CA 94107 (US)**
- **Lee, Ernestine A.**
  **Castra Valley, CA 94552 (US)**
- **Ding, Li**
  **Palo Alto,**
  **CA 94306 (US)**
- **Burford, Neil**
  **Durham,**
  **CT 06422 (US)**
- **Yao, Monique G.**
  **Mountain View,**
  **CA 94043 (US)**
- **Yang, Junming**
  **San Jose,**
  **CA 95129 (US)**
- **Griffin, Jennifer A.**
  **Fremont,**
  **CA 94555 (US)**

(74) Representative: **Wright, Simon Mark**
**J.A. Kemp & Co.**
**14 South Square**
**Gray's Inn**
**London WC1R 5JJ (GB)**


Remarks:
This application was filed on 31-03-2009 as a divisional application to the application mentioned under INID code 62.

EP 2 093 293 A2

(54) **G-Protein coupled receptors**

(57) The invention provides human G-protein coupled receptors (GCREC) and polynucleotides which identify and encode GCREC. The invention also provides expression vectors, host cells, antibodies, agonists, and antagonists. The invention also provides methods for diagnosing, treating, or preventing disorders associated with aberrant expression of GCREC.

## Description

## TECHNICAL FIELD

This invention relates to nucleic acid and amino acid sequences of G-protein coupled receptors and to the use of these sequences in the diagnosis, treatment, and prevention of cell proliferative, neurological, cardiovascular, gastrointestinal, autoimmune/inflammatory, and metabolic disorders, and viral infections, and in the assessment of the effects of exogenous compounds on the expression of nucleic acid and amino acid sequences of G-protein coupled receptors.

## BACKGROUND OF THE INVENTION

Signal transduction is the general process by which cells respond to extracellular signals. Signal transduction across the plasma membrane begins with the binding of a signal molecule, e.g., a hormone, neurotransmitter, or growth factor, to a cell membrane receptor. The receptor, thus activated, triggers an intracellular biochemical cascade that ends with the activation of an intracellular target molecule, such as a transcription factor. This process of signal transduction regulates all types of cell functions including cell proliferation, differentiation, and gene transcription. The G-protein coupled receptors (GPCRs), encoded by one of the largest families of genes yet identified, play a central role in the transduction of extracellular signals across the plasma membrane. GPCRs have a proven history of being successful therapeutic targets.

GPCRs are integral membrane proteins characterized by the presence of seven hydrophobic transmembrane domains which together form a bundle of antiparallel alpha ($\alpha$) helices. GPCRs range in size from under 400 to over 1000 amino acids (Strosberg, A.D. (1991) Eur. J. Biochem. 196:1-10; Coughlin, S.R. (1994) Curr. Opin. Cell Biol. 6: 191-197). The amino-terminus of a GPCR is extracellular, is of variable length, and is often glycosylated. The carboxy-terminus is cytoplasmic and generally phosphorylated. Extracellular loops alternate with intracellular loops and link the transmembrane domains. Cysteine disulfide bridges linking the second and third extracellular loops may interact with agonists and antagonists. The most conserved domains of GPCRs are the transmembrane domains and the first two cytoplasmic loops. The transmembrane domains account, in part, for structural and functional features of the receptor. In most cases, the bundle of $\alpha$ helices forms a ligand-binding pocket. The extracellular N-terminal segment, or one or more of the three extracellular loops, may also participate in ligand binding. Ligand binding activates the receptor by inducing a conformational change in intracellular portions of the receptor. In turn, the large, third intracellular loop of the activated receptor interacts with a heterotrimeric guanine nucleotide binding (G) protein complex which mediates further intracellular signaling activities, including the activation of second messengers such as cyclic AMP (cAMP), phospholipase C, and inositol triphosphate, and the interaction of the activated GPCR with ion channel proteins. (See, e.g., Watson, S. and S. Arkinstall (1994) The G-protein Linked Receptor Facts Book, Academic Press, San Diego CA, pp. 2-6; Bolander, F.F. (1994) Molecular Endocrinology, Academic Press, San Diego CA, pp. 162-176; Baldwin, J.M. (1994) Curr. Opin. Cell Biol. 6:180-190.)

GPCRs include receptors for sensory signal mediators (e.g., light and olfactory stimulatory molecules); adenosine, $\gamma$-aminobutyric acid (GABA), hepatocyte growth factor, melanocortins, neuropeptide Y, opioid peptides, opsins, somatostatin, tachykinins, vasoactive intestinal polypeptide family, and vasopressin; biogenic amines (e.g., dopamine, epinephrine and norepinephrine, histamine, glutamate (metabotropic effect), acetylcholine (muscarinic effect), and serotonin); chemokines; lipid mediators of inflammation (e.g., prostaglandins and prostanoids, platelet activating factor, and leukotrienes); and peptide hormones (e.g., bombesin, bradykinin, calcitonin, C5a anaphylatoxin, endothelin, follicle-stimulating hormone (FSH), gonadotropic-releasing hormone (GnRH), neurokinin, and thyrotropin-releasing hormone (TRH), and oxytocin). GPCRs which act as receptors for stimuli that have yet to be identified are known as orphan receptors.

The diversity of the GPCR family is further increased by alternative splicing. Many GPCR genes contain introns, and there are currently over 30 such receptors for which splice variants have been identified. The largest number of variations are at the protein C-terminus. N-terminal and cytoplasmic loop variants are also frequent, while variants in the extracellular loops or transmembrane domains are less common. Some receptors have more than one site at which variance can occur. The splicing variants appear to be functionally distinct, based upon observed differences in distribution, signaling, coupling, regulation, and ligand binding profiles (Kilpatrick, G.J. et al. (1999) Trends Pharmacol. Sci. 20:294-301).

GPCRs can be divided into three major subfamilies: the rhodopsin-like, secretin-like, and metabotropic glutamate receptor subfamilies. Members of these GPCR subfamilies share similar functions and the characteristic seven transmembrane structure, but have divergent amino acid sequences. The largest family consists of the rhodopsin-like GPCRs, which transmit diverse extracellular signals including hormones, neurotransmitters, and light. Rhodopsin is a photosensitive GPCR found in animal retinas. In vertebrates, rhodopsin molecules are embedded in membranous stacks found in photoreceptor (rod) cells. Each rhodopsin molecule responds to a photon of light by triggering a decrease in cGMP levels which leads to the closure of plasma membrane sodium channels. In this manner, a visual signal is converted to a neural impulse. Other rhodopsin-like GPCRs are directly involved in responding to neurotransmitters. These GPCRs include the receptors for adrenaline (adrenergic receptors), acetylcholine (muscarinic receptors), adenosine, galanin, and glutamate (N-methyl-D-aspartate/NMDA receptors). (Reviewed in Watson, S. and S. Arkinstall (1994) The G-Protein Linked Receptor Facts Book, Academic Press, San Diego CA, pp. 7-9, 19-22, 32-35, 130-131, 214-216, 221-222; Habert-Ortoli, E. et al. (1994) Proc. Natl. Acad. Sci. USA 91:9780-9783.)

The galanin receptors mediate the activity of the neuroendocrine peptide galanin, which inhibits secretion of insulin, acetylcholine, serotonin and noradrenaline, and stimulates prolactin and growth hormone release. Galanin receptors are involved in feeding disorders, pain, depression, and Alzheimer's disease (Kask, K. et al. (1997) Life Sci. 60: 1523-1533). Other nervous system rhodopsin-like GPCRs include a growing family of receptors for lysophosphatidic acid and other lysophospholipids, which appear to have roles in development and neuropathology (Chun, J. et al. (1999) Cell Biochem. Biophys. 30:213-242).

The largest subfamily of GPCRs, the olfactory receptors, are also members of the rhodopsin-like GPCR family. These receptors function by transducing odorant signals. Numerous distinct olfactory receptors are required to distinguish different odors. Each olfactory sensory neuron expresses only one type of olfactory receptor, and distinct spatial zones of neurons expressing distinct receptors are found in nasal passages. For example, the RA1c receptor which was isolated from a rat brain library, has been shown to be limited in expression to very distinct regions of the brain and a defined zone of the olfactory epithelium (Raming, K. et al. (1998) Receptors Channels 6:141-151). However, the expression of olfactory-like receptors is not confined to olfactory tissues. For example, three rat genes encoding olfactory-like receptors having typical GPCR characteristics showed expression patterns not only in taste and olfactory tissue, but also in male reproductive tissue (Thomas, M.B. et al. (1996) Gene 178:1-5).

Members of the secretin-like GPCR subfamily have as their ligands peptide hormones such as secretin, calcitonin, glucagon, growth hormone-releasing hormone, parathyroid hormone, and vasoactive intestinal peptide. For example, the secretin receptor responds to secretin, a peptide hormone that stimulates the secretion of enzymes and ions in the pancreas and small intestine (Watson, supra, pp. 278-283). Secretin receptors are about 450 amino acids in length and are found in the plasma membrane of gastrointestinal cells. Binding of secretin to its receptor stimulates the production of cAMP.

Examples of secretin-like GPCRs implicated in inflammation and the immune response include the EGF module-containing, mucin-like hormone receptor (Emr1) and CD97 receptor proteins. These GPCRs are members of the recently characterized EGF-TM7 receptors subfamily. These seven transmembrane hormone receptors exist as heterodimers in vivo and contain between three and seven potential calcium-binding EGF-like motifs. CD97 is predominantly expressed in leukocytes and is markedly upregulated on activated B and T cells (McKnight, A.J. and S. Gordon (1998) J. Leukoc. Biol. 63:271-280).

The third GPCR subfamily is the metabotropic glutamate receptor family. Glutamate is the major excitatory neurotransmitter in the central nervous system. The metabotropic glutamate receptors modulate the activity of intracellular effectors, and are involved in long-term potentiation (Watson, supra, p.130). The $Ca^{2+}$-sensing receptor, which senses changes in the extracellular concentration of calcium ions, has a large extracellular domain including clusters of acidic amino acids which may be involved in calcium binding. The metabotropic glutamate receptor family also includes pheromone receptors, the $GABA_B$ receptors, and the taste receptors.

Other subfamilies of GPCRs include two groups of chemoreceptor genes found in the nematodes Caenorhabditis elegans and Caenorhabditis briggsae, which are distantly related to the mammalian olfactory receptor genes. The yeast pheromone receptors STE2 and STE3, involved in the response to mating factors on the cell membrane, have their own seven-transmembrane signature, as do the cAMP receptors from the slime mold Dictyostelium discoideum, which are thought to regulate the aggregation of individual cells and control the expression of numerous developmentally-regulated genes.

GPCR mutations, which may cause loss of function or constitutive activation, have been associated with numerous human diseases (Coughlin, supra). For instance, retinitis pigmentosa may arise from mutations in the rhodopsin gene. Furthermore, somatic activating mutations in the thyrotropin receptor have been reported to cause hyperfunctioning thyroid adenomas, suggesting that certain GPCRs susceptible to constitutive activation may behave as protooncogenes (Parma, J. et al. (1993) Nature 365:649-651). GPCR receptors for the following ligands also contain mutations associated with human disease: luteinizing hormone (precocious puberty); vasopressin $V_2$ (X-linked nephrogenic diabetes); glucagon (diabetes and hypertension); calcium (hyperparathyroidism, hypocalcuria, hypercalcemia); parathyroid hormone (short limbed dwarfism); $\beta_3$-adrenoceptor (obesity, non-insulin-dependent diabetes mellitus); growth hormone releasing hormone (dwarfism); and adrenocorticotropin (glucocorticoid deficiency) (Wilson, S. et al. (1998) Br. J. Pharmocol. 125: 1387-1392; Stadel, J.M. et al. (1997) Trends Pharmacol. Sci. 18:430-437). GPCRs are also involved in depression, schizophrenia, sleeplessness, hypertension, anxiety, stress, renal failure, and several cardiovascular disorders (Horn, F. and G. Vriend (1998) J. Mol. Med. 76:464-468).

In addition, within the past 20 years several hundred new drugs have been recognized that are directed towards activating or inhibiting GPCRs. The therapeutic targets of these drugs span a wide range of diseases and disorders, including cardiovascular, gastrointestinal, and central nervous system disorders as well as cancer, osteoporosis and endometriosis (Wilson, supra; Stadel, supra). For example, the dopamine agonist L-dopa is used to treat Parkinson's disease, while a dopamine antagonist is used to treat schizophrenia and the early stages of Huntington's disease. Agonists and antagonists of adrenoceptors have been used for the treatment of asthma, high blood pressure, other cardiovascular disorders, and anxiety; muscarinic agonists are used in the treatment of glaucoma and tachycardia; serotonin 5HT1D antagonists are used against migraine; and histamine H1 antagonists are used against allergic and anaphylactic reactions, hay fever, itching, and motion sickness (Horn, supra).

Recent research suggests potential future therapeutic uses for GPCRs in the treatment of metabolic disorders including diabetes, obesity, and osteoporosis. For example, mutant V2 vasopressin receptors causing nephrogenic diabetes could be functionally rescued in vitro by co-expression of a C-terminal V2 receptor peptide spanning the region containing the mutations. This result suggests a possible novel strategy for disease treatment (Schöneberg, T. et al. (1996) EMBO J. 15:1283-1291). Mutations in melanocortin-4 receptor (MC4R) are implicated in human weight regulation and obesity. As with the vasopressin V2 receptor mutants, these MC4R mutants are defective in trafficking to the plasma membrane (Ho, G. and R.G. MacKenzie (1999) J. Biol. Chem. 274:35816-35822), and thus might be treated with a similar strategy. The type 1 receptor for parathyroid hormone (PTH) is a GPCR that mediates the PTH-dependent regulation of calcium homeostasis in the bloodstream. Study of PTH/receptor interactions may enable the development of novel PTH receptor ligands for the treatment of osteoporosis (Mannstadt, M. et al. (1999) Am. J. Physiol. 277:F665-F675).

The chemokine receptor group of GPCRs have potential therapeutic utility in inflammation and infectious disease. (For review, see Locati, M. and P.M. Murphy (1999) Annu. Rev. Med. 50:425-440.) Chemokines are small polypeptides that act as intracellular signals in the regulation of leukocyte trafficking, hematopoiesis, and angiogenesis. Targeted disruption of various chemokine receptors in mice indicates that these receptors play roles in pathologic inflammation and in autoimmune disorders such as multiple sclerosis. Chemokine receptors are also exploited by infectious agents, including herpesviruses and the human immunodeficiency virus (HIV-1) to facilitate infection. A truncated version of chemokine receptor CCR5, which acts as a coreceptor for infection of T-cells by HIV-1, results in resistance to AIDS, suggesting that CCR5 antagonists could be useful in preventing the development of AIDS.

The discovery of new G-protein coupled receptors and the polynucleotides encoding them satisfies a need in the art by providing new compositions which are useful in the diagnosis, prevention, and treatment of cell proliferative, neurological, cardiovascular, gastrointestinal, autoimmune/inflammatory, and metabolic disorders, and viral infections, and in the assessment of the effects of exogenous compounds on the expression of nucleic acid and amino acid sequences of G-protein coupled receptors.

## SUMMARY OF THE INVENTION

The invention features purified polypeptides, G-protein coupled receptors, referred to collectively as "GCREC" and individually as "GCREC-1," "GCREC-2," "GCREC-3," "GCREC-4," "GCREC-5," "GCREC-6," "GCREC-7," "GCREC-8," "GCREC-9," and "GCREC-10." In one aspect, the invention provides an isolated polypeptide selected from the group consisting of a) a polypeptide comprising an amino acid sequence selected from the group consisting of SEQ ID NO: 1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. In one alternative, the invention provides an isolated polypeptide comprising the amino acid sequence of SEQ ID NO:1-10.

The invention further provides an isolated polynucleotide encoding a polypeptide selected from the group consisting of a) a polypeptide comprising an amino acid sequence selected from the group consisting of SEQ ID NO: 1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. In one alternative, the polynucleotide encodes a polypeptide selected from the group consisting of SEQ ID NO:1-10. In another alternative, the polynucleotide is selected from the group consisting of SEQ ID NO:11-20.

Additionally, the invention provides a recombinant polynucleotide comprising a promoter sequence operably linked to a polynucleotide encoding a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. In one alternative, the invention provides a cell transformed with the recombinant polynucleotide. In another alternative, the invention provides a transgenic organism comprising the recombinant polynucleotide.

The invention also provides a method for producing a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. The method comprises a) culturing a cell under conditions suitable for expression of the polypeptide, wherein said cell is transformed with a recombinant polynucleotide comprising a promoter sequence operably linked to a polynucleotide encoding the polypeptide, and b) recovering the polypeptide so expressed.

Additionally, the invention provides an isolated antibody which specifically binds to a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10.

The invention further provides an isolated polynucleotide selected from the group consisting of a) a polynucleotide comprising a polynucleotide sequence selected from the group consisting of SEQ ID NO:11-20, b) a polynucleotide comprising a naturally occurring polynucleotide sequence at least 90% identical to a polynucleotide sequence selected from the group consisting of SEQ ID NO:11-20, c) a polynucleotide complementary to the polynucleotide of a), d) a polynucleotide complementary to the polynucleotide of b), and e) an RNA equivalent of a)-d). In one alternative, the polynucleotide comprises at least 60 contiguous nucleotides.

Additionally, the invention provides a method for detecting a target polynucleotide in a sample, said target polynucleotide having a sequence of a polynucleotide selected from the group consisting of a) a polynucleotide comprising a polynucleotide sequence selected from the group consisting of SEQ ID NO:11-20, b) a polynucleotide comprising a naturally occurring polynucleotide sequence at least 90% identical to a polynucleotide sequence selected from the group consisting of SEQ ID NO:11-20, c) a polynucleotide complementary to the polynucleotide of a), d) a polynucleotide complementary to the polynucleotide of b), and e) an RNA equivalent of a)-d). The method comprises a) hybridizing the sample with a probe comprising at least 20 contiguous nucleotides comprising a sequence complementary to said target polynucleotide in the sample, and which probe specifically hybridizes to said target polynucleotide, under conditions whereby a hybridization complex is formed between said probe and said target polynucleotide or fragments thereof, and b) detecting the presence or absence of said hybridization complex, and optionally, if present, the amount thereof. In one alternative, the probe comprises at least 60 contiguous nucleotides.

The invention further provides a method for detecting a target polynucleotide in a sample, said target polynucleotide having a sequence of a polynucleotide selected from the group consisting of a) a polynucleotide comprising a polynucleotide sequence selected from the group consisting of SEQ ID NO:11-20, b) a polynucleotide comprising a naturally occurring polynucleotide sequence at least 90% identical to a polynucleotide sequence selected from the group consisting of SEQ ID NO:11-20, c) a polynucleotide complementary to the polynucleotide of a), d) a polynucleotide complementary to the polynucleotide of b), and e) an RNA equivalent of a)-d). The method comprises a) amplifying said target polynucleotide or fragment thereof using polymerase chain reaction amplification, and b) detecting the presence or absence of said amplified target polynucleotide or fragment thereof, and, optionally, if present, the amount thereof.

The invention further provides a composition comprising an effective amount of a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10, and a pharmaceutically acceptable excipient. In one embodiment, the composition comprises an amino acid sequence selected from the group consisting of SEQ ID NO:1-10. The invention additionally provides a method of treating a disease or condition associated with decreased expression of functional GCREC, comprising administering to a patient in need of such treatment the composition.

The invention also provides a method for screening a compound for effectiveness as an agonist of a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. The method comprises a) exposing a sample comprising the polypeptide to a compound, and b) detecting agonist activity in the sample. In one alternative, the invention provides a composition comprising an agonist compound identified by the method and a pharmaceutically acceptable excipient. In another alternative, the invention provides a method of treating a disease or condition associated with decreased expression of functional GCREC, comprising administering to a patient in need of such treatment the composition.

Additionally, the invention provides a method for screening a compound for effectiveness as an antagonist of a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. The method comprises a) exposing a sample comprising the polypeptide to a compound, and b) detecting antagonist activity in the sample. In one alternative, the invention provides a composition comprising an antagonist compound identified by the method and a pharmaceutically acceptable excipient. In another alternative, the invention provides a method of treating a disease or condition associated with overexpression of functional GCREC, comprising administering to a patient in need of such treatment the composition.

The invention further provides a method of screening for a compound that specifically binds to a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. The method comprises a) combining the polypeptide with at least one test compound under suitable conditions, and b) detecting binding of the polypeptide to the test compound, thereby identifying a compound that specifically binds to the polypeptide.

The invention further provides a method of screening for a compound that modulates the activity of a polypeptide selected from the group consisting of a) a polypeptide comprising an amino acid sequence selected from the group consisting of SEQ ID NO:1-10, b) a polypeptide comprising a naturally occurring amino acid sequence at least 90% identical to an amino acid sequence selected from the group consisting of SEQ ID NO:1-10, c) a biologically active fragment of a polypeptide having an amino acid sequence selected from the group consisting of SEQ ID NO:1-10, and d) an immunogenic fragment of a polypeptide having an amino acid sequence selected from the group consisting of SEQ ID NO:1-10. The method comprises a) combining the polypeptide with at least one test compound under conditions permissive for the activity of the polypeptide, b) assessing the activity of the polypeptide in the presence of the test compound, and c) comparing the activity of the polypeptide in the presence of the test compound with the activity of the polypeptide in the absence of the test compound, wherein a change in the activity of the polypeptide in the presence of the test compound is indicative of a compound that modulates the activity of the polypeptide.

The invention further provides a method for screening a compound for effectiveness in altering expression of a target polynucleotide, wherein said target polynucleotide comprises a sequence selected from the group consisting of SEQ ID NO:11-20, the method comprising a) exposing a sample comprising the target polynucleotide to a compound, and b) detecting altered expression of the target polynucleotide.

The invention further provides a method for assessing toxicity of a test compound, said method comprising a) treating a biological sample containing nucleic acids with the test compound; b) hybridizing the nucleic acids of the treated biological sample with a probe comprising at least 20 contiguous nucleotides of a polynucleotide selected from the group consisting of i) a polynucleotide comprising a polynucleotide sequence selected from the group consisting of SEQ ID NO:11-20, ii) a polynucleotide comprising a naturally occurring polynucleotide sequence at least 90% identical to a polynucleotide sequence selected from the group consisting of SEQ ID NO:11-20, iii) a polynucleotide having a sequence complementary to i), iv) a polynucleotide complementary to the polynucleotide of ii), and v) an RNA equivalent of i)-iv). Hybridization occurs under conditions whereby a specific hybridization complex is formed between said probe and a target polynucleotide in the biological sample, said target polynucleotide selected from the group consisting of i) a polynucleotide comprising a polynucleotide sequence selected from the group consisting of SEQ ID NO:11-20, ii) a polynucleotide comprising a naturally occurring polynucleotide sequence at least 90% identical to a polynucleotide sequence selected from the group consisting of SEQ ID NO:11-20, iii) a polynucleotide complementary to the polynucleotide of i), iv) a polynucleotide complementary to the polynucleotide of ii), and v) an RNA equivalent of i)-iv). Alternatively, the target polynucleotide comprises a fragment of a polynucleotide sequence selected from the group consisting of i)-v) above; c) quantifying the amount of hybridization complex; and d) comparing the amount of hybridization complex in the treated biological sample with the amount of hybridization complex in an untreated biological sample, wherein a difference in the amount of hybridization complex in the treated biological sample is indicative of toxicity of the test compound.

## BRIEF DESCRIPTION OF THE TABLES

Table 1 summarizes the nomenclature for the full length polynucleotide and polypeptide sequences of the present invention.

Table 2 shows the GenBank identification number and annotation of the nearest GenBank homolog for polypeptides of the invention. The probability score for the match between each polypeptide and its GenBank homolog is also shown.

Table 3 shows structural features of polypeptide sequences of the invention, including predicted motifs and domains, along with the methods, algorithms, and searchable databases used for analysis of the polypeptides.

Table 4 lists the cDNA and/or genomic DNA fragments which were used to assemble polynucleotide sequences of the invention, along with selected fragments of the polynucleotide sequences.

Table 5 shows the representative cDNA library for polynucleotides of the invention.

Table 6 provides an appendix which describes the tissues and vectors used for construction of the cDNA libraries shown in Table 5.

Table 7 shows the tools, programs, and algorithms used to analyze the polynucleotides and polypeptides of the invention, along with applicable descriptions, references, and threshold parameters.

Table 8 shows tissue-specific expression of polynucleotides of the invention.

## DESCRIPTION OF THE INVENTION

Before the present proteins, nucleotide sequences, and methods are described, it is understood that this invention is not limited to the particular machines, materials and methods described, as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "an," and "the" include plural reference unless the context clearly dictates otherwise. Thus, for example, a reference to "a host cell" includes a plurality of such host cells, and a reference to "an antibody" is a reference to one or more antibodies and equivalents thereof known to those skilled in the art, and so forth.

Unless defined otherwise, all technical and scientific terms used herein have the same meanings as commonly understood by one of ordinary skill in the art to which this invention belongs. Although any machines, materials, and methods similar or equivalent to those described herein can be used to practice or test the present invention, the preferred machines, materials and methods are now described. All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

## DEFINITIONS

"GCREC" refers to the amino acid sequences of substantially purified GCREC obtained from any species, particularly a mammalian species, including bovine, ovine, porcine, murine, equine, and human, and from any source, whether natural, synthetic, semi-synthetic, or recombinant.

The term "agonist" refers to a molecule which intensifies or mimics the biological activity of GCREC. Agonists may include proteins, nucleic acids, carbohydrates, small molecules, or any other compound or composition which modulates the activity of GCREC either by directly interacting with GCREC or by acting on components of the biological pathway in which GCREC participates.

An "allelic variant" is an alternative form of the gene encoding GCREC. Allelic variants may result from at least one mutation in the nucleic acid sequence and may result in altered mRNAs or in polypeptides whose structure or function may or may not be altered. A gene may have none, one, or many allelic variants of its naturally occurring form. Common mutational changes which give rise to allelic variants are generally ascribed to natural deletions, additions, or substitutions of nucleotides. Each of these types of changes may occur alone, or in combination with the others, one or more times in a given sequence.

"Altered" nucleic acid sequences encoding GCREC include those sequences with deletions, insertions, or substitutions of different nucleotides, resulting in a polypeptide the same as GCREC or a polypeptide with at least one functional characteristic of GCREC. Included within this definition are polymorphisms which may or may not be readily detectable using a particular oligonucleotide probe of the polynucleotide encoding GCREC, and improper or unexpected hybridization to allelic variants, with a locus other than the normal chromosomal locus for the polynucleotide sequence encoding GCREC. The encoded protein may also be "altered," and may contain deletions, insertions, or substitutions of amino acid residues which produce a silent change and result in a functionally equivalent GCREC. Deliberate amino acid substitutions may be made on the basis of similarity in polarity, charge, solubility, hydrophobicity, hydrophilicity, and/or the amphipathic nature of the residues, as long as the biological or immunological activity of GCREC is retained. For example, negatively charged amino acids may include aspartic acid and glutamic acid, and positively charged amino acids may include lysine and arginine. Amino acids with uncharged polar side chains having similar hydrophilicity values may include: asparagine and glutamine; and serine and threonine. Amino acids with uncharged side chains having similar hydrophilicity values may include: leucine, isoleucine, and valine; glycine and alanine; and phenylalanine and tyrosine.

The terms "amino acid" and "amino acid sequence" refer to an oligopeptide, peptide, polypeptide, or protein sequence, or a fragment of any of these, and to naturally occurring or synthetic molecules. Where "amino acid sequence" is recited to refer to a sequence of a naturally occurring protein molecule, "amino acid sequence" and like terms are not meant to limit the amino acid sequence to the complete native amino acid sequence associated with the recited protein molecule.

"Amplification" relates to the production of additional copies of a nucleic acid sequence. Amplification is generally carried out using polymerase chain reaction (PCR) technologies well known in the art.

The term "antagonist" refers to a molecule which inhibits or attenuates the biological activity of GCREC. Antagonists may include proteins such as antibodies, nucleic acids, carbohydrates, small molecules, or any other compound or composition which modulates the activity of GCREC either by directly interacting with GCREC or by acting on components of the biological pathway in which GCREC participates.

The term "antibody" refers to intact immunoglobulin molecules as well as to fragments thereof, such as Fab, $F(ab')_2$, and Fv fragments, which are capable of binding an epitopic determinant. Antibodies that bind GCREC polypeptides can be prepared using intact polypeptides or using fragments containing small peptides of interest as the immunizing antigen. The polypeptide or oligopeptide used to immunize an animal (e.g., a mouse, a rat, or a rabbit) can be derived from the translation of RNA, or synthesized chemically, and can be conjugated to a carrier protein if desired. Commonly used carriers that are chemically coupled to peptides include bovine serum albumin, thyroglobulin, and keyhole limpet hemocyanin (KLH). The coupled peptide is then used to immunize the animal.

The term "antigenic determinant" refers to that region of a molecule (i.e., an epitope) that makes contact with a particular antibody. When a protein or a fragment of a protein is used to immunize a host animal, numerous regions of the protein may induce the production of antibodies which bind specifically to antigenic determinants (particular regions or three-dimensional structures on the protein). An antigenic determinant may compete with the intact antigen (i.e., the immunogen used to elicit the immune response) for binding to an antibody.

The term "antisense" refers to any composition capable of base-pairing with the "sense" (coding) strand of a specific nucleic acid sequence. Antisense compositions may include DNA; RNA; peptide nucleic acid (PNA); oligonucleotides having modified backbone linkages such as phosphorothioates, methylphosphonates, or benzylphosphonates; oligonucleotides having modified sugar groups such as 2'-methoxyethyl sugars or 2'-methoxyethoxy sugars; or oligonucleotides having modified bases such as 5-methyl cytosine, 2'-deoxyuracil, or 7-deaza-2'-deoxyguanosine. Antisense molecules may be produced by any method including chemical synthesis or transcription. Once introduced into a cell, the complementary antisense molecule base-pairs with a naturally occurring nucleic acid sequence produced by the cell to form duplexes which block either transcription or translation. The designation "negative" or "minus" can refer to the antisense strand, and the designation "positive" or "plus" can refer to the sense strand of a reference DNA molecule.

The term "biologically active" refers to a protein having structural, regulatory, or biochemical functions of a naturally occurring molecule. Likewise, "immunologically active" or "immunogenic" refers to the capability of the natural, recombinant, or synthetic GCREC, or of any oligopeptide thereof, to induce a specific immune response in appropriate animals or cells and to bind with specific antibodies.

"Complementary" describes the relationship between two single-stranded nucleic acid sequences that anneal by base-pairing. For example, 5'-AGT-3' pairs with its complement, 3'-TCA-5'.

A "composition comprising a given polynucleotide sequence" and a "composition comprising a given amino acid sequence" refer broadly to any composition containing the given polynucleotide or amino acid sequence. The composition may comprise a dry formulation or an aqueous solution. Compositions comprising polynucleotide sequences encoding GCREC or fragments of GCREC may be employed as hybridization probes. The probes may be stored in freeze-dried form and may be associated with a stabilizing agent such as a carbohydrate. In hybridizations, the probe may be deployed in an aqueous solution containing salts (e.g., NaCl), detergents (e.g., sodium dodecyl sulfate; SDS), and other components (e.g., Denhardt's solution, dry milk, salmon sperm DNA, etc.).

"Consensus sequence" refers to a nucleic acid sequence which has been subjected to repeated DNA sequence analysis to resolve uncalled bases, extended using the XL-PCR kit (Applied Biosystems, Foster City CA) in the 5' and/or the 3' direction, and resequenced, or which has been assembled from one or more overlapping cDNA, EST, or genomic DNA fragments using a computer program for fragment assembly, such as the GELVIEW fragment assembly system (GCG, Madison WI) or Phrap (University of Washington, Seattle WA). Some sequences have been both extended and assembled to produce the consensus sequence.

"Conservative amino acid substitutions" are those substitutions that are predicted to least interfere with the properties of the original protein, i.e., the structure and especially the function of the protein is conserved and not significantly changed by such substitutions. The table below shows amino acids which may be substituted for an original amino acid in a protein and which are regarded as conservative amino acid substitutions.

| Original Residue | Conservative Substitution |
|---|---|
| Ala | Gly, Ser |
| Arg | His, Lys |
| Asn | Asp, Gln, His |
| Asp | Asn, Glu |
| Cys | Ala, Ser |
| Gln | Asn, Glu, His |
| Glu | Asp, Gln, His |
| Gly | Ala |
| His | Asn, Arg, Gln, Glu |
| Ile | Leu, Val |
| Leu | Ile, Val |
| Lys | Arg, Gln, Glu |
| Met | Leu, Ile |
| Phe | His, Met, Leu, Trp, Tyr |
| Ser | Cys, Thr |
| Thr | Ser, Val |
| Trp | Phe, Tyr |
| Tyr | His, Phe, Trp |
| Val | Ile, Leu, Thr |

Conservative amino acid substitutions generally maintain (a) the structure of the polypeptide backbone in the area of the substitution, for example, as a beta sheet or alpha helical conformation, (b) the charge or hydrophobicity of the molecule at the site of the substitution, and/or (c) the bulk of the side chain.

A "deletion" refers to a change in the amino acid or nucleotide sequence that results in the absence of one or more amino acid residues or nucleotides.

The term "derivative" refers to a chemically modified polynucleotide or polypeptide. Chemical modifications of a polynucleotide can include, for example, replacement of hydrogen by an alkyl, acyl, hydroxyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

A "detectable label" refers to a reporter molecule or enzyme that is capable of generating a measurable signal and is covalently or noncovalently joined to a polynucleotide or polypeptide.

"Differential expression" refers to increased or upregulated; or decreased, downregulated, or absent gene or protein expression, determined by comparing at least two different samples. Such comparisons may be carried out between, for example, a treated and an untreated sample, or a diseased and a normal sample.

A "fragment" is a unique portion of GCREC or the polynucleotide encoding GCREC which is identical in sequence to but shorter in length than the parent sequence. A fragment may comprise up to the entire length of the defined sequence, minus one nucleotide/amino acid residue. For example, a fragment may comprise from 5 to 1000 contiguous nucleotides or amino acid residues. A fragment used as a probe, primer, antigen, therapeutic molecule, or for other purposes, may be at least 5, 10, 15, 16, 20, 25, 30, 40, 50, 60, 75, 100, 150, 250 or at least 500 contiguous nucleotides or amino acid residues in length. Fragments may be preferentially selected from certain regions of a molecule. For example, a polypeptide fragment may comprise a certain length of contiguous amino acids selected from the first 250 or 500 amino acids (or first 25% or 50%) of a polypeptide as shown in a certain defined sequence. Clearly these lengths are exemplary, and any length that is supported by the specification, including the Sequence Listing, tables, and figures, may be encompassed by the present embodiments.

A fragment of SEQ ID NO:11-20 comprises a region of unique polynucleotide sequence that specifically identifies SEQ ID NO:11-20, for example, as distinct from any other sequence in the genome from which the fragment was obtained. A fragment of SEQ ID NO:11-20 is useful, for example, in hybridization and amplification technologies and in analogous methods that distinguish SEQ ID NO:11-20 from related polynucleotide sequences. The precise length of a fragment of SEQ ID NO:11-20 and the region of SEQ ID NO:11-20 to which the fragment corresponds are routinely determinable by one of ordinary skill in the art based on the intended purpose for the fragment.

A fragment of SEQ ID NO:1-10 is encoded by a fragment of SEQ ID NO:11-20. A fragment of SEQ ID NO:1-10 comprises a region of unique amino acid sequence that specifically identifies SEQ ID NO:1-10. For example, a fragment of SEQ ID NO:1-10 is useful as an immunogenic peptide for the development of antibodies that specifically recognize SEQ ID NO:1-10. The precise length of a fragment of SEQ ID NO:1-10 and the region of SEQ ID NO:1-10 to which the fragment corresponds are routinely determinable by one of ordinary skill in the art based on the intended purpose for the fragment.

A "full length" polynucleotide sequence is one containing at least a translation initiation codon (e.g., methionine) followed by an open reading frame and a translation termination codon. A "full length" polynucleotide sequence encodes a "full length" polypeptide sequence.

"Homology" refers to sequence similarity or, interchangeably, sequence identity, between two or more polynucleotide sequences or two or more polypeptide sequences.

The terms "percent identity" and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences.

Percent identity between polynucleotide sequences may be determined using the default parameters of the CLUSTAL V algorithm as incorporated into the MEGALIGN version 3.12e sequence alignment program. This program is part of the LASERGENE software package, a suite of molecular biological analysis programs (DNASTAR, Madison WI). CLUSTAL V is described in Higgins, D.G. and P.M. Sharp (1989) CABIOS 5:151-153 and in Higgins, D.G. et al. (1992) CABIOS 8:189-191. For pairwise alignments of polynucleotide sequences, the default parameters are set as follows: Ktuple=2, gap penalty=5, window=4, and "diagonals saved"=4. The "weighted" residue weight table is selected as the default. Percent identity is reported by CLUSTAL V as the "percent similarity" between aligned polynucleotide sequences.

Alternatively, a suite of commonly used and freely available sequence comparison algorithms is provided by the National Center for Biotechnology Information (NCBI) Basic Local Alignment Search Tool (BLAST) (Altschul, S.F. et al. (1990) J. Mol. Biol. 215:403-410), which is available from several sources, including the NCBI, Bethesda, MD, and on the Internet at http://www.ncbi.nlm.nih.gov/BLAST/. The BLAST software suite includes various sequence analysis programs including "blastn," that is used to align a known polynucleotide sequence with other polynucleotide sequences from a variety of databases. Also available is a tool called "BLAST 2 Sequences" that is used for direct pairwise comparison of two nucleotide sequences. "BLAST 2 Sequences" can be accessed and used interactively at http://www.ncbi.nlm.nih.gov/gorf/bl2.html. The "BLAST 2 Sequences" tool can be used for both blastn and blastp (discussed below). BLAST programs are commonly used with gap and other parameters set to default settings. For example, to compare two nucleotide sequences, one may use blastn with the "BLAST 2 Sequences" tool Version 2.0.12 (April-21-2000) set at default parameters. Such default parameters may be, for example:

*Matrix: BLOSUM62*
*Reward for match: 1*
*Penalty for mismatch: -2*
*Open Gap: 5 and Extension Gap: 2 penalties*
*Gap x drop-off: 50*
*Expect: 10*
*Word Size: 11*
*Filter: on*

Percent identity may be measured over the length of an entire defined sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length supported by the sequences shown herein, in the tables, figures, or Sequence Listing, may be used to describe a length over which percentage identity may be measured.

Nucleic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences that all encode substantially the same protein.

The phrases "percent identity" and "% identity," as applied to polypeptide sequences, refer to the percentage of residue matches between at least two polypeptide sequences aligned using a standardized algorithm. Methods of polypeptide sequence alignment are well-known. Some alignment methods take into account conservative amino acid substitutions. Such conservative substitutions, explained in more detail above, generally preserve the charge and_ hydrophobicity at the site of substitution, thus preserving the structure (and therefore function) of the polypeptide.

Percent identity between polypeptide sequences may be determined using the default parameters of the CLUSTAL V algorithm as incorporated into the MEGALIGN version 3.12e sequence alignment program (described and referenced above). For pairwise alignments of polypeptide sequences using CLUSTAL V, the default parameters are set as follows: Ktuple=1, gap penalty=3, window=5, and "diagonals saved"=5. The PAM250 matrix is selected as the default residue weight table. As with polynucleotide alignments, the percent identity is reported by CLUSTAL V as the "percent similarity" between aligned polypeptide sequence pairs.

Alternatively the NCBI BLAST software suite may be used. For example, for a pairwise comparison of two polypeptide sequences, one may use the "BLAST 2 Sequences" tool Version 2.0.12 (April-21-2000) with blastp set at default parameters. Such default parameters may be, for example:

*Matrix: BLOSUM62*
*Open Gap: 11 and Extension Gap: 1 penalties*
*Gap x drop-off: 50*
*Expect: 10*
*Word Size: 3*
*Filter: on*

Percent identity may be measured over the length of an entire defined polypeptide sequence, for example, as defined by a particular SEQ ID number, or may be measured over a shorter length, for example, over the length of a fragment taken from a larger, defined polypeptide sequence, for instance, a fragment of at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured.

"Human artificial chromosomes" (HACs) are linear microchromosomes which may contain DNA sequences of about 6 kb to 10 Mb in size and which contain all of the elements required for chromosome replication, segregation and maintenance.

The term "humanized antibody" refers to an antibody molecule in which the amino acid sequence in the non-antigen binding regions has been altered so that the antibody more closely resembles a human antibody, and still retains its original binding ability.

"Hybridization" refers to the process by which a polynucleotide strand anneals with a complementary strand through base pairing under defined hybridization conditions. Specific hybridization is an indication that two nucleic acid sequences share a high degree of complementarity. Specific hybridization complexes form under permissive annealing conditions and remain hybridized after the "washing" step(s). The washing step(s) is particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity. Permissive annealing conditions occur, for example, at 68°C in the presence of about 6 x SSC, about 1% (w/v) SDS, and about 100 μg/ml sheared, denatured salmon sperm DNA.

Generally, stringency of hybridization is expressed, in part, with reference to the temperature under which the wash step is carried out. Such wash temperatures are typically selected to be about 5°C to 20°C lower than the thermal melting point ($T_m$) for the specific sequence at a defined ionic strength and pH. The $T_m$ is the temperature (under defined ionic strength and pH) at which 50% of the target sequence hybridizes to a perfectly matched probe. An equation for calculating $T_m$ and conditions for nucleic acid hybridization are well known and can be found in Sambrook, J. et al. (1989) Molecular Cloning: A Laboratory Manual, 2nd ed., vol. 1-3, Cold Spring Harbor Press, Plainview NY; specifically see volume 2, chapter 9.

High stringency conditions for hybridization between polynucleotides of the present invention include wash conditions of 68°C in the presence of about 0.2 x SSC and about 0.1% SDS, for 1 hour. Alternatively, temperatures of about 65°C, 60°C, 55°C, or 42°C may be used. SSC concentration may be varied from about 0.1 to 2 x SSC, with SDS being present at about 0.1%. Typically, blocking reagents are used to block non-specific hybridization. Such blocking reagents include, for instance, sheared and denatured salmon sperm DNA at about 100-200 μg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, may be suggestive of evolutionary similarity between the nucleotides. Such similarity is strongly indicative of a similar role for the nucleotides and their encoded polypeptides.

The term "hybridization complex" refers to a complex formed between two nucleic acid sequences by virtue of the formation of hydrogen bonds between complementary bases. A hybridization complex may be formed in solution (e.g., $C_0t$ or $R_0t$ analysis) or formed between one nucleic acid sequence present in solution and another nucleic acid sequence immobilized on a solid support (e.g., paper, membranes, filters, chips, pins or glass slides, or any other appropriate substrate to which cells or their nucleic acids have been fixed).

The words "insertion" and "addition" refer to changes in an amino acid or nucleotide sequence resulting in the addition of one or more amino acid residues or nucleotides, respectively. "Immune response" can refer to conditions associated with inflammation, trauma, immune disorders, or infectious or genetic disease, etc. These conditions can be characterized by expression of various factors, e.g., cytokines, chemokines, and other signaling molecules, which may affect cellular and systemic defense systems.

An "immunogenic fragment" is a polypeptide or oligopeptide fragment of GCREC which is capable of eliciting an immune response when introduced into a living organism, for example, a mammal. The term "immunogenic fragment" also includes any polypeptide or oligopeptide fragment of GCREC which is useful in any of the antibody production methods disclosed herein or known in the art.

The term "microarray" refers to an arrangement of a plurality of polynucleotides, polypeptides, or other chemical compounds on a substrate.

The terms "element" and "array element" refer to a polynucleotide, polypeptide, or other chemical compound having a unique and defined position on a microarray.

The term "modulate" refers to a change in the activity of GCREC. For example, modulation may cause an increase or a decrease in protein activity, binding characteristics, or any other biological, functional, or immunological properties of GCREC.

The phrases "nucleic acid" and "nucleic acid sequence" refer to a nucleotide, oligonucleotide, polynucleotide, or any fragment thereof. These phrases also refer to DNA or RNA of genomic or synthetic origin which may be single-stranded or double-stranded and may represent the sense or the antisense strand, to peptide nucleic acid (PNA), or to any DNA-like or RNA-like material.

"Operably linked" refers to the situation in which a first nucleic acid sequence is placed in a functional relationship with a second nucleic acid sequence. For instance, a promoter is operably linked to a coding sequence if the promoter affects the transcription or expression of the coding sequence. Operably linked DNA sequences may be in close proximity or contiguous and, where necessary to join two protein coding regions, in the same reading frame.

"Peptide nucleic acid" (PNA) refers to an antisense molecule or anti-gene agent which comprises an oligonucleotide of at least about 5 nucleotides in length linked to a peptide backbone of amino acid residues ending in lysine. The terminal lysine confers solubility to the composition. PNAs preferentially bind complementary single stranded DNA or RNA and stop transcript elongation, and may be pegylated to extend their lifespan in the cell.

"Post-translational modification" of an GCREC may involve lipidation, glycosylation, phosphorylation, acetylation, racemization, proteolytic cleavage, and other modifications known in the art. These processes may occur synthetically or biochemically. Biochemical modifications will vary by cell type depending on the enzymatic milieu of GCREC.

"Probe" refers to nucleic acid sequences encoding GCREC, their complements, or fragments thereof, which are used to detect identical, allelic or related nucleic acid sequences. Probes are isolated oligonucleotides or polynucleotides attached to a detectable label or reporter molecule. Typical labels include radioactive isotopes, ligands, chemiluminescent agents, and enzymes. "Primers" are short nucleic acids, usually DNA oligonucleotides, which may be annealed to a target polynucleotide by complementary base-pairing. The primer may then be extended along the target DNA strand by a DNA polymerase enzyme. Primer pairs can be used for amplification (and identification) of a nucleic acid sequence, e.g., by the polymerase chain reaction (PCR).

Probes and primers as used in the present invention typically comprise at least 15 contiguous nucleotides of a known sequence. In order to enhance specificity, longer probes and primers may also be employed, such as probes and primers that comprise at least 20, 25, 30, 40, 50, 60, 70, 80, 90, 100, or at least 150 consecutive nucleotides of the disclosed nucleic acid sequences. Probes and primers may be considerably longer than these examples, and it is understood that any length supported by the specification, including the tables, figures, and Sequence Listing, may be used.

Methods for preparing and using probes and primers are described in the references, for example Sambrook, J. et al. (1989) Molecular Cloning: A Laboratory Manual, 2nd ed., vol. 1-3, Cold Spring Harbor Press, Plainview NY; Ausubel, F.M. et al. (1987) Current Protocols in Molecular Biology, Greene Publ. Assoc. & Wiley-Intersciences, New York NY; Innis, M. et al. (1990) PCR Protocols, A Guide to Methods and Applications, Academic Press, San Diego CA. PCR primer pairs can be derived from a known sequence, for example, by using computer programs intended for that purpose such as Primer (Version 0.5, 1991, Whitehead Institute for Biomedical Research, Cambridge MA).

Oligonucleotides for use as primers are selected using software known in the art for such purpose. For example, OLIGO 4.06 software is useful for the selection of PCR primer pairs of up to 100 nucleotides each, and for the analysis of oligonucleotides and larger polynucleotides of up to 5,000 nucleotides from an input polynucleotide sequence of up to 32 kilobases. Similar primer selection programs have incorporated additional features for expanded capabilities. For example, the PrimOU primer selection program (available to the public from the Genome Center at University of Texas South West Medical Center, Dallas TX) is capable of choosing specific primers from megabase sequences and is thus useful for designing primers on a genome-wide scope. The Primer3 primer selection program (available to the public from the Whitehead Institute/MIT Center for Genome Research, Cambridge MA) allows the user to input a "mispriming library," in which sequences to avoid as primer binding sites are user-specified. Primer3 is useful, in particular, for the selection of oligonucleotides for microarrays. (The source code for the latter two primer selection programs may also be obtained from their respective sources and modified to meet the user's specific needs.) The PrimeGen program (available to the public from the UK Human Genome Mapping Project Resource Centre, Cambridge UK) designs primers based on multiple sequence alignments, thereby allowing selection of primers that hybridize to either the most conserved or least conserved regions of aligned nucleic acid sequences. Hence, this program is useful for identification of both unique and conserved oligonucleotides and polynucleotide fragments. The oligonucleotides and polynucleotide fragments identified by any of the above selection methods are useful in hybridization technologies, for example, as PCR or sequencing primers, microarray elements, or specific probes to identify fully or partially complementary polynucleotides in a sample of nucleic acids. Methods of oligonucleotide selection are not limited to those described above.

A "recombinant nucleic acid" is a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, e.g., by genetic engineering techniques such as those described in Sambrook, supra. The term recombinant includes nucleic acids that have been altered solely by addition, substitution, or deletion of a portion of the nucleic acid. Frequently, a recombinant nucleic acid may include a nucleic acid sequence operably linked to a promoter sequence. Such a recombinant nucleic acid may be part of a vector that is used, for example, to transform a cell.

Alternatively, such recombinant nucleic acids may be part of a viral vector, e.g., based on a vaccinia virus, that could be use to vaccinate a mammal wherein the recombinant nucleic acid is expressed, inducing a protective immunological response in the mammal.

A "regulatory element" refers to a nucleic acid sequence usually derived from untranslated regions of a gene and includes enhancers, promoters, introns, and 5' and 3' untranslated regions (UTRs). Regulatory elements interact with host or viral proteins which control transcription, translation, or RNA stability.

"Reporter molecules" are chemical or biochemical moieties used for labeling a nucleic acid, amino acid, or antibody. Reporter molecules include radionuclides; enzymes; fluorescent, chemiluminescent, or chromogenic agents; substrates; cofactors; inhibitors; magnetic particles; and other moieties known in the art.

An "RNA equivalent," in reference to a DNA sequence, is composed of the same linear sequence of nucleotides as the reference DNA sequence with the exception that all occurrences of the nitrogenous base thymine are replaced with uracil, and the sugar backbone is composed of ribose instead of deoxyribose.

The term "sample" is used in its broadest sense. A sample suspected of containing GCREC, nucleic acids encoding GCREC, or fragments thereof may comprise a bodily fluid; an extract from a cell, chromosome, organelle, or membrane isolated from a cell; a cell; genomic DNA, RNA, or cDNA, in solution or bound to a substrate; a tissue; a tissue print; etc.

The terms "specific binding" and "specifically binding" refer to that interaction between a protein or peptide and an agonist, an antibody, an antagonist, a small molecule, or any natural or synthetic binding composition. The interaction is dependent upon the presence of a particular structure of the protein, e.g., the antigenic determinant or epitope, recognized by the binding molecule. For example, if an antibody is specific for epitope "A," the presence of a polypeptide comprising the epitope A, or the presence of free unlabeled A, in a reaction containing free labeled A and the antibody will reduce the amount of labeled A that binds to the antibody.

The term "substantially purified" refers to nucleic acid or amino acid sequences that are removed from their natural environment and are isolated or separated, and are at least 60% free, preferably at least 75% free, and most preferably at least 90% free from other components with which they are naturally associated.

A "substitution" refers to the replacement of one or more amino acid residues or nucleotides by different amino acid residues or nucleotides, respectively.

"Substrate" refers to any suitable rigid or semi-rigid support including membranes, filters, chips, slides, wafers, fibers, magnetic or nonmagnetic beads, gels, tubing, plates, polymers, microparticles and capillaries. The substrate can have a variety of surface forms, such as wells, trenches, pins, channels and pores, to which polynucleotides or polypeptides are bound.

A "transcript image" refers to the collective pattern of gene expression by a particular cell type or tissue under given conditions at a given time.

"Transformation" describes a process by which exogenous DNA is introduced into a recipient cell. Transformation may occur under natural or artificial conditions according to various methods well known in the art, and may rely on any known method for the insertion of foreign nucleic acid sequences into a prokaryotic or eukaryotic host cell. The method for transformation is selected based on the type of host cell being transformed and may include, but is not limited to, bacteriophage or viral infection, electroporation, heat shock, lipofection, and particle bombardment. The term "transformed cells" includes stably transformed cells in which the inserted DNA is capable of replication either as an autonomously replicating plasmid or as part of the host chromosome, as well as transiently transformed cells which express the inserted DNA or RNA for limited periods of time.

A "transgenic organism," as used herein, is any organism, including but not limited to animals and plants, in which one or more of the cells of the organism contains heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. The transgenic organisms contemplated in accordance with the present invention include bacteria, cyanobacteria, fungi, plants and animals. The isolated DNA of the present invention can be introduced into the host by methods known in the art, for example infection, transfection, transformation or transconjugation. Techniques for transferring the DNA of the present invention into such organisms are widely known and provided in references such as Sambrook et al. (1989), supra.

A "variant" of a particular nucleic acid sequence is defined as a nucleic acid sequence having at least 40% sequence identity to the particular nucleic acid sequence over a certain length of one of the nucleic acid sequences using blastn with the "BLAST 2 Sequences" tool Version 2.0.9 (May-07-1999) set at default parameters. Such a pair of nucleic acids may show, for example, at least 50%, at least 60%, at least 70%, at least 80%, at least 85%, at least 90%, at least 91%, at least 92%, at least 93%, at least 94%, at least 95%, at least 96%, at least 97%, at least 98%, or at least 99% or greater sequence identity over a certain defined length. A variant may be described as, for example, an "allelic" (as defined above), "splice," "species," or "polymorphic" variant. A splice variant may have significant identity to a reference molecule, but will generally have a greater or lesser number of polynucleotides due to alternative splicing of exons during mRNA processing. The corresponding polypeptide may possess additional functional domains or lack domains that are present in the reference molecule. Species variants are polynucleotide sequences that vary from one species to another. The resulting polypeptides will generally have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one nucleotide base. The presence of SNPs may be indicative of, for example, a certain population, a disease state, or a propensity for a disease state.

A "variant" of a particular polypeptide sequence is defined as a polypeptide sequence having at least 40% sequence identity to the particular polypeptide sequence over a certain length of one of the polypeptide sequences using blastp with the "BLAST 2 Sequences" tool Version 2.0.9 (May-07-1999) set at default parameters. Such a pair of polypeptides may show, for example, at least 50%, at least 60%, at least 70%, at least 80%, at least 90%, at least 91%, at least 92%, at least 93%, at least 94%, at least 95%, at least 96%, at least 97%, at least 98%, or at least 99% or greater sequence identity over a certain defined length of one of the polypeptides.

## THE INVENTION

The invention is based on the discovery of new human G-protein coupled receptors (GCREC), the polynucleotides encoding GCREC, and the use of these compositions for the diagnosis, treatment, or prevention of cell proliferative, neurological, cardiovascular, gastrointestinal, autoimmune/inflammatory, and metabolic disorders, and viral infections.

Table 1 summarizes the nomenclature for the full length polynucleotide and polypeptide sequences of the invention. Each polynucleotide and its corresponding polypeptide are correlated to a single Incyte project identification number (Incyte Project ID). Each polypeptide sequence is denoted by both a polypeptide sequence identification number (Polypeptide SEQ ID NO:) and an Incyte polypeptide sequence number (Incyte Polypeptide ID) as shown. Each polynucleotide sequence is denoted by both a polynucleotide sequence identification number (Polynucleotide SEQ ID NO:) and an Incyte polynucleotide consensus sequence number (Incyte Polynucleotide ID) as shown.

Table 2 shows sequences with homology to the polypeptides of the invention as identified by BLAST analysis against the GenBank protein (genpept) database. Columns 1 and 2 show the polypeptide sequence identification number (Polypeptide SEQ ID NO:) and the corresponding Incyte polypeptide sequence number (Incyte Polypeptide ID) for polypeptides of the invention. Column 3 shows the GenBank identification number (Genbank ID NO:) of the nearest GenBank homolog. Column 4 shows the probability score for the match between each polypeptide and its GenBank homolog. Column 5 shows the annotation of the GenBank homolog along with relevant citations where applicable, all of which are expressly incorporated by reference herein.

Table 3 shows various structural features of the polypeptides of the invention. Columns 1 and 2 show the polypeptide sequence identification number (SEQ ID NO:) and the corresponding Incyte polypeptide sequence number (Incyte Polypeptide ID) for each polypeptide of the invention. Column 3 shows the number of amino acid residues in each polypeptide. Column 4 shows potential phosphorylation sites, and column 5 shows potential glycosylation sites, as determined by the MOTIFS program of the GCG sequence analysis software package (Genetics Computer Group, Madison WI). Column 6 shows amino acid residues comprising signature sequences, domains, and motifs. Column 7 shows analytical methods for protein structure/function analysis and in some cases, searchable databases to which the analytical methods were applied.

Together, Tables 2 and 3 summarize the properties of polypeptides of the invention, and these properties establish that the claimed polypeptides are G-protein coupled receptors. For example, SEQ ID NO:4 is 44% identical to murine olfactory receptor P2 (GenBank ID g7638409) as determined by the Basic Local Alignment Search Tool (BLAST). (See Table 2.) The BLAST probability score is 4.6e-67, which indicates the probability of obtaining the observed polypeptide sequence alignment by chance. SEQ ID NO:4 also contains a seven transmembrane receptor domain as determined by searching for statistically significant matches in the hidden Markov model (HMM)-based PFAM database of conserved protein family domains. (See Table 3.) Data from BLIMPS, MOTIFS, and PROFILESCAN analyses provide further corroborative evidence that SEQ ID NO:4 is an olfactory G-protein coupled receptor. In an alternative example, SEQ ID NO:5 is 47% identical to murine olfactory receptor P2 (GenBank ID g7638409) as determined by BLAST. (See Table 2.) The BLAST probability score is 2.2e-67. SEQ ID NO:5 also contains G-protein coupled receptor signature domains as determined by searching for statistically significant matches in the HMM-based PFAM database of conserved protein family domains. (See Table 3.) Data from BLIMPS, MOTIFS, and PROFILESCAN analyses provide further corroborative evidence that SEQ ID NO:5 is a G-protein coupled receptor. In an alternative example, SEQ ID NO:7 is 87% identical to mouse odorant receptor S1 (GenBank ID g4680254) as determined by BLAST. (See Table 2.) The BLAST probability score is 1.1e-145. SEQ ID NO:7 also contains a 7 transmembrane receptor domain characteristic of the rhodopsin family, as determined by searching for statistically significant matches in the HMM-based PFAM database of conserved protein family domains. (See Table 3.) Data from BLIMPS, MOTIFS, and PROFILESCAN analyses provide further corroborative evidence that SEQ ID NO:7 is a G-protein coupled receptor. SEQ ID NO:1-3, SEQ ID NO:6, and SEQ ID NO:8-10 were analyzed and annotated in a similar manner. The algorithms and parameters for the analysis of SEQ ID NO:1-10 are described in Table 7.

As shown in Table 4, the full length polynucleotide sequences of the present invention were assembled using cDNA sequences or coding (exon) sequences derived from genomic DNA, or any combination of these two types of sequences. Columns 1 and 2 list the polynucleotide sequence identification number (Polynucleotide SEQ ID NO:) and the corresponding Incyte polynucleotide consensus sequence number (Incyte Polynucleotide ID) for each polynucleotide of the invention. Column 3 shows the length of each polynucleotide sequence in basepairs. Column 4 lists fragments of the polynucleotide sequences which are useful, for example, in hybridization or amplification technologies that identify SEQ ID NO:11-20 or that distinguish between SEQ ID NO:11-20 and related polynucleotide sequences. Column 5 shows identification numbers corresponding to cDNA sequences, coding sequences (exons) predicted from genomic DNA, and/or sequence assemblages comprised of both cDNA and genomic DNA. These sequences were used to assemble the full length polynucleotide sequences of the invention. Columns 6 and 7 of Table 4 show the nucleotide start (5') and stop (3') positions of the cDNA and/or genomic sequences in column 5 relative to their respective full length sequences.

The identification numbers in Column 5 of Table 4 may refer specifically, for example, to Incyte cDNAs along with their corresponding cDNA libraries. For example, 5805033T6 is the identification number of an Incyte cDNA sequence, and BONRFET03 is the cDNA library from which it is derived. Incyte cDNAs for which cDNA libraries are not indicated were derived from pooled cDNA libraries (e.g., 55012833H1). Alternatively, the identification numbers in column 5 may refer to GenBank cDNAs or ESTs which contributed to the assembly of the full length polynucleotide sequences. Alternatively, the identification numbers in column 5 may refer to coding regions predicted by Genscan analysis of genomic DNA. For example, GNN.g7283250_000011_008 is the identification number of a Genscan-predicted coding sequence, with g7283250 being the GenBank identification number of the sequence to which Genscan was applied. The Genscan-predicted coding sequences may have been edited prior to assembly. (See Example IV.) Alternatively, the identification numbers in column 5 may refer to assemblages of both cDNA and Genscan-predicted exons brought together by an "exon stitching" algorithm. For example, FL7472098CB1_00001 represents a "stitched" sequence in which 7472098 is the identification number of the cluster of sequences to which the algorithm was applied, and 00001 is the number of the prediction generated by the algorithm. (See Example V.) Alternatively, the identification numbers in column 5 may refer to assemblages of both cDNA and Genscan-predicted exons brought together by an "exon-stretching" algorithm. For example, FL7474927_g2822142_g4995709 is the identification number of a "stretched" sequence, with 7474927 being the Incyte project identification number, g2822142 being the GenBank identification number of the human genomic sequence to which the "exon-stretching" algorithm was applied, and g4995709 being the GenBank identification number of the nearest GenBank protein homolog. (See Example V.) In some cases, Incyte cDNA coverage redundant with the sequence coverage shown in column 5 was obtained to confirm the final consensus polynucleotide sequence, but the relevant Incyte cDNA identification numbers are not shown.

Table 5 shows the representative cDNA libraries for those full length polynucleotide sequences which were assembled using Incyte cDNA sequences. The representative cDNA library is the Incyte cDNA library which is most frequently represented by the Incyte cDNA sequences which were used to assemble and confirm the above polynucleotide sequences. The tissues and vectors which were used to construct the cDNA libraries shown in Table 5 are described in Table 6.

Table 8 shows tissue-specific expression of polynucleotides of the invention. Column 1 lists groups of tissues which were tested by polymerase chain reaction (PCR) for expression of the polynucleotides. The remaining columns indicate whether a particular polynucleotide was expressed in each tissue group. Detection of a PCR product indicated positive expression, denoted by a "+" sign, while inability to detect a PCR product indicated a lack of expression, denoted by a "-" sign.

The invention also encompasses GCREC variants. A preferred GCREC variant is one which has at least about 80%, or alternatively at least about 90%, or even at least about 95% amino acid sequence identity to the GCREC amino acid sequence, and which contains at least one functional or structural characteristic of GCREC.

The invention also encompasses polynucleotides which encode GCREC. In a particular embodiment, the invention encompasses a polynucleotide sequence comprising a sequence selected from the group consisting of SEQ ID NO:11-20, which encodes GCREC. The polynucleotide sequences of SEQ ID NO:11-20, as presented in the Sequence Listing, embrace the equivalent RNA sequences, wherein occurrences of the nitrogenous base thymine are replaced with uracil, and the sugar backbone is composed of ribose instead of deoxyribose.

The invention also encompasses a variant of a polynucleotide sequence encoding GCREC. In particular, such a variant polynucleotide sequence will have at least about 70%, or alternatively at least about 85%, or even at least about 95% polynucleotide sequence identity to the polynucleotide sequence encoding GCREC. A particular aspect of the invention encompasses a variant of a polynucleotide sequence comprising a sequence selected from the group consisting of SEQ ID NO:11-20 which has at least about 70%, or alternatively at least about 85%, or even at least about 95% polynucleotide sequence identity to a nucleic acid sequence selected from the group consisting of SEQ ID NO: 11-20. Any one of the polynucleotide variants described above can encode an amino acid sequence which contains at least one functional or structural characteristic of GCREC.

It will be appreciated by those skilled in the art that as a result of the degeneracy of the genetic code, a multitude of polynucleotide sequences encoding GCREC, some bearing minimal similarity to the polynucleotide sequences of any known and naturally occurring gene, may be produced. Thus, the invention contemplates each and every possible variation of polynucleotide sequence that could be made by selecting combinations based on possible codon choices. These combinations are made in accordance with the standard triplet genetic code as applied to the polynucleotide sequence of naturally occurring GCREC, and all such variations are to be considered as being specifically disclosed.

Although nucleotide sequences which encode GCREC and its variants are generally capable of hybridizing to the nucleotide sequence of the naturally occurring GCREC under appropriately selected conditions of stringency, it may be advantageous to produce nucleotide sequences encoding GCREC or its derivatives possessing a substantially different codon usage, e.g., inclusion of non-naturally occurring codons. Codons may be selected to increase the rate at which expression of the peptide occurs in a particular prokaryotic or eukaryotic host in accordance with the frequency with which particular codons are utilized by the host. Other reasons for substantially altering the nucleotide sequence encoding GCREC and its derivatives without altering the encoded amino acid sequences include the production of RNA transcripts having more desirable properties, such as a greater half-life, than transcripts produced from the naturally occurring sequence.

The invention also encompasses production of DNA sequences which encode GCREC and GCREC derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding GCREC or any fragment thereof.

Also encompassed by the invention are polynucleotide sequences that are capable of hybridizing to the claimed polynucleotide sequences, and, in particular, to those shown in SEQ ID NO:11-20 and fragments thereof under various conditions of stringency. (See, e.g., Wahl, G.M. and S.L. Berger (1987) Methods Enzymol. 152:399-407; Kimmel, A.R. (1987) Methods Enzymol. 152:507-511.) Hybridization conditions, including annealing and wash conditions, are described in "Definitions."

Methods for DNA sequencing are well known in the art and may be used to practice any of the embodiments of the invention. The methods may employ such enzymes as the Klenow fragment of DNA polymerase I, SEQUENASE (US Biochemical, Cleveland OH), Taq polymerase (Applied Biosystems), thermostable T7 polymerase (Amersham Pharmacia Biotech, Piscataway NJ), or combinations of polymerases and proofreading exonucleases such as those found in the ELONGASE amplification system (Life Technologies, Gaithersburg MD). Preferably, sequence preparation is automated with machines such as the MICROLAB 2200 liquid transfer system (Hamilton, Reno NV), PTC200 thermal cycler (MJ Research, Watertown MA) and ABI CATALYST 800 thermal cycler (Applied Biosystems). Sequencing is then carried out using either the ABI 373 or 377 DNA sequencing system (Applied Biosystems), the MEGABACE 1000 DNA sequencing system (Molecular Dynamics, Sunnyvale CA), or other systems known in the art. The resulting sequences are analyzed using a variety of algorithms which are well known in the art. (See, e.g., Ausubel, F.M. (1997) Short Protocols in Molecular Biology, John Wiley & Sons, New York NY, unit 7.7; Meyers, R.A. (1995) Molecular Biology and Biotechnology, Wiley VCH, New York NY, pp. 856-853.)

The nucleic acid sequences encoding GCREC may be extended utilizing a partial nucleotide sequence and employing various PCR-based methods known in the art to detect upstream sequences, such as promoters and regulatory elements. For example, one method which may be employed, restriction-site PCR, uses universal and nested primers to amplify unknown sequence from genomic DNA within a cloning vector. (See, e.g., Sarkar, G. (1993) PCR Methods Applic. 2:318-322.) Another method, inverse PCR, uses primers that extend in divergent directions to amplify unknown sequence from a circularized template. The template is derived from restriction fragments comprising a known genomic locus and surrounding sequences. (See, e.g., Triglia, T. et al. (1988) Nucleic Acids Res. 16:8186.) A third method, capture PCR, involves PCR amplification of DNA fragments adjacent to known sequences in human and yeast artificial chromosome DNA. (See, e.g., Lagerstrom, M. et al. (1991) PCR Methods Applic. 1:111-119.) In this method, multiple restriction enzyme digestions and ligations may be used to insert an engineered double-stranded sequence into a region of unknown sequence before performing PCR. Other methods which may be used to retrieve unknown sequences are known in the art. (See, e.g., Parker, J.D. et al. (1991) Nucleic Acids Res. 19:3055-3060). Additionally, one may use PCR, nested primers, and PROMOTERFINDER libraries (Clontech, Palo Alto CA) to walk genomic DNA. This procedure avoids the need to screen libraries and is useful in finding intron/exon junctions. For all PCR-based methods, primers may be designed using commercially available software, such as OLIGO 4.06 primer analysis software (National Biosciences, Plymouth MN) or another appropriate program, to be about 22 to 30 nucleotides in length, to have a GC content of about 50% or more, and to anneal to the template at temperatures of about 68°C to 72°C.

When screening for full length cDNAs, it is preferable to use libraries that have been size-selected to include larger cDNAs. In addition, random-primed libraries, which often include sequences containing the 5' regions of genes, are preferable for situations in which an oligo d(T) library does not yield a full-length cDNA. Genomic libraries may be useful for extension of sequence into 5' non-transcribed regulatory regions.

Capillary electrophoresis systems which are commercially available may be used to analyze the size or confirm the nucleotide sequence of sequencing or PCR products. In particular, capillary sequencing may employ flowable polymers for electrophoretic separation, four different nucleotide-specific, laser-stimulated fluorescent dyes, and a charge coupled device camera for detection of the emitted wavelengths. Output/light intensity may be converted to electrical signal using appropriate software (e.g., GENOTYPER and SEQUENCE NAVIGATOR, Applied Biosystems), and the entire process from loading of samples to computer analysis and electronic data display may be computer controlled. Capillary electrophoresis is especially preferable for sequencing small DNA fragments which may be present in limited amounts in a particular sample.

In another embodiment of the invention, polynucleotide sequences or fragments thereof which encode GCREC may be cloned in recombinant DNA molecules that direct expression of GCREC, or fragments or functional equivalents thereof, in appropriate host cells. Due to the inherent degeneracy of the genetic code, other DNA sequences which encode substantially the same or a functionally equivalent amino acid sequence may be produced and used to express GCREC.

The nucleotide sequences of the present invention can be engineered using methods generally known in the art in order to alter GCREC-encoding sequences for a variety of purposes including, but not limited to, modification of the cloning, processing, and/or expression of the gene product. DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth.

The nucleotides of the present invention may be subjected to DNA shuffling techniques such as MOLECULARBREEDING (Maxygen Inc., Santa Clara CA; described in U.S. Patent Number 5,837,458; Chang, C.-C. et al. (1999) Nat. Biotechnol. 17:793-797; Christians, F.C. et al. (1999) Nat. Biotechnol. 17:259-264; and Crameri, A. et al. (1996) Nat. Biotechnol. 14:315-319) to alter or improve the biological properties of GCREC, such as its biological or enzymatic activity or its ability to bind to other molecules or compounds. DNA shuffling is a process by which a library of gene variants is produced using PCR-mediated recombination of gene fragments. The library is then subjected to selection or screening procedures that identify those gene variants with the desired properties. These preferred variants may then be pooled and further subjected to recursive rounds of DNA shuffling and selection/screening. Thus, genetic diversity is created through "artificial" breeding and rapid molecular evolution. For example, fragments of a single gene containing random point mutations may be recombined, screened, and then reshuffled until the desired properties are optimized. Alternatively, fragments of a given gene may be recombined with fragments of homologous genes in the same gene family, either from the same or different species, thereby maximizing the genetic diversity of multiple naturally occurring genes in a directed and controllable manner.

In another embodiment, sequences encoding GCREC may be synthesized, in whole or in part, using chemical methods well known in the art. (See, e.g., Caruthers, M.H. et al. (1980) Nucleic Acids Symp. Ser. 7:215-223; and Horn, T. et al. (1980) Nucleic Acids Symp. Ser. 7:225-232.)
Alternatively, GCREC itself or a fragment thereof may be synthesized using chemical methods. For example, peptide synthesis can be performed using various solution-phase or solid-phase techniques. (See, e.g., Creighton, T. (1984) Proteins, Structures and Molecular Properties, WH Freeman, New York NY, pp. 55-60; and Roberge, J.Y. et al. (1995) Science 269:202-204.) Automated synthesis may be achieved using the ABI 431A peptide synthesizer (Applied Biosystems). Additionally, the amino acid sequence of GCREC, or any part thereof, may be altered during direct synthesis and/or combined with sequences from other proteins, or any part thereof, to produce a variant polypeptide or a polypeptide having a sequence of a naturally occurring polypeptide.

The peptide may be substantially purified by preparative high performance liquid chromatography. (See, e.g., Chiez, R.M. and F.Z. Regnier (1990) Methods Enzymol. 182:392-421.) The composition of the synthetic peptides may be confirmed by amino acid analysis or by sequencing. (See, e.g., Creighton, supra, pp. 28-53.)

In order to express a biologically active GCREC, the nucleotide sequences encoding GCREC or derivatives thereof may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3'untranslated regions in the vector and in polynucleotide sequences encoding GCREC. Such elements may vary in their strength and specificity. Specific initiation signals may also be used to achieve more efficient translation of sequences encoding GCREC. Such signals include the ATG initiation codon and adjacent sequences, e.g. the Kozak sequence. In cases where sequences encoding GCREC and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used. (See, e.g., Scharf, D. et al. (1994) Results Probl. Cell Differ. 20:125-162.)

Methods which are well known to those skilled in the art may be used to construct expression vectors containing sequences encoding GCREC and appropriate transcriptional and translational control elements. These methods include in vitro recombinant DNA techniques, synthetic techniques, and in vivo genetic recombination. (See, e.g., Sambrook, J. et al. (1989) Molecular Cloning, A Laboratory Manual, Cold Spring Harbor Press, Plainview NY, ch. 4, 8, and 16-17; Ausubel, F.M. et al. (1995) Current Protocols in Molecular Biology, John Wiley & Sons, New York NY, ch. 9, 13, and 16.)

A variety of expression vector/host systems may be utilized to contain and express sequences encoding GCREC. These include, but are not limited to, microorganisms such as bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus, CaMV, or tobacco mosaic virus, TMV) or with bacterial expression vectors (e.g., Ti or pBR322 plasmids); or animal cell systems. (See, e.g., Sambrook, supra; Ausubel, supra; Van Heeke, G. and S.M. Schuster (1989) J. Biol. Chem. 264:5503-5509; Engelhard, E.K. et al. (1994) Proc. Natl. Acad. Sci. USA 91:3224-3227; Sandig, V. et al. (1996) Hum. Gene Ther. 7:1937-1945; Takamatsu, N. (1987) EMBO J. 6:307-311; The McGraw Hill Yearbook of Science and Technology (1992) McGraw Hill, New York NY, pp. 191-196; Logan, J. and T. Shenk (1984) Proc. Natl. Acad. Sci. USA 81:3655-3659; and Harrington, J.J. et al. (1997) Nat. Genet. 15:345-355.) Expression vectors derived from retroviruses, adenoviruses, or herpes or vaccinia viruses, or from various bacterial plasmids, may be used for delivery of nucleotide sequences to the targeted organ, tissue, or cell population. (See, e.g., Di Nicola, M. et al. (1998) Cancer Gen. Ther. 5(6):350-356; Yu, M. et al. (1993) Proc. Natl. Acad. Sci. USA 90(13):6340-6344; Buller, R.M. et al. (1985) Nature 317(6040):813-815; McGregor, D.P. et al. (1994) Mol. Immunol. 31(3):219-226; and Verma, I.M. and N. Somia (1997) Nature 389:239-242.) The invention is not limited by the host cell employed.

In bacterial systems, a number of cloning and expression vectors may be selected depending upon the use intended for polynucleotide sequences encoding GCREC. For example, routine cloning, subcloning, and propagation of polynucleotide sequences encoding GCREC can be achieved using a multifunctional E. coli vector such as PBLUESCRIPT (Stratagene, La Jolla CA) or PSPORT1 plasmid (Life Technologies). Ligation of sequences encoding GCREC into the vector's multiple cloning site disrupts the *lacZ* gene, allowing a colorimetric screening procedure for identification of transformed bacteria containing recombinant molecules. In addition, these vectors may be useful for in vitro transcription, dideoxy sequencing, single strand rescue with helper phage, and creation of nested deletions in the cloned sequence. (See, e.g., Van Heeke, G. and S.M. Schuster (1989) J. Biol. Chem. 264:5503-5509.) When large quantities of GCREC are needed, e.g. for the production of antibodies, vectors which direct high level expression of GCREC may be used. For example, vectors containing the strong, inducible SP6 or T7 bacteriophage promoter may be used.

Yeast expression systems may be used for production of GCREC. A number of vectors containing constitutive or inducible promoters, such as alpha factor, alcohol oxidase, and PGH promoters, may be used in the yeast Saccharomyces cerevisiae or Pichia pastoris. In addition, such vectors direct either the secretion or intracellular retention of expressed proteins and enable integration of foreign sequences into the host genome for stable propagation. (See, e.g., Ausubel, 1995, supra; Bitter, G.A. et al. (1987) Methods Enzymol. 153:516-544; and Scorer, C.A. et al. (1994) Bio/Technology 12:181-184.)

Plant systems may also be used for expression of GCREC. Transcription of sequences encoding GCREC may be driven by viral promoters, e.g., the 35S and 19S promoters of CaMV used alone or in combination with the omega leader sequence from TMV (Takamatsu, N. (1987) EMBO J. 6:307-311). Alternatively, plant promoters such as the small subunit of RUBISCO or heat shock promoters may be used. (See, e.g., Coruzzi, G. et al. (1984) EMBO J. 3:1671-1680; Broglie, R. et al. (1984) Science 224:838-843; and Winter, J. et al. (1991) Results Probl. Cell Differ. 17:85-105.) These constructs can be introduced into plant cells by direct DNA transformation or pathogen-mediated transfection. (See, e.g., The McGraw Hill Yearbook of Science and Technology (1992) McGraw Hill, New York NY, pp. 191-196.)

In mammalian cells, a number of viral-based expression systems may be utilized. In cases where an adenovirus is used as an expression vector, sequences encoding GCREC may be ligated into an adenovirus transcription/translation complex consisting of the late promoter and tripartite leader sequence. Insertion in a non-essential E1 or E3 region of the viral genome may be used to obtain infective virus which expresses GCREC in host cells. (See, e.g., Logan, J. and T. Shenk (1984) Proc. Natl. Acad. Sci. USA 81:3655-3659.) In addition, transcription enhancers, such as the Rous sarcoma virus (RSV) enhancer, may be used to increase expression in mammalian host cells. SV40 or EBV-based vectors may also be used for high-level protein expression.

Human artificial chromosomes (HACs) may also be employed to deliver larger fragments of DNA than can be contained in and expressed from a plasmid. HACs of about 6 kb to 10 Mb are constructed and delivered via conventional delivery methods (liposomes, polycationic amino polymers, or vesicles) for therapeutic purposes. (See, e.g., Harrington, J.J. et al. (1997) Nat. Genet. 15:345-355.)

For long term production of recombinant proteins in mammalian systems, stable expression of GCREC in cell lines is preferred. For example, sequences encoding GCREC can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

Any number of selection systems may be used to recover transformed cell lines. These include, but are not limited to, the herpes simplex virus thymidine kinase and adenine phosphoribosyltransferase genes, for use in *tk*$^-$ and *apr*$^-$ cells, respectively. (See, e.g., Wigler, M. et al. (1977) Cell 11:223-232; Lowy, I. et al. (1980) Cell 22:817-823.) Also, antimetabolite, antibiotic, or herbicide resistance can be used as the basis for selection. For example, *dhfr* confers resistance to methotrexate; *neo* confers resistance to the aminoglycosides neomycin and G-418; and *als* and *pat* confer resistance to chlorsulfuron and phosphinotricin acetyltransferase, respectively. (See, e.g., Wigler, M. et al. (1980) Proc. Natl. Acad. Sci. USA 77:3567-3570; Colbere-Garapin, F. et al. (1981) J. Mol. Biol. 150:1-14.) Additional selectable genes have been described, e.g., *trpB* and *hisD*, which alter cellular requirements for metabolites. (See, e.g., Hartman, S.C. and R.C. Mulligan (1988) Proc. Natl. Acad. Sci. USA 85:8047-8051.) Visible markers, e.g., anthocyanins, green fluorescent proteins (GFP; Clontech), B glucuronidase and its substrate β-glucuronide, or luciferase and its substrate luciferin may be used. These markers can be used not only to identify transformants, but also to quantify the amount of transient or stable protein expression attributable to a specific vector system. (See, e.g., Rhodes, C.A. (1995) Methods Mol. Biol. 55:121-131.)

Although the presence/absence of marker gene expression suggests that the gene of interest is also present, the presence and expression of the gene may need to be confirmed. For example, if the sequence encoding GCREC is inserted within a marker gene sequence, transformed cells containing sequences encoding GCREC can be identified by the absence of marker gene function. Alternatively, a marker gene can be placed in tandem with a sequence encoding GCREC under the control of a single promoter. Expression of the marker gene in response to induction or selection usually indicates expression of the tandem gene as well.

In general, host cells that contain the nucleic acid sequence encoding GCREC and that express GCREC may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of GCREC using either specific polyclonal or monoclonal antibodies are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on GCREC is preferred, but a competitive binding assay may be employed. These and other assays are well known in the art. (See, e.g., Hampton, R. et al. (1990) Serological Methods, a Laboratory Manual, APS Press, St. Paul MN, Sect. IV; Coligan, J.E. et al. (1997) Current Protocols in Immunology, Greene Pub. Associates and Wiley-Interscience, New York NY; and Pound, J.D. (1998) Immunochemical Protocols, Humana Press, Totowa NJ.)

A wide variety of labels and conjugation techniques are known by those skilled in the art and may be used in various nucleic acid and amino acid assays. Means for producing labeled hybridization or PCR probes for detecting sequences related to polynucleotides encoding GCREC include oligolabeling, nick translation, end-labeling, or PCR amplification using a labeled nucleotide. Alternatively, the sequences encoding GCREC, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits, such as those provided by Amersham Pharmacia Biotech, Promega (Madison WI), and US Biochemical. Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding GCREC may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode GCREC may be designed to contain signal sequences which direct secretion of GCREC through a prokaryotic or eukaryotic cell membrane.

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" or "pro" form of the protein may also be used to specify protein targeting, folding, and/or activity. Different host cells which have specific cellular machinery and characteristic mechanisms for post-translational activities (e.g., CHO, HeLa, MDCK, HEK293, and WI38) are available from the American Type Culture Collection (ATCC, Manassas VA) and may be chosen to ensure the correct modification and processing of the foreign protein.

In another embodiment of the invention, natural, modified, or recombinant nucleic acid sequences encoding GCREC may be ligated to a heterologous sequence resulting in translation of a fusion protein in any of the aforementioned host systems. For example, a chimeric GCREC protein containing a heterologous moiety that can be recognized by a commercially available antibody may facilitate the screening of peptide libraries for inhibitors of GCREC activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His, FLAG, *c-myc*, and hemagglutinin (HA). GST, MBP, Trx, CBP, and 6-His enable purification of their cognate fusion proteins on immobilized glutathione, maltose, phenylarsine oxide, calmodulin, and metal-chelate resins, respectively. FLAG, c-myc, and hemagglutinin (HA) enable immunoaffinity purification of fusion proteins using commercially available monoclonal and polyclonal antibodies that specifically recognize these epitope tags. A fusion protein may also be engineered to contain a proteolytic cleavage site located between the GCREC encoding sequence and the heterologous protein sequence, so that GCREC may be cleaved away from the heterologous moiety following purification. Methods for fusion protein expression and purification are discussed in Ausubel (1995, supra, ch. 10). A variety of commercially available kits may also be used to facilitate expression and purification of fusion proteins.

In a further embodiment of the invention, synthesis of radiolabeled GCREC may be achieved in vitro using the TNT rabbit reticulocyte lysate or wheat germ extract system (Promega). These systems couple transcription and translation of protein-coding sequences operably associated with the T7, T3, or SP6 promoters. Translation takes place in the presence of a radiolabeled amino acid precursor, for example, $^{31}$S-methionine.

GCREC of the present invention or fragments thereof may be used to screen for compounds that specifically bind to GCREC. At least one and up to a plurality of test compounds may be screened for specific binding to GCREC. Examples of test compounds include antibodies, oligonucleotides, proteins (e.g., receptors), or small molecules.

In one embodiment, the compound thus identified is closely related to the natural ligand of GCREC, e.g., a ligand or fragment thereof, a natural substrate, a structural or functional mimetic, or a natural binding partner. (See, e.g., Coligan, J.E. et al. (1991) Current Protocols in Immunology 1(2): Chapter 5.) Similarly, the compound can be closely related to the natural receptor to which GCREC binds, or to at least a fragment of the receptor, e.g., the ligand binding site. In either case, the compound can be rationally designed using known techniques. In one embodiment, screening for these compounds involves producing appropriate cells which express GCREC, either as a secreted protein or on the cell membrane. Preferred cells include cells from mammals, yeast, Drosophila, or E. coli. Cells expressing GCREC or cell membrane fractions which contain GCREC are then contacted with a test compound and binding, stimulation, or inhibition of activity of either GCREC or the compound is analyzed.

An assay may simply test binding of a test compound to the polypeptide, wherein binding is detected by a fluorophore, radioisotope, enzyme conjugate, or other detectable label. For example, the assay may comprise the steps of combining at least one test compound with GCREC, either in solution or affixed to a solid support, and detecting the binding of GCREC to the compound. Alternatively, the assay may detect or measure binding of a test compound in the presence of a labeled competitor. Additionally, the assay may be carried out using cell-free preparations, chemical libraries, or natural product mixtures, and the test compound(s) may be free in solution or affixed to a solid support.

GCREC of the present invention or fragments thereof may be used to screen for compounds that modulate the activity of GCREC. Such compounds may include agonists, antagonists, or partial or inverse agonists. In one embodiment, an assay is performed under conditions permissive for GCREC activity, wherein GCREC is combined with at least one test compound, and the activity of GCREC in the presence of a test compound is compared with the activity of GCREC in the absence of the test compound. A change in the activity of GCREC in the presence of the test compound is indicative of a compound that modulates the activity of GCREC. Alternatively, a test compound is combined with an in vitro or cell-free system comprising GCREC under conditions suitable for GCREC activity, and the assay is performed. In either of these assays, a test compound which modulates the activity of GCREC may do so indirectly and need not come in direct contact with the test compound. At least one and up to a plurality of test compounds may be screened.

In another embodiment, polynucleotides encoding GCREC or their mammalian homologs may be "knocked out" in an animal model system using homologous recombination in embryonic stem (ES) cells. Such techniques are well known in the art and are useful for the generation of animal models of human disease. (See, e.g., U.S. Patent Number 5,175,383 and U.S. Patent Number 5,767,337.) For example, mouse ES cells, such as the mouse 129/SvJ cell line, are derived from the early mouse embryo and grown in culture. The ES cells are transformed with a vector containing the gene of interest disrupted by a marker gene, e.g., the neomycin phosphotransferase gene (neo; Capecchi, M.R. (1989) Science 244:1288-1292). The vector integrates into the corresponding region of the host genome by homologous recombination. Alternatively, homologous recombination takes place using the Cre-loxP system to knockout a gene of interest in a tissue- or developmental stage-specific manner (Marth, J.D. (1996) Clin. Invest. 97:1999-2002; Wagner, K.U. et al. (1997) Nucleic Acids Res. 25:4323-4330). Transformed ES cells are identified and microinjected into mouse cell blastocysts such as those from the C57BL/6 mouse strain. The blastocysts are surgically transferred to pseudopregnant dams, and the resulting chimeric progeny are genotyped and bred to produce heterozygous or homozygous strains. Transgenic animals thus generated may be tested with potential therapeutic or toxic agents.

Polynucleotides encoding GCREC may also be manipulated in vitro in ES cells derived from human blastocysts. Human ES cells have the potential to differentiate into at least eight separate cell lineages including endoderm, mesoderm, and ectodermal cell types. These cell lineages differentiate into, for example, neural cells, hematopoietic lineages, and cardiomyocytes (Thomson, J.A. et al. (1998) Science 282:1145-1147).

Polynucleotides encoding GCREC can also be used to create "knockin" humanized animals (pigs) or transgenic animals (mice or rats) to model human disease. With knockin technology, a region of a polynucleotide encoding GCREC is injected into animal ES cells, and the injected sequence integrates into the animal cell genome. Transformed cells are injected into blastulae, and the blastulae are implanted as described above. Transgenic progeny or inbred lines are studied and treated with potential pharmaceutical agents to obtain information on treatment of a human disease. Alternatively, a mammal inbred to overexpress GCREC, e.g., by secreting GCREC in its milk, may also serve as a convenient source of that protein (Janne, J. et al. (1998) Biotechnol. Annu. Rev. 4:55-74).

## THERAPEUTICS

Chemical and structural similarity, e.g., in the context of sequences and motifs, exists between regions of GCREC and G-protein coupled receptors. In addition, the expression of GCREC is closely associated with bone and nasal tissue. In particular, the expression of SEQ ID NO:15 is associated with thymus tissue. Therefore, GCREC appears to play a role in cell proliferative, neurological, cardiovascular, gastrointestinal, autoimmune/inflammatory, and metabolic disorders, and viral infections. In the treatment of disorders associated with increased GCREC expression or activity, it is desirable to decrease the expression or activity of GCREC. In the treatment of disorders associated with decreased GCREC expression or activity, it is desirable to increase the expression or activity of GCREC.

Therefore, in one embodiment, GCREC or a fragment or derivative thereof may be administered to a subject to treat or prevent a disorder associated with decreased expression or activity of GCREC. Examples of such disorders include, but are not limited to, a cell proliferative disorder such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and, in particular, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus; a neurological disorder such as epilepsy, ischemic cerebrovascular disease, stroke, cerebral neoplasms, Alzheimer's disease, Pick's disease, Huntington's disease, dementia, Parkinson's disease and other extrapyramidal disorders, amyotrophic lateral sclerosis and other motor neuron disorders, progressive neural muscular atrophy, retinitis pigmentosa, hereditary ataxias, multiple sclerosis and other demyelinating diseases, bacterial and viral meningitis, brain abscess, subdural empyema, epidural abscess, suppurative intracranial thrombophlebitis, myelitis and radiculitis, viral central nervous system disease, prion diseases including kuru, Creutzfeldt-Jakob disease, and Gerstmann-Straussler-Scheinker syndrome, fatal familial insomnia, nutritional and metabolic diseases of the nervous system, neurofibromatosis, tuberous sclerosis, cerebelloretinal hemangioblastomatosis, encephalotrigeminal syndrome, mental retardation and other developmental disorders of the central nervous system, cerebral palsy, neuroskeletal disorders, autonomic nervous system disorders, cranial nerve disorders, spinal cord diseases, muscular dystrophy and other neuromuscular disorders, peripheral nervous system disorders, dermatomyositis and polymyositis, inherited, metabolic, endocrine, and toxic myopathies, myasthenia gravis, periodic paralysis, mental disorders including mood, anxiety, and schizophrenic disorders, seasonal affective disorder (SAD), akathesia, amnesia, catatonia, diabetic neuropathy, tardive dyskinesia, dystonias, paranoid psychoses, postherpetic neuralgia, Tourette's disorder, progressive supranuclear palsy, corticobasal degeneration, and familial frontotemporal dementia; a cardiovascular disorder such as arteriovenous fistula, atherosclerosis, hypertension, vasculitis, Raynaud's disease, aneurysms, arterial dissections, varicose veins, thrombophlebitis and phlebothrombosis, vascular tumors, complications of thrombolysis, balloon angioplasty, vascular replacement, and coronary artery bypass graft surgery, congestive heart failure, ischemic heart disease, angina pectoris, myocardial infarction, hypertensive heart disease, degenerative valvular heart disease, calcific aortic valve stenosis, congenitally bicuspid aortic valve, mitral annular calcification, mitral valve prolapse, rheumatic fever and rheumatic heart disease, infective endocarditis, nonbacterial thrombotic endocarditis, endocarditis of systemic lupus erythematosus, carcinoid heart disease, cardiomyopathy, myocarditis, pericarditis, neoplastic heart disease, congenital heart disease, and complications of cardiac transplantation; a gastrointestinal disorder such as dysphagia, peptic esophagitis, esophageal spasm, esophageal stricture, esophageal carcinoma, dyspepsia, indigestion, gastritis, gastric carcinoma, anorexia, nausea, emesis, gastroparesis, antral or pyloric edema, abdominal angina, pyrosis, gastroenteritis, intestinal obstruction, infections of the intestinal tract, peptic ulcer, cholelithiasis, cholecystitis, cholestasis, pancreatitis, pancreatic carcinoma, biliary tract disease, hepatitis, hyperbilirubinemia, cirrhosis, passive congestion of the liver, hepatoma, infectious colitis, ulcerative colitis, ulcerative proctitis, Crohn's disease, Whipple's disease, Mallory-Weiss syndrome, colonic carcinoma, colonic obstruction, irritable bowel syndrome, short bowel syndrome, diarrhea, constipation, gastrointestinal hemorrhage, acquired immunodeficiency syndrome (AIDS) enteropathy, jaundice, hepatic encephalopathy, hepatorenal syndrome, hepatic steatosis, hemochromatosis, Wilson's disease, alpha$_1$-antitrypsin deficiency, Reye's syndrome, primary sclerosing cholangitis, liver infarction, portal vein obstruction and thrombosis, centrilobular necrosis, peliosis hepatis, hepatic vein thrombosis, veno-occlusive disease, preeclampsia, eclampsia, acute fatty liver of pregnancy, intrahepatic cholestasis of pregnancy, and hepatic tumors including nodular hyperplasias, adenomas, and carcinomas; an autoimmune/inflammatory disorder such as acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, autoimmune polyendocrinopathy-candidiasis-ectodermal dystrophy (APECED), bronchitis, cholecystitis, contact dermatitis, Crohn's disease, atopic dermatitis, dermatomyositis, diabetes mellitus, emphysema, episodic lymphopenia with lymphocytotoxins, erythroblastosis fetalis, erythema nodosum, atrophic gastritis, glomerulonephritis, Goodpasture's syndrome, gout, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polymyositis, psoriasis, Reiter's syndrome, rheumatoid arthritis, scleroderma, Sjögren's syndrome, systemic anaphylaxis, systemic lupus erythematosus, systemic sclerosis, thrombocytopenic purpura, ulcerative colitis, uveitis, Werner syndrome, complications of cancer, hemodialysis, and extracorporeal circulation, viral, bacterial, fungal, parasitic, protozoal, and helminthic infections, and trauma; a metabolic disorder such as diabetes, obesity, and osteoporosis; and an infection by a viral agent classified as adenovirus, arenavirus, bunyavirus, calicivirus, coronavirus, filovirus, hepadnavirus, herpesvirus, flavivirus, orthomyxovirus, parvovirus, papovavirus, paramyxovirus, picornavirus, poxvirus, reovirus, retrovirus, rhabdovirus, and tongavirus.

In another embodiment, a vector capable of expressing GCREC or a fragment or derivative thereof may be administered to a subject to treat or prevent a disorder associated with decreased expression or activity of GCREC including, but not limited to, those described above.

In a further embodiment, a composition comprising a substantially purified GCREC in conjunction with a suitable pharmaceutical carrier may be administered to a subject to treat or prevent a disorder associated with decreased expression or activity of GCREC including, but not limited to, those provided above.

In still another embodiment, an agonist which modulates the activity of GCREC may be administered to a subject to treat or prevent a disorder associated with decreased expression or activity of GCREC including, but not limited to, those listed above.

In a further embodiment, an antagonist of GCREC may be administered to a subject to treat or prevent a disorder associated with increased expression or activity of GCREC. Examples of such disorders include, but are not limited to, those cell proliferative, neurological, cardiovascular, gastrointestinal, autoimmune/inflammatory, and metabolic disorders, and viral infections, described above. In one aspect, an antibody which specifically binds GCREC may be used directly as an antagonist or indirectly as a targeting or delivery mechanism for bringing a pharmaceutical agent to cells or tissues which express GCREC.

In an additional embodiment, a vector expressing the complement of the polynucleotide encoding GCREC may be administered to a subject to treat or prevent a disorder associated with increased expression or activity of GCREC including, but not limited to, those described above.

In other embodiments, any of the proteins, antagonists, antibodies, agonists, complementary sequences, or vectors of the invention may be administered in combination with other appropriate therapeutic agents. Selection of the appropriate agents for use in combination therapy may be made by one of ordinary skill in the art, according to conventional pharmaceutical principles. The combination of therapeutic agents may act synergistically to effect the treatment or prevention of the various disorders described above. Using this approach, one may be able to achieve therapeutic efficacy with lower dosages of each agent, thus reducing the potential for adverse side effects.

An antagonist of GCREC may be produced using methods which are generally known in the art. In particular, purified GCREC may be used to produce antibodies or to screen libraries of pharmaceutical agents to identify those which specifically bind GCREC. Antibodies to GCREC may also be generated using methods that are well known in the art. Such antibodies may include, but are not limited to, polyclonal, monoclonal, chimeric, and single chain antibodies, Fab fragments, and fragments produced by a Fab expression library. Neutralizing antibodies (i.e., those which inhibit dimer formation) are generally preferred for therapeutic use.

For the production of antibodies, various hosts including goats, rabbits, rats, mice, humans, and others may be immunized by injection with GCREC or with any fragment or oligopeptide thereof which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol. Among adjuvants used in humans, BCG (bacilli Calmette-Guerin) and Corynebacterium parvum are especially preferable.

It is preferred that the oligopeptides, peptides, or fragments used to induce antibodies to GCREC have an amino acid sequence consisting of at least about 5 amino acids, and generally will consist of at least about 10 amino acids. It is also preferable that these oligopeptides, peptides, or fragments are identical to a portion of the amino acid sequence of the natural protein. Short stretches of GCREC amino acids may be fused with those of another protein, such as KLH, and antibodies to the chimeric molecule may be produced.

Monoclonal antibodies to GCREC may be prepared using any technique which provides for the production of antibody molecules by continuous cell lines in culture. These include, but are not limited to, the hybridoma technique, the human B-cell hybridoma technique, and the EBV-hybridoma technique. (See, e.g., Kohler, G. et al. (1975) Nature 256:495-497; Kozbor, D. et al. (1985) J. Immunol. Methods 81:31-42; Cote, R.J. et al. (1983) Proc. Natl. Acad. Sci. USA 80:2026-2030; and Cole, S.P. et al. (1984) Mol. Cell Biol. 62:109-120.)

In addition, techniques developed for the production of "chimeric antibodies," such as the splicing of mouse antibody genes to human antibody genes to obtain a molecule with appropriate antigen specificity and biological activity, can be used. (See, e.g., Morrison, S.L. et al. (1984) Proc. Natl. Acad. Sci. USA 81:6851-6855; Neuberger, M.S. et al. (1984) Nature 312:604-608; and Takeda, S. et al. (1985) Nature 314:452-454.) Alternatively, techniques described for the production of single chain antibodies may be adapted, using methods known in the art, to produce GCREC-specific single chain antibodies. Antibodies with related specificity, but of distinct idiotypic composition, may be generated by chain shuffling from random combinatorial immunoglobulin libraries. (See, e.g., Burton, D.R. (1991) Proc. Natl. Acad. Sci. USA 88:10134-10137.)

Antibodies may also be produced by inducing in vivo production in the lymphocyte population or by screening immunoglobulin libraries or panels of highly specific binding reagents as disclosed in the literature. (See, e.g., Orlandi, R. et al. (1989) Proc. Natl. Acad. Sci. USA 86:3833-3837; Winter, G. et al. (1991) Nature 349:293-299.)

Antibody fragments which contain specific binding sites for GCREC may also be generated. For example, such fragments include, but are not limited to, $F(ab)_2$ fragments produced by pepsin digestion of the antibody molecule and Fab fragments generated by reducing the disulfide bridges of the F(ab')2 fragments. Alternatively, Fab expression libraries may be constructed to allow rapid and easy identification of monoclonal Fab fragments with the desired specificity. (See, e.g., Huse, W.D. et al. (1989) Science 246:1275-1281.)

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between GCREC and its specific antibody. A two-site, monoclonal-based immunoassay utilizing monoclonal antibodies reactive to two non-interfering GCREC epitopes is generally used, but a competitive binding assay may also be employed (Pound, supra).

Various methods such as Scatchard analysis in conjunction with radioimmunoassay techniques may be used to assess the affinity of antibodies for GCREC. Affinity is expressed as an association constant, $K_a$, which is defined as the molar concentration of GCREC-antibody complex divided by the molar concentrations of free antigen and free antibody under equilibrium conditions. The $K_a$ determined for a preparation of polyclonal antibodies, which are heterogeneous in their affinities for multiple GCREC epitopes, represents the average affinity, or avidity, of the antibodies for GCREC. The $K_a$ determined for a preparation of monoclonal antibodies, which are monospecific for a particular GCREC epitope, represents a true measure of affinity. High-affinity antibody preparations with $K_a$ ranging from about $10^9$ to $10^{12}$ L/mole are preferred for use in immunoassays in which the GCREC-antibody complex must withstand rigorous manipulations. Low-affinity antibody preparations with $K_a$ ranging from about $10^6$ to $10^7$ L/mole are preferred for use in immunopurification and similar procedures which ultimately require dissociation of GCREC, preferably in active form, from the antibody (Catty, D. (1988) Antibodies, Volume I: A Practical Approach, IRL Press, Washington DC; Liddell, J.E. and A. Cryer (1991) A Practical Guide to Monoclonal Antibodies, John Wiley & Sons, New York NY).

The titer and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is generally employed in procedures requiring precipitation of GCREC-antibody complexes. Procedures for evaluating antibody specificity, titer, and avidity, and guidelines for antibody quality and usage in various applications, are generally available. (See, e.g., Catty, supra, and Coligan et al. supra.)

In another embodiment of the invention, the polynucleotides encoding GCREC, or any fragment or complement thereof, may be used for therapeutic purposes. In one aspect, modifications of gene expression can be achieved by designing complementary sequences or antisense molecules (DNA, RNA, PNA, or modified oligonucleotides) to the coding or regulatory regions of the gene encoding GCREC. Such technology is well known in the art, and antisense oligonucleotides or larger fragments can be designed from various locations along the coding or control regions of sequences encoding GCREC. (See, e.g., Agrawal, S., ed. (1996) Antisense Therapeutics, Humana Press Inc., Totawa NJ.)

In therapeutic use, any gene delivery system suitable for introduction of the antisense sequences into appropriate target cells can be used. Antisense sequences can be delivered intracellularly in the form of an expression plasmid which, upon transcription, produces a sequence complementary to at least a portion of the cellular sequence encoding the target protein. (See, e.g., Slater, J.E. et al. (1998) J. Allergy Clin. Immunol. 102(3):469-475; and Scanlon, K.J. et al. (1995) 9(13):1288-1296.) Antisense sequences can also be introduced intracellularly through the use of viral vectors, such as retrovirus and adeno-associated virus vectors. (See, e.g., Miller, A.D. (1990) Blood 76:271; Ausubel, supra; Uckert, W. and W. Walther (1994) Pharmacol. Ther. 63(3):323-347.) Other gene delivery mechanisms include liposome-derived systems, artificial viral envelopes, and other systems known in the art. (See, e.g., Rossi, J.J. (1995) Br. Med. Bull. 51(1):217-225; Boado, R.J. et al. (1998) J. Pharm. Sci. 87(11):1308-1315; and Morris, M.C. et al. (1997) Nucleic Acids Res. 25(14):2730-2736.)

In another embodiment of the invention, polynucleotides encoding GCREC may be used for somatic or germline gene therapy. Gene therapy may be performed to (i) correct a genetic deficiency (e.g., in the cases of severe combined immunodeficiency (SCID)-X1 disease characterized by X-linked inheritance (Cavazzana-Calvo, M. et al. (2000) Science 288:669-672), severe combined immunodeficiency syndrome associated with an inherited adenosine deaminase (ADA) deficiency (Blaese, R.M. et al. (1995) Science 270:475-480; Bordignon, C. et al. (1995) Science 270:470-475), cystic fibrosis (Zabner, J. et al. (1993) Cell 75:207-216; Crystal, R.G. et al. (1995) Hum. Gene Therapy 6:643-666; Crystal, R.G. et al. (1995) Hum. Gene Therapy 6:667-703), thalassamias, familial hypercholesterolemia, and hemophilia resulting from Factor VIII or Factor IX deficiencies (Crystal, R.G. (1995) Science 270:404-410; Verma, I.M. and N. Somia (1997) Nature 389:239-242)), (ii) express a conditionally lethal gene product (e.g., in the case of cancers which result from unregulated cell proliferation), or (iii) express a protein which affords protection against intracellular parasites (e.g., against human retroviruses, such as human immunodeficiency virus (HIV) (Baltimore, D. (1988) Nature 335:395-396; Poeschla, E. et al. (1996) Proc. Natl. Acad. Sci. USA. 93:11395-11399), hepatitis B or C virus (HBV, HCV); fungal parasites, such as Candida albicans and Paracoccidioides brasiliensis; and protozoan parasites such as Plasmodium falciparum and Trypanosoma cruzi). In the case where a genetic deficiency in GCREC expression or regulation causes disease, the expression of GCREC from an appropriate population of transduced cells may alleviate the clinical manifestations caused by the genetic deficiency.

In a further embodiment of the invention, diseases or disorders caused by deficiencies in GCREC are treated by constructing mammalian expression vectors encoding GCREC and introducing these vectors by mechanical means into GCREC-deficient cells. Mechanical transfer technologies for use with cells in vivo or ex vitro include (i) direct DNA microinjection into individual cells, (ii) ballistic gold particle delivery, (iii) liposome-mediated transfection, (iv) receptor-mediated gene transfer, and (v) the use of DNA transposons (Morgan, R.A. and W.F. Anderson (1993) Annu. Rev. Biochem. 62:191-217; Ivics, Z. (1997) Cell 91:501-510; Boulay, J-L. and H. Récipon (1998) Curr. Opin. Biotechnol. 9: 445-450).

Expression vectors that may be effective for the expression of GCREC include, but are not limited to, the PCDNA 3.1, EPITAG, PRCCMV2, PREP, PVAX vectors (Invitrogen, Carlsbad CA), PCMV-SCRIPT, PCMV-TAG, PEGSH/PERV (Stratagene, La Jolla CA), and PTET-OFF, PTET-ON, PTRE2, PTRE2-LUC, PTK-HYG (Clontech, Palo Alto CA). GCREC may be expressed using (i) a constitutively active promoter, (e.g., from cytomegalovirus (CMV), Rous sarcoma virus (RSV), SV40 virus, thymidine kinase (TK), or β-acting genes), (ii) an inducible promoter (e.g., the tetracycline-regulated promoter (Gossen, M. and H. Bujard (1992) Proc. Natl. Acad. Sci. USA 89:5547-5551; Gossen, M. et al. (1995) Science 268:1766-1769; Rossi, F.M.V. and H.M. Blau (1998) Curr. Opin. Biotechnol. 9:451-456), commercially available in the T-REX plasmid (Invitrogen)); the ecdysone-inducible promoter (available in the plasmids PVGRXR and PIND; Invitrogen); the FK506/rapamycin inducible promoter; or the RU486/mifepristone inducible promoter (Rossi, F.M.V. and Blau, H.M. supra)), or (iii) a tissue-specific promoter or the native promoter of the endogenous gene encoding GCREC from a normal individual.

Commercially available liposome transformation kits (e.g., the PERFECT LIPID TRANSFECTION KIT, available from Invitrogen) allow one with ordinary skill in the art to deliver polynucleotides to target cells in culture and require minimal effort to optimize experimental parameters. In the alternative, transformation is performed using the calcium phosphate method (Graham, F.L. and A.J. Eb (1973) Virology 52:456-467), or by electroporation (Neumann, E. et al. (1982) EMBO J. 1:841-845). The introduction of DNA to primary cells requires modification of these standardized mammalian transfection protocols.

In another embodiment of the invention, diseases or disorders caused by genetic defects with respect to GCREC expression are treated by constructing a retrovirus vector consisting of (i) the polynucleotide encoding GCREC under the control of an independent promoter or the retrovirus long terminal repeat (LTR) promoter, (ii) appropriate RNA packaging signals, and (iii) a Rev-responsive element (RRE) along with additional retrovirus cis-acting RNA sequences and coding sequences required for efficient vector propagation. Retrovirus vectors (e.g., PFB and PFBNEO) are commercially available (Stratagene) and are based on published data (Riviere, I. et al. (1995) Proc. Natl. Acad. Sci. USA 92:6733-6737), incorporated by reference herein. The vector is propagated in an appropriate vector producing cell line (VPCL) that expresses an envelope gene with a tropism for receptors on the target cells or a promiscuous envelope protein such as VSVg (Armentano, D. et al. (1987) J. Virol. 61:1647-1650; Bender, M.A. et al. (1987) J. Virol. 61: 1639-1646; Adam, M.A. and A.D. Miller (1988) J. Virol. 62:3802-3806; Dull, T. et al. (1998) J. Virol. 72:8463-8471; Zufferey, R. et al. (1998) J. Virol. 72:9873-9880). U.S. Patent Number 5,910,434 to Rigg ("Method for obtaining retrovirus packaging cell lines producing high transducing efficiency retroviral supernatant") discloses a method for obtaining retrovirus packaging cell lines and is hereby incorporated by reference. Propagation of retrovirus vectors, transduction of a population of cells (e.g., $CD4^+$ T-cells), and the return of transduced cells to a patient are procedures well known to persons skilled in the art of gene therapy and have been well documented (Ranga, U. et al. (1997) J. Virol. 71: 7020-7029; Bauer, G. et al. (1997) Blood 89:2259-2267; Bonyhadi, M.L. (1997) J. Virol. 71:4707-4716; Ranga, U. et al. (1998) Proc. Natl. Acad. Sci. USA 95:1201-1206; Su, L. (1997) Blood 89:2283-2290).

In the alternative, an adenovirus-based gene therapy delivery system is used to deliver polynucleotides encoding GCREC to cells which have one or more genetic abnormalities with respect to the expression of GCREC. The construction and packaging of adenovirus-based vectors are well known to those with ordinary skill in the art. Replication defective adenovirus vectors have proven to be versatile for importing genes encoding immunoregulatory proteins into intact islets in the pancreas (Csete, M.E. et al. (1995) Transplantation 27:263-268). Potentially useful adenoviral vectors are described in U.S. Patent Number 5,707,618 to Armentano ("Adenovirus vectors for gene therapy"), hereby incorporated by reference. For adenoviral vectors, see also Antinozzi, P.A. et al. (1999) Annu. Rev. Nutr. 19:511-544 and Verma, I.M. and N. Somia (1997) Nature 18:389:239-242, both incorporated by reference herein.

In another alternative, a herpes-based, gene therapy delivery system is used to deliver polynucleotides encoding GCREC to target cells which have one or more genetic abnormalities with respect to the expression of GCREC. The use of herpes simplex virus (HSV)-based vectors may be especially valuable for introducing GCREC to cells of the central nervous system, for which HSV has a tropism. The construction and packaging of herpes-based vectors are well known to those with ordinary skill in the art. A replication-competent herpes simplex virus (HSV) type 1-based vector has been used to deliver a reporter gene to the eyes of primates (Liu, X. et al. (1999) Exp. Eye Res. 169:385-395). The construction of a HSV-1 virus vector has also been disclosed in detail in U.S. Patent Number 5,804,413 to DeLuca ("Herpes simplex virus strains for gene transfer"), which is hereby incorporated by reference. U.S. Patent Number 5,804,413 teaches the use of recombinant HSV d92 which consists of a genome containing at least one exogenous gene to be transferred to a cell under the control of the appropriate promoter for purposes including human gene therapy. Also taught by this patent are the construction and use of recombinant HSV strains deleted for ICP4, ICP27 and ICP22. For HSV vectors, see also Goins, W.F. et al. (1999) J. Virol. 73:519-532 and Xu, H. et al. (1994) Dev. Biol. 163:152-161, hereby incorporated by reference. The manipulation of cloned herpesvirus sequences, the generation of recombinant virus following the transfection of multiple plasmids containing different segments of the large herpesvirus genomes, the growth and propagation of herpesvirus, and the infection of cells with herpesvirus are techniques well known to those of ordinary skill in the art.

In another alternative, an alphavirus (positive, single-stranded RNA virus) vector is used to deliver polynucleotides encoding GCREC to target cells. The biology of the prototypic alphavirus, Semliki Forest Virus (SFV), has been studied extensively and gene transfer vectors have been based on the SFV genome (Garoff, H. and K.-J. Li (1998) Curr. Opin. Biotechnol. 9:464-469). During alphavirus RNA replication, a subgenomic RNA is generated that normally encodes the viral capsid proteins. This sub genomic RNA replicates to higher levels than the full length genomic RNA, resulting in the overproduction of capsid proteins relative to the viral proteins with enzymatic activity (e.g., protease and polymerase). Similarly, inserting the coding sequence for GCREC into the alphavirus genome in place of the capsid-coding region results in the production of a large number of GCREC-coding RNAs and the synthesis of high levels of GCREC in vector transduced cells. While alphavirus infection is typically associated with cell lysis within a few days, the ability to establish a persistent infection in hamster normal kidney cells (BHK-21) with a variant of Sindbis virus (SIN) indicates that the lytic replication of alphaviruses can be altered to suit the needs of the gene therapy application (Dryga, S.A. et al. (1997) Virology 228:74-83). The wide host range of alphaviruses will allow the introduction of GCREC into a variety of cell types. The specific transduction of a subset of cells in a population may require the sorting of cells prior to transduction. The methods of manipulating infectious cDNA clones of alphaviruses, performing alphavirus cDNA and RNA transfections, and performing alphavirus infections, are well known to those with ordinary skill in the art.

Oligonucleotides derived from the transcription initiation site, e.g., between about positions -10 and +10 from the start site, may also be employed to inhibit gene expression. Similarly, inhibition can be achieved using triple helix base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature. (See, e.g., Gee, J.E. et al. (1994) in Huber, B.E. and B.I. Carr, Molecular and Immunologic Approaches, Futura Publishing, Mt. Kisco NY, pp. 163-177.) A complementary sequence or antisense molecule may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. For example, engineered hammerhead motif ribozyme molecules may specifically and efficiently catalyze endonucleolytic cleavage of sequences encoding GCREC.

Specific ribozyme cleavage sites within any potential RNA target are initially identified by scanning the target molecule for ribozyme cleavage sites, including the following sequences: GUA, GUU, and GUC. Once identified, short RNA sequences of between 15 and 20 ribonucleotides, corresponding to the region of the target gene containing the cleavage site, may be evaluated for secondary structural features which may render the oligonucleotide inoperable. The suitability of candidate targets may also be evaluated by testing accessibility to hybridization with complementary oligonucleotides using ribonuclease protection assays.

Complementary ribonucleic acid molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding GCREC. Such DNA sequences may be incorporated into a wide variety of vectors with suitable RNA polymerase promoters such as T7 or SP6. Alternatively, these cDNA constructs that synthesize complementary RNA, constitutively or inducibly, can be introduced into cell lines, cells, or tissues.

RNA molecules may be modified to increase intracellular stability and half-life. Possible modifications include, but are not limited to, the addition of flanking sequences at the 5' and/or 3' ends of the molecule, or the use of phosphorothioate or 2'O-methyl rather than phosphodiesterase linkages within the backbone of the molecule. This concept is inherent in the production of PNAs and can be extended in all of these molecules by the inclusion of nontraditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

An additional embodiment of the invention encompasses a method for screening for a compound which is effective in altering expression of a polynucleotide encoding GCREC. Compounds which may be effective in altering expression of a specific polynucleotide may include, but are not limited to, oligonucleotides, antisense oligonucleotides, triple helix-forming oligonucleotides, transcription factors and other polypeptide transcriptional regulators, and non-macromolecular chemical entities which are capable of interacting with specific polynucleotide sequences. Effective compounds may alter polynucleotide expression by acting as either inhibitors or promoters of polynucleotide expression. Thus, in the treatment of disorders associated with increased GCREC expression or activity, a compound which specifically inhibits expression of the polynucleotide encoding GCREC may be therapeutically useful, and in the treatment of disorders associated with decreased GCREC expression or activity, a compound which specifically promotes expression of the polynucleotide encoding GCREC may be therapeutically useful.

At least one, and up to a plurality, of test compounds may be screened for effectiveness in altering expression of a specific polynucleotide. A test compound may be obtained by any method commonly known in the art, including chemical modification of a compound known to be effective in altering polynucleotide expression; selection from an existing, commercially-available or proprietary library of naturally-occurring or non-natural chemical compounds; rational design of a compound based on chemical and/or structural properties of the target polynucleotide; and selection from a library of chemical compounds created combinatorially or randomly. A sample comprising a polynucleotide encoding GCREC is exposed to at least one test compound thus obtained. The sample may comprise, for example, an intact or permeabilized cell, or an in vitro cell-free or reconstituted biochemical system. Alterations in the expression of a polynucleotide encoding GCREC are assayed by any method commonly known in the art. Typically, the expression of a specific nucleotide is detected by hybridization with a probe having a nucleotide sequence complementary to the sequence of the polynucleotide encoding GCREC. The amount of hybridization may be quantified, thus forming the basis for a comparison of the expression of the polynucleotide both with and without exposure to one or more test compounds. Detection of a change in the expression of a polynucleotide exposed to a test compound indicates that the test compound is effective in altering the expression of the polynucleotide. A screen for a compound effective in altering expression of a specific polynucleotide can be carried out, for example, using a Schizosaccharomyces pombe gene expression system (Atkins, D. et al. (1999) U.S. Patent No. 5,932,435; Amdt, G.M. et al. (2000) Nucleic Acids Res. 28:E15) or a human cell line such as HeLa cell (Clarke, M.L. et al. (2000) Biochem. Biophys. Res. Commun. 268:8-13). A particular embodiment of the present invention involves screening a combinatorial library of oligonucleotides (such as deoxyribonucleotides, ribonucleotides, peptide nucleic acids, and modified oligonucleotides) for antisense activity against a specific polynucleotide sequence (Bruice, T.W. et al. (1997) U.S. Patent No. 5,686,242; Bruice, T.W. et al. (2000) U.S. Patent No. 6,022,691).

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection, by liposome injections, or by polycationic amino polymers may be achieved using methods which are well known in the art. (See, e.g., Goldman, C.K. et al. (1997) Nat. Biotechnol. 15:462-466.)

Any of the therapeutic methods described above may be applied to any subject in need of such therapy, including, for example, mammals such as humans, dogs, cats, cows, horses, rabbits, and monkeys.

An additional embodiment of the invention relates to the administration of a composition which generally comprises an active ingredient formulated with a pharmaceutically acceptable excipient. Excipients may include, for example, sugars, starches, celluloses, gums, and proteins. Various formulations are commonly known and are thoroughly discussed in the latest edition of Remington's Pharmaceutical Sciences (Maack Publishing, Easton PA). Such compositions may consist of GCREC, antibodies to GCREC, and mimetics, agonists, antagonists, or inhibitors of GCREC.

The compositions utilized in this invention may be administered by any number of routes including, but not limited to, oral, intravenous, intramuscular, intra-arterial, intramedullary, intrathecal, intraventricular, pulmonary, transdermal, subcutaneous, intraperitoneal, intranasal, enteral, topical, sublingual, or rectal means.

Compositions for pulmonary administration may be prepared in liquid or dry powder form. These compositions are generally aerosolized immediately prior to inhalation by the patient. In the case of small molecules (e.g. traditional low molecular weight organic drugs), aerosol delivery of fast-acting formulations is well-known in the art. In the case of macromolecules (e.g. larger peptides and proteins), recent developments in the field of pulmonary delivery via the alveolar region of the lung have enabled the practical delivery of drugs such as insulin to blood circulation (see, e.g., Patton, J.S. et al., U.S. Patent No. 5,997,848). Pulmonary delivery has the advantage of administration without needle injection, and obviates the need for potentially toxic penetration enhancers.

Compositions suitable for use in the invention include compositions wherein the active ingredients are contained in an effective amount to achieve the intended purpose. The determination of an effective dose is well within the capability of those skilled in the art.

Specialized forms of compositions may be prepared for direct intracellular delivery of macromolecules comprising GCREC or fragments thereof. For example, liposome preparations containing a cell-impermeable macromolecule may promote cell fusion and intracellular delivery of the macromolecule. Alternatively, GCREC or a fragment thereof may be joined to a short cationic N-terminal portion from the HIV Tat-1 protein. Fusion proteins thus generated have been found to transduce into the cells of all tissues, including the brain, in a mouse model system (Schwarze, S.R. et al. (1999) Science 285:1569-1572).

For any compound, the therapeutically effective dose can be estimated initially either in cell culture assays, e.g., of neoplastic cells, or in animal models such as mice, rats, rabbits, dogs, monkeys, or pigs. An animal model may also be used to determine the appropriate concentration range and route of administration. Such information can then be used to determine useful doses and routes for administration in humans.

A therapeutically effective dose refers to that amount of active ingredient, for example GCREC or fragments thereof, antibodies of GCREC, and agonists, antagonists or inhibitors of GCREC, which ameliorates the symptoms or condition. Therapeutic efficacy and toxicity may be determined by standard pharmaceutical procedures in cell cultures or with experimental animals, such as by calculating the $ED_{50}$ (the dose therapeutically effective in 50% of the population) or $LD_{50}$ (the dose lethal to 50% of the population) statistics. The dose ratio of toxic to therapeutic effects is the therapeutic index, which can be expressed as the $LD_{50}/ED_{50}$ ratio. Compositions which exhibit large therapeutic indices are preferred.

The data obtained from cell culture assays and animal studies are used to formulate a range of dosage for human use. The dosage contained in such compositions is preferably within a range of circulating concentrations that includes the $ED_{50}$ with little or no toxicity. The dosage varies within this range depending upon the dosage form employed, the sensitivity of the patient, and the route of administration.

The exact dosage will be determined by the practitioner, in light of factors related to the subject requiring treatment. Dosage and administration are adjusted to provide sufficient levels of the active moiety or to maintain the desired effect. Factors which may be taken into account include the severity of the disease state, the general health of the subject, the age, weight, and gender of the subject, time and frequency of administration, drug combination(s), reaction sensitivities, and response to therapy. Long-acting compositions may be administered every 3 to 4 days, every week, or biweekly depending on the half-life and clearance rate of the particular formulation.

Normal dosage amounts may vary from about 0.1 μg to 100,000 μg, up to a total dose of about 1 gram, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

**DIAGNOSTICS**

In another embodiment, antibodies which specifically bind GCREC may be used for the diagnosis of disorders characterized by expression of GCREC, or in assays to monitor patients being treated with GCREC or agonists, antagonists, or inhibitors of GCREC. Antibodies useful for diagnostic purposes may be prepared in the same manner as described above for therapeutics. Diagnostic assays for GCREC include methods which utilize the antibody and a label to detect GCREC in human body fluids or in extracts of cells or tissues. The antibodies may be used with or without modification, and may be labeled by covalent or non-covalent attachment of a reporter molecule. A wide variety of reporter molecules, several of which are described above, are known in the art and may be used.

A variety of protocols for measuring GCREC, including ELISAs, RIAs, and FACS, are known in the art and provide a basis for diagnosing altered or abnormal levels of GCREC expression. Normal or standard values for GCREC expression are established by combining body fluids or cell extracts taken from normal mammalian subjects, for example, human subjects, with antibodies to GCREC under conditions suitable for complex formation. The amount of standard complex formation may be quantitated by various methods, such as photometric means. Quantities of GCREC expressed in subject, control, and disease samples from biopsied tissues are compared with the standard values. Deviation between standard and subject values establishes the parameters for diagnosing disease.

In another embodiment of the invention, the polynucleotides encoding GCREC may be used for diagnostic purposes. The polynucleotides which may be used include oligonucleotide sequences, complementary RNA and DNA molecules, and PNAs. The polynucleotides may be used to detect and quantify gene expression in biopsied tissues in which expression of GCREC may be correlated with disease. The diagnostic assay may be used to determine absence, presence, and excess expression of GCREC, and to monitor regulation of GCREC levels during therapeutic intervention.

In one aspect, hybridization with PCR probes which are capable of detecting polynucleotide sequences, including genomic sequences, encoding GCREC or closely related molecules may be used to identify nucleic acid sequences which encode GCREC. The specificity of the probe, whether it is made from a highly specific region, e.g., the 5'regulatory region, or from a less specific region, e.g., a conserved motif, and the stringency of the hybridization or amplification will determine whether the probe identifies only naturally occurring sequences encoding GCREC, allelic variants, or related sequences.

Probes may also be used for the detection of related sequences, and may have at least 50% sequence identity to any of the GCREC encoding sequences. The hybridization probes of the subject invention may be DNA or RNA and may be derived from the sequence of SEQ ID NO:11-20 or from genomic sequences including promoters, enhancers, and introns of the GCREC gene.

Means for producing specific hybridization probes for DNAs encoding GCREC include the cloning of polynucleotide sequences encoding GCREC or GCREC derivatives into vectors for the production of mRNA probes. Such vectors are known in the art, are commercially available, and may be used to synthesize RNA probes in vitro by means of the addition of the appropriate RNA polymerases and the appropriate labeled nucleotides. Hybridization probes may be labeled by a variety of reporter groups, for example, by radionuclides such as $^{32}P$ or $^{35}S$, or by enzymatic labels, such as alkaline phosphatase coupled to the probe via avidin/biotin coupling systems, and the like.

Polynucleotide sequences encoding GCREC may be used for the diagnosis of disorders associated with expression of GCREC. Examples of such disorders include, but are not limited to, a cell proliferative disorder such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and; in particular, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus; a neurological disorder such as epilepsy, ischemic cerebrovascular disease, stroke, cerebral neoplasms, Alzheimer's disease, Pick's disease, Huntington's disease, dementia, Parkinson's disease and other extrapyramidal disorders, amyotrophic lateral sclerosis and other motor neuron disorders, progressive neural muscular atrophy, retinitis pigmentosa, hereditary ataxias, multiple sclerosis and other demyelinating diseases, bacterial and viral meningitis, brain abscess, subdural empyema, epidural abscess, suppurative intracranial thrombophlebitis, myelitis and radiculitis, viral central nervous system disease, prion diseases including kuru, Creutzfeldt-Jakob disease, and Gerstmann-Straussler-Scheinker syndrome, fatal familial insomnia, nutritional and metabolic diseases of the nervous system, neurofibromatosis, tuberous sclerosis, cerebelloretinal hemangioblastomatosis, encephalotrigeminal syndrome, mental retardation and other developmental disorders of the central nervous system, cerebral palsy, neuroskeletal disorders, autonomic nervous system disorders, cranial nerve disorders, spinal cord diseases, muscular dystrophy and other neuromuscular disorders, peripheral nervous system disorders, dermatomyositis and polymyositis, inherited, metabolic, endocrine, and toxic myopathies, myasthenia gravis, periodic paralysis, mental disorders including mood, anxiety, and schizophrenic disorders, seasonal affective disorder (SAD), akathesia, amnesia, catatonia, diabetic neuropathy, tardive dyskinesia, dystonias, paranoid psychoses, postherpetic neuralgia, Tourette's disorder, progressive supranuclear palsy, corticobasal degeneration, and familial frontotemporal dementia; a cardiovascular disorder such as arteriovenous fistula, atherosclerosis, hypertension, vasculitis, Raynaud's disease, aneurysms, arterial dissections, varicose veins, thrombophlebitis and phlebothrombosis, vascular tumors, complications of thrombolysis, balloon angioplasty, vascular replacement, and coronary artery bypass graft surgery, congestive heart failure, ischemic heart disease, angina pectoris, myocardial infarction, hypertensive heart disease, degenerative valvular heart disease, calcific aortic valve stenosis, congenitally bicuspid aortic valve, mitral annular calcification, mitral valve prolapse, rheumatic fever and rheumatic heart disease, infective endocarditis, nonbacterial thrombotic endocarditis, endocarditis of systemic lupus erythematosus, carcinoid heart disease, cardiomyopathy, myocarditis, pericarditis, neoplastic heart disease, congenital heart disease, and complications of cardiac transplantation; a gastrointestinal disorder such as dysphagia, peptic esophagitis, esophageal spasm, esophageal stricture, esophageal carcinoma, dyspepsia, indigestion, gastritis, gastric carcinoma, anorexia, nausea, emesis, gastroparesis, antral or pyloric edema, abdominal angina, pyrosis, gastroenteritis, intestinal obstruction, infections of the intestinal tract, peptic ulcer, cholelithiasis, cholecystitis, cholestasis, pancreatitis, pancreatic carcinoma, biliary tract disease, hepatitis, hyperbilirubinemia, cirrhosis, passive congestion of the liver, hepatoma, infectious colitis, ulcerative colitis, ulcerative proctitis, Crohn's disease, Whipple's disease, Mallory-Weiss syndrome, colonic carcinoma, colonic obstruction, irritable bowel syndrome, short bowel syndrome, diarrhea, constipation, gastrointestinal hemorrhage, acquired immunodeficiency syndrome (AIDS) enteropathy, jaundice, hepatic encephalopathy, hepatorenal syndrome, hepatic steatosis, hemochromatosis, Wilson's disease, $alpha_1$-antitrypsin deficiency, Reye's syndrome, primary sclerosing cholangitis, liver infarction, portal vein obstruction and thrombosis, centrilobular necrosis, peliosis hepatis, hepatic vein thrombosis, veno-occlusive disease, preeclampsia, eclampsia, acute fatty liver of pregnancy, intrahepatic cholestasis of pregnancy, and hepatic tumors including nodular hyperplasias, adenomas, and carcinomas; an autoimmune/inflammatory disorder such as acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, autoimmune polyendocrinopathy-candidiasis-ectodermal dystrophy (APECED), bronchitis, cholecystitis, contact dermatitis, Crohn's disease, atopic dermatitis, dermatomyositis, diabetes mellitus, emphysema, episodic lymphopenia with lymphocytotoxins, erythroblastosis fetalis, erythema nodosum, atrophic gastritis, glomerulonephritis, Goodpasture's syndrome, gout, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polymyositis, psoriasis, Reiter's syndrome, rheumatoid arthritis, scleroderma, Sjögren's syndrome, systemic anaphylaxis, systemic lupus erythematosus, systemic sclerosis, thrombocytopenic purpura, ulcerative colitis, uveitis, Werner syndrome, complications of cancer, hemodialysis, and extracorporeal circulation, viral, bacterial, fungal, parasitic, protozoal, and helminthic infections, and trauma; a metabolic disorder such as diabetes, obesity, and osteoporosis; and an infection by a viral agent classified as adenovirus, arenavirus, bunyavirus, calicivirus, coronavirus, filovirus, hepadnavirus, herpesvirus, flavivirus, orthomyxovirus, parvovirus, papovavirus, paramyxovirus, picornavirus, poxvirus, reovirus, retrovirus, rhabdovirus, and tongavirus. The polynucleotide sequences encoding GCREC may be used in Southern or northern analysis, dot blot, or other membrane-based technologies; in PCR technologies; in dipstick, pin, and multiformat ELISA-like assays; and in microarrays utilizing fluids or tissues from patients to detect altered GCREC expression. Such qualitative or quantitative methods are well known in the art.

In a particular aspect, the nucleotide sequences encoding GCREC may be useful in assays that detect the presence of associated disorders, particularly those mentioned above. The nucleotide sequences encoding GCREC may be labeled by standard methods and added to a fluid or tissue sample from a patient under conditions suitable for the formation of hybridization complexes. After a suitable incubation period, the sample is washed and the signal is quantified and compared with a standard value. If the amount of signal in the patient sample is significantly altered in comparison to a control sample then the presence of altered levels of nucleotide sequences encoding GCREC in the sample indicates the presence of the associated disorder. Such assays may also be used to evaluate the efficacy of a particular therapeutic treatment regimen in animal studies, in clinical trials, or to monitor the treatment of an individual patient.

In order to provide a basis for the diagnosis of a disorder associated with expression of GCREC, a normal or standard profile for expression is established. This may be accomplished by combining body fluids or cell extracts taken from normal subjects, either animal or human, with a sequence, or a fragment thereof, encoding GCREC, under conditions suitable for hybridization or amplification. Standard hybridization may be quantified by comparing the values obtained from normal subjects with values from an experiment in which a known amount of a substantially purified polynucleotide is used. Standard values obtained in this manner may be compared with values obtained from samples from patients who are symptomatic for a disorder. Deviation from standard values is used to establish the presence of a disorder.

Once the presence of a disorder is established and a treatment protocol is initiated, hybridization assays may be repeated on a regular basis to determine if the level of expression in the patient begins to approximate that which is observed in the normal subject. The results obtained from successive assays may be used to show the efficacy of treatment over a period ranging from several days to months.

With respect to cancer, the presence of an abnormal amount of transcript (either under- or overexpressed) in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Additional diagnostic uses for oligonucleotides designed from the sequences encoding GCREC may involve the use of PCR. These oligomers may be chemically synthesized, generated enzymatically, or produced in vitro. Oligomers will preferably contain a fragment of a polynucleotide encoding GCREC, or a fragment of a polynucleotide complementary to the polynucleotide encoding GCREC, and will be employed under optimized conditions for identification of a specific gene or condition. Oligomers may also be employed under less stringent conditions for detection or quantification of closely related DNA or RNA sequences.

In a particular aspect, oligonucleotide primers derived from the polynucleotide sequences encoding GCREC may be used to detect single nucleotide polymorphisms (SNPs). SNPs are substitutions, insertions and deletions that are a frequent cause of inherited or acquired genetic disease in humans. Methods of SNP detection include, but are not limited to, single-stranded conformation polymorphism (SSCP) and fluorescent SSCP (fSSCP) methods. In SSCP, oligonucleotide primers derived from the polynucleotide sequences encoding GCREC are used to amplify DNA using the polymerase chain reaction (PCR). The DNA may be derived, for example, from diseased or normal tissue, biopsy samples, bodily fluids, and the like. SNPs in the DNA cause differences in the secondary and tertiary structures of PCR products in single-stranded form, and these differences are detectable using gel electrophoresis in non-denaturing gels. In fSCCP, the oligonucleotide primers are fluorescently labeled, which allows detection of the amplimers in high-throughput equipment such as DNA sequencing machines. Additionally, sequence database analysis methods, termed in silico SNP (isSNP), are capable of identifying polymorphisms by comparing the sequence of individual overlapping DNA fragments which assemble into a common consensus sequence. These computer-based methods filter out sequence variations due to laboratory preparation of DNA and sequencing errors using statistical models and automated analyses of DNA sequence chromatograms. In the alternative, SNPs may be detected and characterized by mass spectrometry using, for example, the high throughput MASSARRAY system (Sequenom, Inc., San Diego CA).

Methods which may also be used to quantify the expression of GCREC include radiolabeling or biotinylating nucleotides, coamplification of a control nucleic acid, and interpolating results from standard curves. (See, e.g., Melby, P.C. et al. (1993) J. Immunol. Methods 159:235-244; Duplaa, C. et al. (1993) Anal. Biochem. 212:229-236.) The speed of quantitation of multiple samples may be accelerated by running the assay in a high-throughput format where the oligomer or polynucleotide of interest is presented in various dilutions and a spectrophotometric or colorimetric response gives rapid quantitation.

In further embodiments, oligonucleotides or longer fragments derived from any of the polynucleotide sequences described herein may be used as elements on a microarray. The microarray can be used in transcript imaging techniques which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents which are highly effective and display the fewest side effects may be selected for a patient based on his/her pharmacogenomic profile.

In another embodiment, GCREC, fragments of GCREC, or antibodies specific for GCREC may be used as elements on a microarray. The microarray may be used to monitor or measure protein-protein interactions, drug-target interactions, and gene expression profiles, as described above.

A particular embodiment relates to the use of the polynucleotides of the present invention to generate a transcript image of a tissue or cell type. A transcript image represents the global pattern of gene expression by a particular tissue or cell type. Global gene expression patterns are analyzed by quantifying the number of expressed genes and their relative abundance under given conditions and at a given time. (See Seilhamer et al., "Comparative Gene Transcript Analysis," U.S. Patent Number 5,840,484, expressly incorporated by reference herein.) Thus a transcript image may be generated by hybridizing the polynucleotides of the present invention or their complements to the totality of transcripts or reverse transcripts of a particular tissue or cell type. In one embodiment, the hybridization takes place in high-throughput format, wherein the polynucleotides of the present invention or their complements comprise a subset of a plurality of elements on a microarray. The resultant transcript image would provide a profile of gene activity.

Transcript images may be generated using transcripts isolated from tissues, cell lines, biopsies, or other biological samples. The transcript image may thus reflect gene expression in vivo, as in the case of a tissue or biopsy sample, or in vitro, as in the case of a cell line.

Transcript images which profile the expression of the polynucleotides of the present invention may also be used in conjunction with in vitro model systems and preclinical evaluation of pharmaceuticals, as well as toxicological testing of industrial and naturally-occurring environmental compounds. All compounds induce characteristic gene expression patterns, frequently termed molecular fingerprints or toxicant signatures, which are indicative of mechanisms of action and toxicity (Nuwaysir, E.F. et al. (1999) Mol. Carcinog. 24:153-159; Steiner, S. and N.L. Anderson (2000) Toxicol. Lett. 112-113:467-471, expressly incorporated by reference herein). If a test compound has a signature similar to that of a compound with known toxicity, it is likely to share those toxic properties. These fingerprints or signatures are most useful and refined when they contain expression information from a large number of genes and gene families. Ideally, a genome-wide measurement of expression provides the highest quality signature. Even genes whose expression is not altered by any tested compounds are important as well, as the levels of expression of these genes are used to normalize the rest of the expression data. The normalization procedure is useful for comparison of expression data after treatment with different compounds. While the assignment of gene function to elements of a toxicant signature aids in interpretation of toxicity mechanisms, knowledge of gene function is not necessary for the statistical matching of signatures which leads to prediction of toxicity. (See, for example, Press Release 00-02 from the National Institute of Environmental Health Sciences, released February 29, 2000, available at http://www.niehs.nih.gov/oc/news/toxchip.htm.) Therefore, it is important and desirable in toxicological screening using toxicant signatures to include all expressed gene sequences.

In one embodiment, the toxicity of a test compound is assessed by treating a biological sample containing nucleic acids with the test compound. Nucleic acids that are expressed in the treated biological sample are hybridized with one or more probes specific to the polynucleotides of the present invention, so that transcript levels corresponding to the polynucleotides of the present invention may be quantified. The transcript levels in the treated biological sample are compared with levels in an untreated biological sample. Differences in the transcript levels between the two samples are indicative of a toxic response caused by the test compound in the treated sample.

Another particular embodiment relates to the use of the polypeptide sequences of the present invention to analyze the proteome of a tissue or cell type. The term proteome refers to the global pattern of protein expression in a particular tissue or cell type. Each protein component of a proteome can be subjected individually to further analysis. Proteome expression patterns, or profiles, are analyzed by quantifying the number of expressed proteins and their relative abundance under given conditions and at a given time. A profile of a cell's proteome may thus be generated by separating and analyzing the polypeptides of a particular tissue or cell type. In one embodiment, the separation is achieved using two-dimensional gel electrophoresis, in which proteins from a sample are separated by isoelectric focusing in the first dimension, and then according to molecular weight by sodium dodecyl sulfate slab gel electrophoresis in the second dimension (Steiner and Anderson, supra). The proteins are visualized in the gel as discrete and uniquely positioned spots, typically by staining the gel with an agent such as Coomassie Blue or silver or fluorescent stains. The optical density of each protein spot is generally proportional to the level of the protein in the sample. The optical densities of equivalently positioned protein spots from different samples, for example, from biological samples either treated or untreated with a test compound or therapeutic agent, are compared to identify any changes in protein spot density related to the treatment. The proteins in the spots are partially sequenced using, for example, standard methods employing chemical or enzymatic cleavage followed by mass spectrometry. The identity of the protein in a spot may be determined by comparing its partial sequence, preferably of at least 5 contiguous amino acid residues, to the polypeptide sequences of the present invention. In some cases, further sequence data may be obtained for definitive protein identification.

A proteomic profile may also be generated using antibodies specific for GCREC to quantify the levels of GCREC expression. In one embodiment, the antibodies are used as elements on a microarray, and protein expression levels are quantified by exposing the microarray to the sample and detecting the levels of protein bound to each array element (Lueking, A. et al. (1999) Anal. Biochem. 270:103-111; Mendoze, L.G. et al. (1999) Biotechniques 27:778-788). Detection may be performed by a variety of methods known in the art, for example, by reacting the proteins in the sample with a thiol- or amino-reactive fluorescent compound and detecting the amount of fluorescence bound at each array element.

Toxicant signatures at the proteome level are also useful for toxicological screening, and should be analyzed in parallel with toxicant signatures at the transcript level. There is a poor correlation between transcript and protein abundances for some proteins in some tissues (Anderson, N.L. and J. Seilhamer (1997) Electrophoresis 18:533-537), so proteome toxicant signatures may be useful in the analysis of compounds which do not significantly affect the transcript image, but which alter the proteomic profile. In addition, the analysis of transcripts in body fluids is difficult, due to rapid degradation of mRNA, so proteomic profiling may be more reliable and informative in such cases.

In another embodiment, the toxicity of a test compound is assessed by treating a biological sample containing proteins with the test compound. Proteins that are expressed in the treated biological sample are separated so that the amount of each protein can be quantified. The amount of each protein is compared to the amount of the corresponding protein in an untreated biological sample. A difference in the amount of protein between the two samples is indicative of a toxic response to the test compound in the treated sample. Individual proteins are identified by sequencing the amino acid residues of the individual proteins and comparing these partial sequences to the polypeptides of the present invention.

In another embodiment, the toxicity of a test compound is assessed by treating a biological sample containing proteins with the test compound. Proteins from the biological sample are incubated with antibodies specific to the polypeptides of the present invention. The amount of protein recognized by the antibodies is quantified. The amount of protein in the treated biological sample is compared with the amount in an untreated biological sample. A difference in the amount of protein between the two samples is indicative of a toxic response to the test compound in the treated sample.

Microarrays may be prepared, used, and analyzed using methods known in the art. (See, e.g., Brennan, T.M. et al. (1995) U.S. Patent No. 5,474,796; Schena, M. et al. (1996) Proc. Natl. Acad. Sci. USA 93:10614-10619; Baldeschweiler et al. (1995) PCT application WO95/251116; Shalon, D. et al. (1995) PCT application WO95/35505; Heller, R.A. et al. (1997) Proc. Natl. Acad. Sci. USA 94:2150-2155; and Heller, M.J. et al. (1997) U.S. Patent No. 5,605,662.) Various types of microarrays are well known and thoroughly described in DNA Microarrays: A Practical Approach, M. Schena, ed. (1999) Oxford University Press, London, hereby expressly incorporated by reference.

In another embodiment of the invention, nucleic acid sequences encoding GCREC may be used to generate hybridization probes useful in mapping the naturally occurring genomic sequence. Either coding or noncoding sequences may be used, and in some instances, noncoding sequences may be preferable over coding sequences. For example, conservation of a coding sequence among members of a multi-gene family may potentially cause undesired cross hybridization during chromosomal mapping. The sequences may be mapped to a particular chromosome, to a specific region of a chromosome, or to artificial chromosome constructions, e.g., human artificial chromosomes (HACs), yeast artificial chromosomes (YACs), bacterial artificial chromosomes (BACs), bacterial P1 constructions, or single chromosome cDNA libraries. (See, e.g., Harrington, J.J. et al. (1997) Nat. Genet. 15:345-355; Price, C.M. (1993) Blood Rev. 7:127-134; and Trask, B.J. (1991) Trends Genet. 7:149-154.) Once mapped, the nucleic acid sequences of the invention may be used to develop genetic linkage maps, for example, which correlate the inheritance of a disease state with the inheritance of a particular chromosome region or restriction fragment length polymorphism (RFLP). (See, for example, Lander, E.S. and D. Botstein (1986) Proc. Natl. Acad. Sci. USA 83:7353-7357.)

Fluorescent in situ hybridization (FISH) may be correlated with other physical and genetic map data. (See, e.g., Heinz-Ulrich, et al. (1995) in Meyers, supra, pp. 965-968.) Examples of genetic map data can be found in various scientific journals or at the Online Mendelian Inheritance in Man (OMIM) World Wide Web site. Correlation between the location of the gene encoding GCREC on a physical map and a specific disorder, or a predisposition to a specific disorder, may help define the region of DNA associated with that disorder and thus may further positional cloning efforts.

In situ hybridization of chromosomal preparations and physical mapping techniques, such as linkage analysis using established chromosomal markers, may be used for extending genetic maps. Often the placement of a gene on the chromosome of another mammalian species, such as mouse, may reveal associated markers even if the exact chromosomal locus is not known. This information is valuable to investigators searching for disease genes using positional cloning or other gene discovery techniques. Once the gene or genes responsible for a disease or syndrome have been crudely localized by genetic linkage to a particular genomic region, e.g., ataxia-telangiectasia to 11q22-23, any sequences mapping to that area may represent associated or regulatory genes for further investigation. (See, e.g., Gatti, R.A. et al. (1988) Nature 336:577-580.) The nucleotide sequence of the instant invention may also be used to detect differences in the chromosomal location due to translocation, inversion, etc., among normal, carrier, or affected individuals.

In another embodiment of the invention, GCREC, its catalytic or immunogenic fragments, or oligopeptides thereof can be used for screening libraries of compounds in any of a variety of drug screening techniques. The fragment employed in such screening may be free in solution, affixed to a solid support, borne on a cell surface, or located intracellularly. The formation of binding complexes between GCREC and the agent being tested may be measured.

Another technique for drug screening provides for high throughput screening of compounds having suitable binding affinity to the protein of interest. (See, e.g., Geysen, et al. (1984) PCT application WO84/03564.) In this method, large numbers of different small test compounds are synthesized on a solid substrate. The test compounds are reacted with GCREC, or fragments thereof, and washed. Bound GCREC is then detected by methods well known in the art. Purified GCREC can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding GCREC specifically compete with a test compound for binding GCREC. In this manner, antibodies can be used to detect the presence of any peptide which shares one or more antigenic determinants with GCREC.

In additional embodiments, the nucleotide sequences which encode GCREC may be used in any molecular biology techniques that have yet to be developed, provided the new techniques rely on properties of nucleotide sequences that are currently known, including, but not limited to, such properties as the triplet genetic code and specific base pair interactions.

Without further elaboration, it is believed that one skilled in the art can, using the preceding description, utilize the present invention to its fullest extent. The following embodiments are, therefore, to be construed as merely illustrative, and not limitative of the remainder of the disclosure in any way whatsoever.

The disclosures of all patents, applications and publications, mentioned above and below, including U.S. Ser. No. 60/212,483, U.S. Ser. No. 60/213,950, U.S. Ser. No. 60/214,062, U.S. Ser. No. 60/216,595, U.S. Ser. No. 60/218,936, and U.S. Ser. No. 60/219,154, are expressly incorporated by reference herein.

## EXAMPLES

### I. Construction of cDNA Libraries

Incyte cDNAs were derived from cDNA libraries described in the LIFESEQ GOLD database (Incyte Genomics, Palo Alto CA) and shown in Table 4, column 5. Some tissues were homogenized and lysed in guanidinium isothiocyanate, while others were homogenized and lysed in phenol or in a suitable mixture of denaturants, such as TRIZOL (Life Technologies), a monophasic solution of phenol and guanidine isothiocyanate. The resulting lysates were centrifuged over CsCl cushions or extracted with chloroform. RNA was precipitated from the lysates with either isopropanol or sodium acetate and ethanol, or by other routine methods.

Phenol extraction and precipitation of RNA were repeated as necessary to increase RNA purity. In some cases, RNA was treated with DNase. For most libraries, poly(A)+ RNA was isolated using oligo d(T)-coupled paramagnetic particles (Promega), OLIGOTEX latex particles (QIAGEN, Chatsworth CA), or an OLIGOTEX mRNA purification kit (QIAGEN). Alternatively, RNA was isolated directly from tissue lysates using other RNA isolation kits, e.g., the POLY (A)PURE mRNA purification kit (Ambion, Austin TX).

In some cases, Stratagene was provided with RNA and constructed the corresponding cDNA libraries. Otherwise, cDNA was synthesized and cDNA libraries were constructed with the UNIZAP vector system (Stratagene) or SUPERSCRIPT plasmid system (Life Technologies), using the recommended procedures or similar methods known in the art. (See, e.g., Ausubel, 1997, supra, units 5.1-6.6.) Reverse transcription was initiated using oligo d(T) or random primers. Synthetic oligonucleotide adapters were ligated to double stranded cDNA, and the cDNA was digested with the appropriate restriction enzyme or enzymes. For most libraries, the cDNA was size-selected (300-1000 bp) using SEPHACRYL S1000, SEPHAROSE CL2B, or SEPHAROSE CL4B column chromatography (Amersham Pharmacia Biotech) or preparative agarose gel electrophoresis. cDNAs were ligated into compatible restriction enzyme sites of the polylinker of a suitable plasmid, e.g., PBLUESCRIPT plasmid (Stratagene), PSPORT1 plasmid (Life Technologies), PCDNA2.1 plasmid (Invitrogen, Carlsbad CA), PBK-CMV plasmid (Stratagene), or pINCY (Incyte Genomics, Palo Alto CA), or derivatives thereof. Recombinant plasmids were transformed into competent E. coli cells including XL1-Blue, XL1-BlueMRF, or SOLR from Stratagene or DH5a, DH10B, or ElectroMAX DH10B from Life Technologies.

### II. Isolation of cDNA Clones

Plasmids obtained as described in Example I were recovered from host cells by in vivo excision using the UNIZAP vector system (Stratagene) or by cell lysis. Plasmids were purified using at least one of the following: a Magic or WIZARD Minipreps DNA purification system (Promega); an AGTC Miniprep purification kit (Edge Biosystems, Gaithersburg MD); and QIAWELL 8 Plasmid, QIAWELL 8 Plus Plasmid, QIAWELL 8 Ultra Plasmid purification systems or the R.E.A.L. PREP 96 plasmid purification kit from QIAGEN. Following precipitation, plasmids were resuspended in 0.1 ml of distilled water and stored, with or without lyophilization, at 4°C.

Alternatively, plasmid DNA was amplified from host cell lysates using direct link PCR in a high-throughput format (Rao, V.B. (1994) Anal. Biochem. 216:1-14). Host cell lysis and thermal cycling steps were carried out in a single reaction mixture. Samples were processed and stored in 384-well plates, and the concentration of amplified plasmid DNA was quantified fluorometrically using PICOGREEN dye (Molecular Probes, Eugene OR) and a FLUOROSKAN II fluorescence scanner (Labsystems Oy, Helsinki, Finland).

### III. Sequencing and Analysis

Incyte cDNA recovered in plasmids as described in Example II were sequenced as follows. Sequencing reactions were processed using standard methods or high-throughput instrumentation such as the ABI CATALYST 800 (Applied Biosystems) thermal cycler or the PTC-200 thermal cycler (MJ Research) in conjunction with the HYDRA microdispenser (Robbins Scientific) or the MICROLAB 2200 (Hamilton) liquid transfer system. cDNA sequencing reactions were prepared using reagents provided by Amersham Pharmacia Biotech or supplied in ABI sequencing kits such as the ABI PRISM BIGDYE Terminator cycle sequencing ready reaction kit (Applied Biosystems). Electrophoretic separation of cDNA sequencing reactions and detection of labeled polynucleotides were carried out using the MEGABACE 1000 DNA sequencing system (Molecular Dynamics); the ABI PRISM 373 or 377 sequencing system (Applied Biosystems) in conjunction with standard ABI protocols and base calling software; or other sequence analysis systems known in the art. Reading frames within the cDNA sequences were identified using standard methods (reviewed in Ausubel, 1997, supra, unit 7.7). Some of the cDNA sequences were selected for extension using the techniques disclosed in Example VIII.

The polynucleotide sequences derived from Incyte cDNAs were validated by removing vector, linker, and poly (A) sequences and by masking ambiguous bases, using algorithms and programs based on BLAST, dynamic programming, and dinucleotide nearest neighbor analysis. The Incyte cDNA sequences or translations thereof were then queried against a selection of public databases such as the GenBank primate, rodent, mammalian, vertebrate, and eukaryote databases, and BLOCKS, PRINTS, DOMO, PRODOM, and hidden Markov model (HMM)-based protein family databases such as PFAM. (HMM is a probabilistic approach which analyzes consensus primary structures of gene families. See, for example, Eddy, S.R. (1996) Curr. Opin. Struct. Biol. 6:361-365.) The queries were performed using programs based on BLAST, FASTA, BLIMPS, and HMMER. The Incyte cDNA sequences were assembled to produce full length polynucleotide sequences. Alternatively, GenBank cDNAs, GenBank ESTs, stitched sequences, stretched sequences, or Genscan-predicted coding sequences (see Examples IV and V) were used to extend Incyte cDNA assemblages to full length. Assembly was performed using programs based on Phred, Phrap, and Consed, and cDNA assemblages were screened for open reading frames using programs based on GeneMark, BLAST, and FASTA. The full length polynucleotide sequences were translated to derive the corresponding full length polypeptide sequences. Alternatively, a polypeptide of the invention may begin at any of the methionine residues of the full length translated polypeptide. Full length polypeptide sequences were subsequently analyzed by querying against databases such as the GenBank protein databases (genpept), SwissProt, BLOCKS, PRINTS, DOMO, PRODOM, Prosite, and hidden Markov model (HMM)-based protein family databases such as PFAM. Full length polynucleotide sequences are also analyzed using MACDNASIS PRO software (Hitachi Software Engineering, South San Francisco CA) and LASERGENE software (DNASTAR). Polynucleotide and polypeptide sequence alignments are generated using default parameters specified by the CLUSTAL algorithm as incorporated into the MEGALIGN multisequence alignment program (DNASTAR), which also calculates the percent identity between aligned sequences.

Table 7 summarizes the tools, programs, and algorithms used for the analysis and assembly of Incyte cDNA and full length sequences and provides applicable descriptions, references, and threshold parameters. The first column of Table 7 shows the tools, programs, and algorithms used, the second column provides brief descriptions thereof, the third column presents appropriate references, all of which are incorporated by reference herein in their entirety, and the fourth column presents, where applicable, the scores, probability values, and other parameters used to evaluate the strength of a match between two sequences (the higher the score or the lower the probability value, the greater the identity between two sequences).

The programs described above for the assembly and analysis of full length polynucleotide and polypeptide sequences were also used to identify polynucleotide sequence fragments from SEQ ID NO:11-20. Fragments from about 20 to about 4000 nucleotides which are useful in hybridization and amplification technologies are described in Table 4, column 4.

### IV. Identification and Editing of Coding Sequences from Genomic DNA

Putative G-protein coupled receptors were initially identified by running the Genscan gene identification program against public genomic sequence databases (e.g., gbpri and gbhtg). Genscan is a general-purpose gene identification program which analyzes genomic DNA sequences from a variety of organisms (See Burge, C. and S. Karlin (1997) J. Mol. Biol. 268:78-94, and Burge, C. and S. Karlin (1998) Curr. Opin. Struct. Biol. 8:346-354). The program concatenates predicted exons to form an assembled cDNA sequence extending from a methionine to a stop codon. The output of Genscan is a FASTA database of polynucleotide and polypeptide sequences. The maximum range of sequence for Genscan to analyze at once was set to 30 kb. To determine which of these Genscan predicted cDNA sequences encode G-protein coupled receptors, the encoded polypeptides were analyzed by querying against PFAM models for G-protein coupled receptors. Potential G-protein coupled receptors were also identified by homology to Incyte cDNA sequences that had been annotated as G-protein coupled receptors. These selected Genscan-predicted sequences were then compared by BLAST analysis to the genpept and gbpri public databases. Where necessary, the Genscan-predicted sequences were then edited by comparison to the top BLAST hit from genpept to correct errors in the sequence predicted by Genscan, such as extra or omitted exons. BLAST analysis was also used to find any Incyte cDNA or public cDNA coverage of the Genscan-predicted sequences, thus providing evidence for transcription. When Incyte cDNA coverage was available, this information was used to correct or confirm the Genscan predicted sequence. Full length polynucleotide sequences were obtained by assembling Genscan-predicted coding sequences with Incyte cDNA sequences and/or public cDNA sequences using the assembly process described in Example III. Alternatively, full length polynucleotide sequences were derived entirely from edited or unedited Genscan-predicted coding sequences.

### V. Assembly of Genomic Sequence Data with cDNA Sequence Data

#### "Stitched" Sequences

Partial cDNA sequences were extended with exons predicted by the Genscan gene identification program described in Example IV. Partial cDNAs assembled as described in Example III were mapped to genomic DNA and parsed into clusters containing related cDNAs and Genscan exon predictions from one or more genomic sequences. Each cluster was analyzed using an algorithm based on graph theory and dynamic programming to integrate cDNA and genomic information, generating possible splice variants that were subsequently confirmed, edited, or extended to create a full length sequence. Sequence intervals in which the entire length of the interval was present on more than one sequence in the cluster were identified, and intervals thus identified were considered to be equivalent by transitivity. For example, if an interval was present on a cDNA and two genomic sequences, then all three intervals were considered to be equivalent. This process allows unrelated but consecutive genomic sequences to be brought together, bridged by cDNA sequence. Intervals thus identified were then "stitched" together by the stitching algorithm in the order that they appear along their parent sequences to generate the longest possible sequence, as well as sequence variants. Linkages between intervals which proceed along one type of parent sequence (cDNA to cDNA or genomic sequence to genomic sequence) were given preference over linkages which change parent type (cDNA to genomic sequence). The resultant stitched sequences were translated and compared by BLAST analysis to the genpept and gbpri public databases. Incorrect exons predicted by Genscan were corrected by comparison to the top BLAST hit from genpept. Sequences were further extended with additional cDNA sequences, or by inspection of genomic DNA, when necessary.

#### "Stretched" Sequences

Partial DNA sequences were extended to full length with an algorithm based on BLAST analysis. First, partial cDNAs assembled as described in Example III were queried against public databases such as the GenBank primate, rodent, mammalian, vertebrate, and eukaryote databases using the BLAST program. The nearest GenBank protein homolog was then compared by BLAST analysis to either Incyte cDNA sequences or GenScan exon predicted sequences described in Example IV. A chimeric protein was generated by using the resultant high-scoring segment pairs (HSPs) to map the translated sequences onto the GenBank protein homolog. Insertions or deletions may occur in the chimeric protein with respect to the original GenBank protein homolog. The GenBank protein homolog, the chimeric protein, or both were used as probes to search for homologous genomic sequences from the public human genome databases. Partial DNA sequences were therefore "stretched" or extended by the addition of homologous genomic sequences. The resultant stretched sequences were examined to determine whether it contained a complete gene.

### VI. Chromosomal Mapping of GCREC Encoding Polynucleotides

The sequences which were used to assemble SEQ ID NO:11-20 were compared with sequences from the Incyte LIFESEQ database and public domain databases using BLAST and other implementations of the Smith-Waterman algorithm. Sequences from these databases that matched SEQ ID NO:11-20 were assembled into clusters of contiguous and overlapping sequences using assembly algorithms such as Phrap (Table 7). Radiation hybrid and genetic mapping data available from public resources such as the Stanford Human Genome Center (SHGC), Whitehead Institute for Genome Research (WIGR), and Généthon were used to determine if any of the clustered sequences had been previously mapped. Inclusion of a mapped sequence in a cluster resulted in the assignment of all sequences of that cluster, including its particular SEQ ID NO:, to that map location.

Map locations are represented by ranges, or intervals, of human chromosomes. The map position of an interval, in centiMorgans, is measured relative to the terminus of the chromosome's p-arm. (The centiMorgan (cM) is a unit of measurement based on recombination frequencies between chromosomal markers. On average, 1 cM is roughly equivalent to 1 megabase (Mb) of DNA in humans, although this can vary widely due to hot and cold spots of recombination.) The cM distances are based on genetic markers mapped by Généthon which provide boundaries for radiation hybrid markers whose sequences were included in each of the clusters. Human genome maps and other resources available to the public, such as the NCBI "GeneMap'99" World Wide Web site (http://www.ncbi.nlm.nih.gov/genemap/), can be employed to determine if previously identified disease genes map within or in proximity to the intervals indicated above.

### VII. Analysis of Polynucleotide Expression

Northern analysis is a laboratory technique used to detect the presence of a transcript of a gene and involves the hybridization of a labeled nucleotide sequence to a membrane on which RNAs from a particular cell type or tissue have been bound. (See, e.g., Sambrook, supra, ch. 7; Ausubel (1995) supra, ch. 4 and 16.)

Analogous computer techniques applying BLAST were used to search for identical or related molecules in cDNA databases such as GenBank or LIFESEQ (Incyte Genomics). This analysis is much faster than multiple membrane-based hybridizations. In addition, the sensitivity of the computer search can be modified to determine whether any particular match is categorized as exact or similar. The basis of the search is the product score, which is defined as:

$$\frac{\text{BLAST Score x Percent Identity}}{\text{5 x minimum \{length(Seq. 1), length(Seq. 2)\}}}$$

The product score takes into account both the degree of similarity between two sequences and the length of the sequence match. The product score is a normalized value between 0 and 100, and is calculated as follows: the BLAST score is multiplied by the percent nucleotide identity and the product is divided by (5 times the length of the shorter of the two sequences). The BLAST score is calculated by assigning a score of +5 for every base that matches in a high-scoring segment pair (HSP), and -4 for every mismatch. Two sequences may share more than one HSP (separated by gaps). If there is more than one HSP, then the pair with the highest BLAST score is used to calculate the product score. The product score represents a balance between fractional overlap and quality in a BLAST alignment. For example, a product score of 100 is produced only for 100% identity over the entire length of the shorter of the two sequences being compared. A product score of 70 is produced either by 100% identity and 70% overlap at one end, or by 88% identity and 100% overlap at the other. A product score of 50 is produced either by 100% identity and 50% overlap at one end, or 79% identity and 100% overlap.

Alternatively, polynucleotide sequences encoding GCREC are analyzed with respect to the tissue sources from which they were derived. For example, some full length sequences are assembled, at least in part, with overlapping Incyte cDNA sequences (see Example III). Each cDNA sequence is derived from a cDNA library constructed from a human tissue. Each human tissue is classified into one of the following organ/tissue categories: cardiovascular system; connective tissue; digestive system; embryonic structures; endocrine system; exocrine glands; genitalia, female; genitalia, male; germ cells; hemic and immune system; liver; musculoskeletal system; nervous system; pancreas; respiratory system; sense organs; skin; stomatognathic system; unclassified/mixed; or urinary tract. The number of libraries in each category is counted and divided by the total number of libraries across all categories. Similarly, each human tissue is classified into one of the following disease/condition categories: cancer, cell line, developmental, inflammation, neurological, trauma, cardiovascular, pooled, and other, and the number of libraries in each category is counted and divided by the total number of libraries across all categories. The resulting percentages reflect the tissue- and disease-specific expression of cDNA encoding GCREC. cDNA sequences and cDNA library/tissue information are found in the LIFESEQ GOLD database (Incyte Genomics, Palo Alto CA).

### VIII. Extension of GCREC Encoding Polynucleotides

Full length polynucleotide sequences were also produced by extension of an appropriate fragment of the full length molecule using oligonucleotide primers designed from this fragment. One primer was synthesized to initiate 5' extension of the known fragment, and the other primer was synthesized to initiate 3' extension of the known fragment. The initial primers were designed using OLIGO 4.06 software (National Biosciences), or another appropriate program, to be about 22 to 30 nucleotides in length, to have a GC content of about 50% or more, and to anneal to the target sequence at temperatures of about 68°C to about 72°C. Any stretch of nucleotides which would result in hairpin structures and primer-primer dimerizations was avoided.

Selected human cDNA libraries were used to extend the sequence. If more than one extension was necessary or desired, additional or nested sets of primers were designed.

High fidelity amplification was obtained by PCR using methods well known in the art. PCR was performed in 96-well plates using the PTC-200 thermal cycler (MJ Research, Inc.). The reaction mix contained DNA template, 200 nmol of each primer, reaction buffer containing $Mg^{2+}$, $(NH_4)_2SO_4$, and 2-mercaptoethanol, Taq DNA polymerase (Amersham Pharmacia Biotech), ELONGASE enzyme (Life Technologies), and Pfu DNA polymerase (Stratagene), with the following parameters for primer pair PCI A and PCI B: Step 1: 94°C, 3 min; Step 2: 94°C, 15 sec; Step 3: 60°C, 1 min; Step 4: 68°C, 2 min; Step 5: Steps 2, 3, and 4 repeated 20 times; Step 6: 68°C, 5 min; Step 7: storage at 4°C. In the alternative, the parameters for primer pair T7 and SK+ were as follows: Step 1: 94°C, 3 min; Step 2: 94°C, 15 sec; Step 3: 57°C, 1 min; Step 4: 68°C, 2 min; Step 5: Steps 2, 3, and 4 repeated 20 times; Step 6: 68°C, 5 min; Step 7: storage at 4°C.

The concentration of DNA in each well was determined by dispensing 100 μl PICOGREEN quantitation reagent (0.25% (v/v) PICOGREEN; Molecular Probes, Eugene OR) dissolved in 1X TE and 0.5 μl of undiluted PCR product into each well of an opaque fluorimeter plate (Coming Costar, Acton MA), allowing the DNA to bind to the reagent. The plate was scanned in a Fluoroskan II (Labsystems Oy, Helsinki, Finland) to measure the fluorescence of the sample and to quantify the concentration of DNA. A 5 μl to 10 μl aliquot of the reaction mixture was analyzed by electrophoresis on a 1 % agarose gel to determine which reactions were successful in extending the sequence.

The extended nucleotides were desalted and concentrated, transferred to 384-well plates, digested with CviJI cholera virus endonuclease (Molecular Biology Research, Madison WI), and sonicated or sheared prior to religation into pUC 18 vector (Amersham Pharmacia Biotech). For shotgun sequencing, the digested nucleotides were separated on low concentration (0.6 to 0.8%) agarose gels, fragments were excised, and agar digested with Agar ACE (Promega). Extended clones were religated using T4 ligase (New England Biolabs, Beverly MA) into pUC 18 vector (Amersham Pharmacia Biotech), treated with Pfu DNA polymerase (Stratagene) to fill-in restriction site overhangs, and transfected into competent E. coli cells. Transformed cells were selected on antibiotic-containing media, and individual colonies were picked and cultured overnight at 37°C in 384-well plates in LB/2x carb liquid media.

The cells were lysed, and DNA was amplified by PCR using Taq DNA polymerase (Amersham Pharmacia Biotech) and Pfu DNA polymerase (Stratagene) with the following parameters: Step 1: 94°C, 3 min; Step 2: 94°C, 15 sec; Step 3: 60°C, 1 min; Step 4: 72°C, 2 min; Step 5: steps 2, 3, and 4 repeated 29 times; Step 6: 72°C, 5 min; Step 7: storage at 4°C. DNA was quantified by PICOGREEN reagent (Molecular Probes) as described above. Samples with low DNA recoveries were reamplified using the same conditions as described above. Samples were diluted with 20% dimethysulfoxide (1:2, v/v), and sequenced using DYENAMIC energy transfer sequencing primers and the DYENAMIC DIRECT kit (Amersham Pharmacia Biotech) or the ABI PRISM BIGDYE Terminator cycle-sequencing ready reaction kit (Applied Biosystems).

In like manner, full length polynucleotide sequences are verified using the above procedure or are used to obtain 5' regulatory sequences using the above procedure along with oligonucleotides designed for such extension, and an appropriate genomic library.

**IX. Labeling and Use of Individual Hybridization Probes**

Hybridization probes derived from SEQ ID NO:11-20 are employed to screen cDNAs, genomic DNAs, or mRNAs. Although the labeling of oligonucleotides, consisting of about 20 base pairs, is specifically described, essentially the same procedure is used with larger nucleotide fragments. Oligonucleotides are designed using state-of-the-art software such as OLIGO 4.06 software (National Biosciences) and labeled by combining 50 pmol of each oligomer, 250 μCi of [$\gamma$-$^{32}$P] adenosine triphosphate (Amersham Pharmacia Biotech), and T4 polynucleotide kinase (DuPont NEN, Boston MA). The labeled oligonucleotides are substantially purified using a SEPHADEX G-25 superfine size exclusion dextran bead column (Amersham Pharmacia Biotech). An aliquot containing $10^7$ counts per minute of the labeled probe is used in a typical membrane-based hybridization analysis of human genomic DNA digested with one of the following endonucleases: Ase I, Bgl II, Eco RI, Pst I, Xba I, or Pvu II (DuPont NEN).

The DNA from each digest is fractionated on a 0,7% agarose gel and transferred to nylon membranes (Nytran Plus, Schleicher & Schuell, Durham NH). Hybridization is carried out for 16 hours at 40°C. To remove nonspecific signals, blots are sequentially washed at room temperature under conditions of up to, for example, 0.1 x saline sodium citrate and 0.5% sodium dodecyl sulfate. Hybridization patterns are visualized using autoradiography or an alternative imaging means and compared.

**X. Microarrays**

The linkage or synthesis of array elements upon a microarray can be achieved utilizing photolithography, piezoelectric printing (ink-jet printing, See, e.g., Baldeschweiler, supra), mechanical microspotting technologies, and derivatives thereof. The substrate in each of the aforementioned technologies should be uniform and solid with a non-porous surface (Schena (1999), supra). Suggested substrates include silicon, silica, glass slides; glass chips, and silicon wafers. Alternatively, a procedure analogous to a dot or slot blot may also be used to arrange and link elements to the surface of a substrate using thermal, UV, chemical, or mechanical bonding procedures. A typical array may be produced using available methods and machines well known to those of ordinary skill in the art and may contain any appropriate number of elements. (See, e.g., Schena, M. et al. (1995) Science 270:467-470; Shalon, D. et al. (1996) Genome Res. 6:639-645; Marshall, A. and J. Hodgson (1998) Nat. Biotechnol. 16:27-31.)

Full length cDNAs, Expressed Sequence Tags (ESTs), or fragments or oligomers thereof may comprise the elements of the microarray. Fragments or oligomers suitable for hybridization can be selected using software well known in the art such as LASERGENE software (DNASTAR). The array elements are hybridized with polynucleotides in a biological sample. The polynucleotides in the biological sample are conjugated to a fluorescent label or other molecular tag for ease of detection. After hybridization, nonhybridized nucleotides from the biological sample are removed, and a fluorescence scanner is used to detect hybridization at each array element. Alternatively, laser desorbtion and mass spectrometry may be used for detection of hybridization. The degree of complementarity and the relative abundance of each polynucleotide which hybridizes to an element on the microarray may be assessed. In one embodiment, microarray preparation and usage is described in detail below.

## Tissue or Cell Sample Preparation

Total RNA is isolated from tissue samples using the guanidinium thiocyanate method and poly(A)$^+$ RNA is purified using the oligo-(dT) cellulose method. Each poly(A)$^+$ RNA sample is reverse transcribed using MMLV reverse-transcriptase, 0.05 pg/µl oligo-(dT) primer (21mer), 1X first strand buffer, 0.03 units/µl RNase inhibitor, 500 µM dATP, 500 µM dGTP, 500 µM dTTP, 40 µM dCTP, 40 µM dCTP-Cy3 (BDS) or dCTP-Cy5 (Amersham Pharmacia Biotech). The reverse transcription reaction is performed in a 25 ml volume containing 200 ng poly(A) + RNA with GEMBRIGHT kits (Incyte). Specific control poly(A)$^+$ RNAs are synthesized by in vitro transcription from non-coding yeast genomic DNA. After incubation at 37°C for 2 hr, each reaction sample (one with Cy3 and another with Cy5 labeling) is treated with 2.5 ml of 0.5M sodium hydroxide and incubated for 20 minutes at 85°C to the stop the reaction and degrade the RNA. Samples are purified using two successive CHROMA SPIN 30 gel filtration spin columns (CLONTECH Laboratories, Inc. (CLONTECH), Palo Alto CA) and after combining, both reaction samples are ethanol precipitated using 1 ml of glycogen (1 mg/ml), 60 ml sodium acetate, and 300 ml of 100% ethanol. The sample is then dried to completion using a SpeedVAC (Savant Instruments Inc., Holbrook NY and resuspended in 14 µl 5X SSC/0.2% SDS.

## Microarray Preparation

Sequences of the present invention are used to generate array elements. Each array element is amplified from bacterial cells containing vectors with cloned cDNA inserts. PCR amplification uses primers complementary to the vector sequences flanking the cDNA insert. Array elements are amplified in thirty cycles of PCR from an initial quantity of 1-2 ng to a final quantity greater than 5 µg. Amplified array elements are then purified using SEPHACRYL-400 (Amersham Pharmacia Biotech).

Purified array elements are immobilized on polymer-coated glass slides. Glass microscope slides (Coming) are cleaned by ultrasound in 0.1 % SDS and acetone, with extensive distilled water washes between and after treatments. Glass slides are etched in 4% hydrofluoric acid (VWR Scientific Products Corporation (VWR), West Chester PA), washed extensively in distilled water, and coated with 0.05% aminopropyl silane (Sigma) in 95% ethanol. Coated slides are cured in a 110°C oven.

Array elements are applied to the coated glass substrate using a procedure described in US Patent No. 5,807,522, incorporated herein by reference. 1 µl of the array element DNA, at an average concentration of 100 ng/µl, is loaded into the open capillary printing element by a high-speed robotic apparatus. The apparatus then deposits about 5 nl of array element sample per slide.

Microarrays are UV-crosslinked using a STRATALINKER UV-crosslinker (Stratagene). Microarrays are washed at room temperature once in 0.2% SDS and three times in distilled water. Non-specific binding sites are blocked by incubation of microarrays in 0.2% casein in phosphate buffered saline (PBS) (Tropix, Inc., Bedford MA) for 30 minutes at 60° C followed by washes in 0.2% SDS and distilled water as before.

## Hybridization

Hybridization reactions contain 9 µl of sample mixture consisting of 0.2 µg each of Cy3 and Cy5 labeled cDNA synthesis products in 5X SSC, 0.2% SDS hybridization buffer. The sample mixture is heated to 65°C for 5 minutes and is aliquoted onto the microarray surface and covered with an 1.8 $cm^2$ coverslip. The arrays are transferred to a waterproof chamber having a cavity just slightly larger than a microscope slide. The chamber is kept at 100% humidity internally by the addition of 140 µl of 5X SSC in a corner of the chamber. The chamber containing the arrays is incubated for about 6.5 hours at 60°C. The arrays are washed for 10 min at 45°C in a first wash buffer (1X SSC, 0.1% SDS), three times for 10 minutes each at 45°C in a second wash buffer (0.1X SSC), and dried.

## Detection

Reporter-labeled hybridization complexes are detected with a microscope equipped with an Innova 70 mixed gas 10 W laser (Coherent, Inc., Santa Clara CA) capable of generating spectral lines at 488 nm for excitation of Cy3 and at 632 nm for excitation of Cy5. The excitation laser light is focused on the array using a 20X microscope objective (Nikon, Inc., Melville NY). The slide containing the array is placed on a computer-controlled X-Y stage on the microscope and raster-scanned past the objective. The 1.8 cm x 1.8 cm array used in the present example is scanned with a resolution of 20 micrometers.

In two separate scans, a mixed gas multiline laser excites the two fluorophores sequentially. Emitted light is split, based on wavelength, into two photomultiplier tube detectors (PMT R1477, Hamamatsu Photonics Systems, Bridgewater NJ) corresponding to the two fluorophores. Appropriate filters positioned between the array and the photomultiplier tubes are used to filter the signals. The emission maxima of the fluorophores used are 565 nm for Cy3 and 650 nm for Cy5. Each array is typically scanned twice, one scan per fluorophore using the appropriate filters at the laser source, although the apparatus is capable of recording the spectra from both fluorophores simultaneously.

The sensitivity of the scans is typically calibrated using the signal intensity generated by a cDNA control species added to the sample mixture at a known concentration. A specific location on the array contains a complementary DNA sequence, allowing the intensity of the signal at that location to be correlated with a weight ratio of hybridizing species of 1:100,000. When two samples from different sources (e.g., representing test and control cells), each labeled with a different fluorophore, are hybridized to a single array for the purpose of identifying genes that are differentially expressed, the calibration is done by labeling samples of the calibrating cDNA with the two fluorophores and adding identical amounts of each to the hybridization mixture.

The output of the photomultiplier tube is digitized using a 12-bit RTI-835H analog-to-digital (A/D) conversion board (Analog Devices, Inc., Norwood MA) installed in an IBM-compatible PC computer. The digitized data are displayed as an image where the signal intensity is mapped using a linear 20-color transformation to a pseudocolor scale ranging from blue (low signal) to red (high signal). The data is also analyzed quantitatively. Where two different fluorophores are excited and measured simultaneously, the data are first corrected for optical crosstalk (due to overlapping emission spectra) between the fluorophores using each fluorophore's emission spectrum.

A grid is superimposed over the fluorescence signal image such that the signal from each spot is centered in each element of the grid. The fluorescence signal within each element is then integrated to obtain a numerical value corresponding to the average intensity of the signal. The software used for signal analysis is the GEMTOOLS gene expression analysis program (Incyte).

## XI. Complementary Polynucleotides

Sequences complementary to the GCREC-encoding sequences, or any parts thereof, are used to detect, decrease, or inhibit expression of naturally occurring GCREC. Although use of oligonucleotides comprising from about 15 to 30 base pairs is described, essentially the same procedure is used with smaller or with larger sequence fragments. Appropriate oligonucleotides are designed using OLIGO 4.06 software (National Biosciences) and the coding sequence of GCREC. To inhibit transcription, a complementary oligonucleotide is designed from the most unique 5' sequence and used to prevent promoter binding to the coding sequence. To inhibit translation, a complementary oligonucleotide is designed to prevent ribosomal binding to the GCREC-encoding transcript.

## XII. Expression of GCREC

Expression and purification of GCREC is achieved using bacterial or virus-based expression systems. For expression of GCREC in bacteria, cDNA is subcloned into an appropriate vector containing an antibiotic resistance gene and an inducible promoter that directs high levels of cDNA transcription. Examples of such promoters include, but are not limited to, the *trp-lac* (*tac*) hybrid promoter and the T5 or T7 bacteriophage promoter in conjunction with the lac operator regulatory element. Recombinant vectors are transformed into suitable bacterial hosts, e.g., BL21(DE3). Antibiotic resistant bacteria express GCREC upon induction with isopropyl beta-D-thiogalactopyranoside (IPTG). Expression of GCREC in eukaryotic cells is achieved by infecting insect or mammalian cell lines with recombinant Autographica californica nuclear polyhedrosis virus (AcMNPV), commonly known as baculovirus. The nonessential polyhedrin gene of baculovirus is replaced with cDNA encoding GCREC by either homologous recombination or bacterial-mediated transposition involving transfer plasmid intermediates. Viral infectivity is maintained and the strong polyhedrin promoter drives high levels of cDNA transcription. Recombinant baculovirus is used to infect Spodoptera frugiperda (Sf9) insect cells in most cases, or human hepatocytes, in some cases. Infection of the latter requires additional genetic modifications to baculovirus. (See Engelhard, E.K. et al. (1994) Proc. Natl. Acad. Sci. USA 91:3224-3227; Sandig, V. et al. (1996) Hum. Gene Ther. 7:1937-1945.)

In most expression systems, GCREC is synthesized as a fusion protein with, e.g., glutathione S-transferase (GST) or a peptide epitope tag, such as FLAG or 6-His, permitting rapid, single-step, affinity-based purification of recombinant fusion protein from crude cell lysates. GST, a 26-kilodalton enzyme from Schistosoma japonicum, enables the purification of fusion proteins on immobilized glutathione under conditions that maintain protein activity and antigenicity (Amersham Pharmacia Biotech). Following purification, the GST moiety can be proteolytically cleaved from GCREC at specifically engineered sites. FLAG, an 8-amino acid peptide, enables immunoaffinity purification using commercially available monoclonal and polyclonal anti-FLAG antibodies (Eastman Kodak). 6-His, a stretch of six consecutive histidine residues, enables purification on metal-chelate resins (QIAGEN). Methods for protein expression and purification are discussed in Ausubel (1995, supra, ch. 10 and 16). Purified GCREC obtained by these methods can be used directly in the assays shown in Examples XVL XVII, and XVIII, where applicable.

### XIII. Functional Assays

GCREC function is assessed by expressing the sequences encoding GCREC at physiologically elevated levels in mammalian cell culture systems. cDNA is subcloned into a mammalian expression vector containing a strong promoter that drives high levels of cDNA expression. Vectors of choice include PCMV SPORT (Life Technologies) and PCR3.1 (Invitrogen, Carlsbad CA), both of which contain the cytomegalovirus promoter. 5-10 μg of recombinant vector are transiently transfected into a human cell line, for example, an endothelial or hematopoietic cell line, using either liposome formulations or electroporation. 1-2 μg of an additional plasmid containing sequences encoding a marker protein are co-transfected. Expression of a marker protein provides a means to distinguish transfected cells from nontransfected cells and is a reliable predictor of cDNA expression from the recombinant vector. Marker proteins of choice include, e.g., Green Fluorescent Protein (GFP; Clontech), CD64, or a CD64-GFP fusion protein. Flow cytometry (FCM), an automated, laser optics-based technique, is used to identify transfected cells expressing GFP or CD64-GFP and to evaluate the apoptotic state of the cells and other cellular properties. FCM detects and quantifies the uptake of fluorescent molecules that diagnose events preceding or coincident with cell death. These events include changes in nuclear DNA content as measured by staining of DNA with propidium iodide; changes in cell size and granularity as measured by forward light scatter and 90 degree side light scatter; down-regulation of DNA synthesis as measured by decrease in bromodeoxyuridine uptake; alterations in expression of cell surface and intracellular proteins as measured by reactivity with specific antibodies; and alterations in plasma membrane composition as measured by the binding of fluorescein-conjugated Annexin V protein to the cell surface. Methods in flow cytometry are discussed in Ormerod, M.G. (1994) Flow Cytometry, Oxford, New York NY.

The influence of GCREC on gene expression can be assessed using highly purified populations of cells transfected with sequences encoding GCREC and either CD64 or CD64-GFP. CD64 and CD64-GFP are expressed on the surface of transfected cells and bind to conserved regions of human immunoglobulin G (IgG). Transfected cells are efficiently separated from nontransfected cells using magnetic beads coated with either human IgG or antibody against CD64 (DYNAL, Lake Success NY). mRNA can be purified from the cells using methods well known by those of skill in the art. Expression of mRNA encoding GCREC and other genes of interest can be analyzed by northern analysis or microarray techniques.

### XIV. Production of GCREC Specific Antibodies

GCREC substantially purified using polyacrylamide gel electrophoresis (PAGE; see, e.g., Harrington, M.G. (1990) Methods Enzymol. 182:488-495), or other purification techniques, is used to immunize rabbits and to produce antibodies using standard protocols.

Alternatively, the GCREC amino acid sequence is analyzed using LASERGENE software (DNASTAR) to determine regions of high immunogenicity, and a corresponding oligopeptide is synthesized and used to raise antibodies by means known to those of skill in the art. Methods for selection of appropriate epitopes, such as those near the C-terminus or in hydrophilic regions are well described in the art. (See, e.g., Ausubel, 1995, supra, ch. 11.)

Typically, oligopeptides of about 15 residues in length are synthesized using an ABI 431A peptide synthesizer (Applied Biosystems) using FMOC chemistry and coupled to KLH (Sigma-Aldrich, St. Louis MO) by reaction with N-maleimidobenzoyl-N-hydroxysuccinimide ester (MBS) to increase immunogenicity. (See, e.g., Ausubel, 1995, supra.) Rabbits are immunized with the oligopeptide-KLH complex in complete Freund's adjuvant. Resulting antisera are tested for antipeptide and anti-GCREC activity by, for example, binding the peptide or GCREC to a substrate, blocking with 1% BSA, reacting with rabbit antisera, washing, and reacting with radio-iodinated goat anti-rabbit IgG.

### XV. Purification of Naturally Occurring GCREC Using Specific Antibodies

Naturally occurring or recombinant GCREC is substantially purified by immunoaffinity chromatography using antibodies specific for GCREC. An immunoaffinity column is constructed by covalently coupling anti-GCREC antibody to an activated chromatographic resin, such as CNBr-activated SEPHAROSE (Amersham Pharmacia Biotech). After the coupling, the resin is blocked and washed according to the manufacturer's instructions.

Media containing GCREC are passed over the immunoaffinity column, and the column is washed under conditions that allow the preferential absorbance of GCREC (e.g., high ionic strength buffers in the presence of detergent). The column is eluted under conditions that disrupt antibody/GCREC binding (e.g., a buffer of pH 2 to pH 3, or a high concentration of a chaotrope, such as urea or thiocyanate ion), and GCREC is collected.

## XVI. Identification of Molecules Which Interact with GCREC

Molecules which interact with GCREC may include agonists and antagonists, as well as molecules involved in signal transduction, such as G proteins. GCREC, or a fragment thereof, is labeled with $^{125}I$ Bolton-Hunter reagent. (See, e.g., Bolton A.E. and W.M. Hunter (1973) Biochem. J. 133:529-539.) A fragment of GCREC includes, for example, a fragment comprising one or more of the three extracellular loops, the extracellular N-terminal region, or the third intracellular loop. Candidate molecules previously arrayed in the wells of a multi-well plate are incubated with the labeled GCREC, washed, and any wells with labeled GCREC complex are assayed. Data obtained using different concentrations of GCREC are used to calculate values for the number, affinity, and association of GCREC with the candidate ligand molecules.

Alternatively, molecules interacting with GCREC are analyzed using the yeast two-hybrid system as described in Fields, S. and O. Song (1989) Nature 340:245-246, or using commercially available kits based on the two-hybrid system, such as the MATCHMAKER system (Clontech). GCREC may also be used in the PATHCALLING process (CuraGen Corp., New Haven CT) which employs the yeast two-hybrid system in a high-throughput manner to determine all interactions between the proteins encoded by two large libraries of genes (Nandabalan, K. et al. (2000) U.S. Patent No. 6,057,101).

Potential GCREC agonists or antagonists may be tested for activation or inhibition of GCREC receptor activity using the assays described in sections XVII and XVIII. Candidate molecules may be selected from known GPCR agonists or antagonists, peptide libraries, or combinatorial chemical libraries.

Methods for detecting interactions of GCREC with intracellular signal transduction molecules such as G proteins are based on the premise that internal segments or cytoplasmic domains from an orphan G protein-coupled seven transmembrane receptor may be exchanged with the analogous domains of a known G protein-coupled seven transmembrane receptor and used to identify the G-proteins and downstream signaling pathways activated by the orphan receptor domains (Kobilka, B.K. et al. (1988) Science 240:1310-1316). In an analogous fashion, domains of the orphan receptor may be cloned as a portion of a fusion protein and used in binding assays to demonstrate interactions with specific G proteins. Studies have shown that the third intracellular loop of G protein-coupled seven transmembrane receptors is important for G protein interaction and signal transduction (Conklin, B.R. et al. (1993) Cell 73:631-641). For example, the DNA fragment corresponding to the third intracellular loop of GCREC may be amplified by the polymerase chain reaction (PCR) and subcloned into a fusion vector such as pGEX (Pharmacia Biotech). The construct is transformed into an appropriate bacterial host, induced, and the fusion protein is purified from the cell lysate by glutathione-Sepharose 4B (Pharmacia Biotech) affinity chromatography.

For in vitro binding assays, cell extracts containing G proteins are prepared by extraction with 50 mM Tris, pH 7.8, 1 mM EGTA, 5 mM $MgCl_2$, 20 mM CHAPS, 20% glycerol, 10 μg of both aprotinin and leupeptin, and 20 μl of 50 mM phenylmethylsulfonyl fluoride. The lysate is incubated on ice for 45 min with constant stirring, centrifuged at 23,000 g for 15 min at 4°C, and the supernatant is collected. 750 μg of cell extract is incubated with glutathione S-transferase (GST) fusion protein beads for 2 h at 4°C. The GST beads are washed five times with phosphate-buffered saline. Bound G subunits are detected by [$^{32}P$]ADP-ribosylation with pertussis or cholera toxins. The reactions are terminated by the addition of SDS sample buffer (4.6% (w/v) SDS, 10% (v/v) β-mercaptoethanol, 20% (w/v) glycerol, 95.2 mM Tris-HCl, pH 6.8, 0.01% (w/v) bromphenol blue). The [$^{32}P$]ADP-labeled proteins are separated on 10% SDS-PAGE gels, and autoradiographed. The separated proteins in these gels are transferred to nitrocellulose paper, blocked with blotto (5% nonfat dried milk, 50 mM Tris-HCl (pH 8.0), 2 mM $CaCl_2$, 80 mM NaCl, 0.02% $NaN_3$. and 0.2% Nonidet P-40) for 1 hour at room temperature, followed by incubation for 1.5 hours with G α subtype selective antibodies (1:500; Calbiochem-Novabiochem). After three washes, blots are incubated with horseradish peroxidase (HRP)-conjugated goat anti-rabbit immunoglobulin (1:2000, Cappel, Westchester PA) and visualized by the chemiluminescence-based ECL method (Amersham Corp.).

## XVII. Demonstration of GCREC Activity

An assay for GCREC activity measures the expression of GCREC on the cell surface. cDNA encoding GCREC is transfected into an appropriate mammalian cell line. Cell surface proteins are labeled with biotin as described (de la Fuente, M.A. et al. (1997) Blood 90:2398-2405). Immunoprecipitations are performed using GCREC-specific antibodies, and immunoprecipitated samples are analyzed using sodium dodecyl sulfate polyacrylamide gel electrophoresis (SDS-PAGE) and immunoblotting techniques. The ratio of labeled immunoprecipitant to unlabeled immunoprecipitant is proportional to the amount of GCREC expressed on the cell surface.

In the alternative, an assay for GCREC activity is based on a prototypical assay for ligand/receptor-mediated modulation of cell proliferation. This assay measures the rate of DNA synthesis in Swiss mouse 3T3 cells. A plasmid containing polynucleotides encoding GCREC is added to quiescent 3T3 cultured cells using transfection methods well known in the art. The transiently transfected cells are then incubated in the presence of [$^3$H]thymidine, a radioactive DNA precursor molecule. Varying amounts of GCREC ligand are then added to the cultured cells. Incorporation of [$^3$H]thymidine into acid-precipitable DNA is measured over an appropriate time interval using a radioisotope counter, and the amount incorporated is directly proportional to the amount of newly synthesized DNA. A linear dose-response curve over at least a hundred-fold GCREC ligand concentration range is indicative of receptor activity. One unit of activity per milliliter is defined as the concentration of GCREC producing a 50% response level, where 100% represents maximal incorporation of [$^3$H]thymidine into acid-precipitable DNA (McKay, I. and I. Leigh, eds. (1993) Growth Factors: A Practical Approach, Oxford University Press, New York NY, p. 73.)

In a further alternative, the assay for GCREC activity is based upon the ability of GPCR family proteins to modulate G protein-activated second messenger signal transduction pathways (e.g., cAMP; Gaudin, P. et al. (1998) J. Biol. Chem. 273:4990-4996). A plasmid encoding full length GCREC is transfected into a mammalian cell line (e.g., Chinese hamster ovary (CHO) or human embryonic kidney (HEK-293) cell lines) using methods well-known in the art. Transfected cells are grown in 12-well trays in culture medium for 48 hours, then the culture medium is discarded, and the attached cells are gently washed with PBS. The cells are then incubated in culture medium with or without ligand for 30 minutes, then the medium is removed and cells lysed by treatment with 1 M perchloric acid. The cAMP levels in the lysate are measured by radioimmunoassay using methods well-known in the art. Changes in the levels of cAMP in the lysate from cells exposed to ligand compared to those without ligand are proportional to the amount of GCREC present in the transfected cells.

To measure changes in inositol phosphate levels, the cells are grown in 24-well plates containing $1x10^5$ cells/well and incubated with inositol-free media and [$^3$H]myoinositol, 2 $\mu$Ci/well, for 48 hr. The culture medium is removed, and the cells washed with buffer containing 10 mM LiCl followed by addition of ligand. The reaction is stopped by addition of perchloric acid. Inositol phosphates are extracted and separated on Dowex AG1-X8 (Bio-Rad) anion exchange resin, and the total labeled inositol phosphates counted by liquid scintillation. Changes in the levels of labeled inositol phosphate from cells exposed to ligand compared to those without ligand are proportional to the amount of GCREC present in the transfected cells.

### XVIII. Identification of GCREC Ligands

GCREC is expressed in a eukaryotic cell line such as CHO (Chinese Hamster Ovary) or HEK (Human Embryonic Kidney) 293 which have a good history of GPCR expression and which contain a wide range of G-proteins allowing for functional coupling of the expressed GCREC to downstream effectors. The transformed cells are assayed for activation of the expressed receptors in the presence of candidate ligands. Activity is measured by changes in intracellular second messengers, such as cyclic AMP or $Ca^{2+}$. These may be measured directly using standard methods well known in the art, or by the use of reporter gene assays in which a luminescent protein (e.g. firefly luciferase or green fluorescent protein) is under the transcriptional control of a promoter responsive to the stimulation of protein kinase C by the activated receptor (Milligan, G. et al. (1996) Trends Pharmacol. Sci. 17:235-237). Assay technologies are available for both of these second messenger systems to allow high throughput readout in multi-well plate format, such as the adenylyl cyclase activation FlashPlate Assay (NEN Life Sciences Products), or fluorescent $Ca^{2+}$ indicators such as Fluo-4 AM (Molecular Probes) in combination with the FLIPR fluorimetric plate reading system (Molecular Devices). In cases where the physiologically relevant second messenger pathway is not known, GCREC may be coexpressed with the G-proteins $G_{\alpha 15/16}$ which have been demonstrated to couple to a wide range of G-proteins (Offermanns, S. and M.I. Simon (1995) J. Biol. Chem. 270:15175-15180), in order to funnel the signal transduction of the GCREC through a pathway involving phospholipase C and $Ca^{2+}$ mobilization. Alternatively, GCREC may be expressed in engineered yeast systems which lack endogenous GPCRs, thus providing the advantage of a null background for GCREC activation screening. These yeast systems substitute a human GPCR and $G_\alpha$ protein for the corresponding components of the endogenous yeast pheromone receptor pathway. Downstream signaling pathways are also modified so that the normal yeast response to the signal is converted to positive growth on selective media or to reporter gene expression (Broach, J.R. and J. Thorner (1996) Nature 384 (supp.): 14-16). The receptors are screened against putative ligands including known GPCR ligands and other naturally occurring bioactive molecules. Biological extracts from tissues, biological fluids and cell supernatants are also screened.

Various modifications and variations of the described methods and systems of the invention will be apparent to those skilled in the art without departing from the scope and spirit of the invention. Although the invention has been described in connection with certain embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

Table 1

| Incyte Project ID | Polypeptide SEQ ID NO: | Incyte Polypeptide ID | Polynucleotide SEQ ID NO: | Incyte Polynucleotide ID |
|---|---|---|---|---|
| 7474927 | 1 | 7474927CD1 | 11 | 7474927CB1 |
| 7475194 | 2 | 7475194CD1 | 12 | 7475194CB1 |
| 7475203 | 3 | 7475203CD1 | 13 | 7475203CB1 |
| 7474987 | 4 | 7474987CD1 | 14 | 7474987CB1 |
| 5617631 | 5 | 5617631CD1 | 15 | 5617631CB1 |
| 7472098 | 6 | 7472098CD1 | 16 | 7472098CB1 |
| 7476775 | 7 | 7476775CD1 | 17 | 7476775CB1 |
| 7477937 | 8 | 7477937CD1 | 18 | 7477937CB1 |
| 7476798 | 9 | 7476798CD1 | 19 | 7476798CB1 |
| 7477889 | 10 | 7477889CD1 | 20 | 7477889CB1 |

Table 2

| Polypeptide SEQ ID NO: | Incyte Polypeptide ID | GenBank ID NO: | Probability Score | GenBank Homolog |
|---|---|---|---|---|
| 1 | 7474927CD1 | g3892596 | 6.20E-26 | [Mus musculus] pheromone receptor 2 (seven domain GPCR) |
| | | g10732802 | 0 | [Homo sapiens] vomeronasal receptor 1 |
| 2 | 7475194CD1 | g683747 | 2.10E-100 | [Homo sapiens] extracellular calcium-sensing receptor (GPCR) (Garrett, J.E. et al. (1995) J. Biol. Chem. 270:12919-12925) |
| | | g13936377 | 0 | [Mus musculus] taste receptor T1R3 |
| 3 | 7475203CD1 | g12745520 | 0 | [Mus musculus] putative sweet taste receptor T1R1 |
| | | g1836094 | 1.70E-110 | [Homo sapiens] calcium-sensing receptor, CaSR, human, medullary (GPCR) (Freichel, M. et al. (1996) Endocrinology 137:3842-3848) |
| 4 | 7474987CD1 | g6691938 | 7.20E-85' | [Homo sapiens] novel 7 transmembrane receptor |
| | | g7638409 | 4.60E-67 | [Mus musculus] olfactory receptor P2 (Zheng, C. et al. (2000) Neuron 26:81-91) |

(continued)

| Polypeptide SEQ ID NO: | Incyte Polypeptide ID | GenBank ID NO: | Probability Score | GenBank Homolog |
|---|---|---|---|---|
| 5 | 5617631CD1 | g7638409 | 2.20E-67 | [Mus musculus] olfactory receptor P2 (Zheng, C. et al. (2000) Neuron 26:81-91) |
| | | g12007428 | 8.00E-73 | [Mus musculus] B5 olfactory receptor |
| 6 | 7472098CD1 | g11908221 | 1.06E-89 | [Mus musculus] MOR 3'Beta6 |
| | | g4761598 | 3.70E-81 | [Mus musculus] MOR 3'Beta2 (Bulger, M. et al. (1999) Proc. Natl. Acad. Sci. USA 96:5129-5134) |
| 7 | 7476775CD1 | g4680254 | 1.10E-145 | [Mus musculus] odorant receptor S1 |
| 8 | 7477937CD1 | g2765660 | 5.90E-76 | [Gallus gallus] chick olfactory receptor 7 (Nef, S. and P. Nef. (1997) Proc. Natl. Acad. Sci. USA 94: 4766-4771) |
| 9 | 7476798CD1 | g12007423 | 2.00E-81 | [Mus musculus] T2 olfactory receptor |
| | | g7638409 | 8.90E-73 | [Mus musculus] olfactory receptor P2 |
| 10 | 7477889CD1 | g7638409 | 2.80E-62 | [Mus musculus] olfactory receptor P2 |

Table 3

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| 1 | 7474927CD1 | 353 | T5, S164, S200, S209, S213, S217, S258, S262, T341 | N117, N183, N198, N256 | RECEPTOR PHEROMONE, G-PROTEIN COUPLED VN1 VN2 VN3 VN7 VN4 VN5: PD009900: I73-F335 | BLAST-PRODOM |
| | | | | | G-protein coupled receptor: BL00237C: S266-L292 | BLIMPS-BLOCKS |
| 2 | 7475194CD1 | 863 | T102, T153, S175, S189, S214, S289, S293, S477, T480, S539, S562, S570, S678, | N85, N130, N264, N285, N380, N411, N432, N475, N748 | Metabotropic glutamate GPCR signature: PR00248A: K32-G44; PR00248B: G69-N84; PR00248C: N84-C103; PR00248D: V141-Y167; PR00248E: L174-Q193; PR00248F: Q193-V209; PR00248G: V209-F226; PR00248H: S607-C629; PR00248J: A692-P715; PR00248K: T747-N770; PR00248L: N770-P791; PR00592D: N130-G143; PR00592E: D215-C236 | BLIMPS-PRINTS |
| | | | | | Transmembrane domains: L581-F601; L617-F635; A692-L711 | HMMER |
| | | | | | Receptor family ligand binding region (ANF_ receptor): V61-D470 | HMMER-PFAM |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | G-protein coupled receptor: BL00979A: L71-A118; BL00979B: S147-L194; BL00979C: T195-F226;; BL00979F: G384-K422; BL00979I: P506-H526; BL00979J: Y530-L581; BL00979K: L586-V632; BL00979L: L633-S673; BL00979M: A746-V796 | BLIMPS-BLOCKS |
| | | | | | RECEPTOR, G-PROTEIN COUPLED, TRANSMEMBRANE GLYCOPROTEIN, PHEROMONE PRECURSOR, METABOTROPIC GLUTAMATE: PD001315: W575-N835 | BLAST-PRODOM |
| | | | | | G-PROTEIN COUPLED RECEPTORS FAMILY 3: DM00837\|I59362\|1-893: L9-H341 | BLAST-DOMO |
| | | | | | G-protein coupled receptor motif (0754.pdpc): C528-C552 | MOTIFS |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| 3 | 7475203CD1 | 841 | T5, S28, T60, N87, N88, S67, T115, N95, N291, T149, S177, N479, N529, S216, S275, N822 T293, Y341, T650, S663, S781, S830, | | G-protein coupled receptor: BL00979A: H74-L121; BL00979B: T149-V196; BL00979C: E197-L228; BL00979G: V428-D455; BL00979I: P493-H513; BL00979K: A573-L619; BL00979L: Y620-F660; BL00979M: L733-Y783; BL00979N: Y787-S823 | BLIMPS-BLOCKS |
| | | | T835 | | Metabotropic glutamate GPCR signature: PR00248A: P35-H47; PR00248B: G72-N87; PR00248C: N87-C106; PR00248D: V143-Y169; PR00248E: L176-Q195; PR00248F: Q195-I211; PR00248G: I211-L228; PR00248H: T594-S616; PR00248I: A639-F660; PR00248J: A679-T702; PR00248K: Y734-N757; PR00248L: N757-T778 | BLIMPS-PRINTS |
| | | | | | Signal peptide: M1-S25 | SIGPEPT |
| | | | | | Signal cleavage: M1-S25 | SPSCAN |
| | | | | | Transmembrane domains: V569-W590; L681-W701; T763-Y783 | HMMER |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | Receptor family ligand binding region (ANF_ receptor): C66-E480 | HMMER-PFAM |
| | | | | | 7 transmembrane receptor (metabotropic (7tm_3)): A572-N822 | HMMER-PFAM |
| | | | | | RECEPTOR, G-PROTEIN COUPLED; TRANSMEMBRANE GLYCOPROTEIN; PHEROMONE PRECURSOR; SIGNAL METABOTROPIC GLUTAMATE GPCR: PD001315: V569-N822 | BLAST-PRODOM |
| | | | | | G-PROTEIN COUPLED RECEPTORS FAMILY 3: DM00837\|P35384\|1-894: L363-S830 | BLAST-DOMO |
| 4 | 7474987CD1 | 309 | T47 S65 S186 S265 T17 T222 S288 T307 | N3 N63 N87 N88 | transmembrane domains: I27-I46; I195-I214 | HMMER |
| | | | | | 7 transmembrane receptor (rhodopsin family): G39-Y287 | HMMER-PFAM |
| | | | | | G-protein coupled receptor: BL00237A: N88-P127; BL00237C: S16-L42; BL00237D: P279-K295 | BLIMPS-BLOCKS |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | G-protein coupled receptors signature: F100-V145 | PROFILESCAN |
| | | | | | G_Protein Receptor: A108-I124 | MOTIFS |
| | | | | | Olfactory receptor signature: PR00245A: V57-K78; PR00245B: F175-E189; PR00245C: Y236-T251; PR00245D: I271-F282; PR00245E: S288-I302 | BLIMPS-PRINTS |
| | | | | | Rhodopsin-like GPCR superfamily signature: PR00237A: L24-T48; PR00237B: V57-K78; PR00237C: L102-I124; PR00237E: L197-F220; PR00237F: E194-H218; PR00237G: A269-K295 | BLIMPS-PRINTS |
| | | | | | RECEPTOR OLFACTORY PROTEIN G-PROTEIN COUPLED TRANSMEMBRANE GLYCOPROTEIN MULTIGENE FAMILY PD000921: L164-L244 | BLAST-PRODOM |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | G-PROTEIN COUPLED RECEPTORS DM00013\|P23274\|18-306: L28-L298 DM00013\|S29707\|18-306: L28-L301 DM00013\|P23266\|17-306: I27-L298 DM00013\|P23269\|15-304: L28-L298 | BLAST-DOMO |
| 5 | 5617631CD1 | 317 | S22, S50, S68, S138, S164, S189, S233, S292 | N4 | Olfactory receptor signature: PR00245A: M60-Q81; PR00245B: F178-D192; PR00245C: F239-T254; PR00245D: F275-C286; PR00245E: S292-L306 | BLIMPS-PRINTS |
| | | | | | OLFACTORY RECEPTOR PROTEIN, RECEPTOR-LIKE G-PROTEIN COUPLED TRANSMEMBRANE GLYCOPROTEIN, MULTI-GENE FAMILY: PD149621: T247-R308 | BLAST-PRODOM |
| | | | | | G-protein Receptor: A111-I127 | MOTIFS |
| | | | | | G-PROTEIN COUPLED RECEPTORS: DM00013\|P23270\|18-311: R24-L306 | BLAST-DOMO |
| | | | | | Signal cleavage: M1-A40 | SPSCAN |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| 6 | 7472098CD1 | 317 | S180, T191 | N44 | Transmembrane domain: A25-I48; M60-I79; F195-T215 | HMMER |
| | | | | | 7 transmembrane receptor (rhodopsin family), 7tm_1: M41-Y291 | HMMER-PFAM |
| | | | | | G-protein coupled receptor: BL00237A: H91-P130; BL00237D: T283-K299 | BLIMPS-BLOCKS |
| | | | | | G-protein coupled receptors signature: Y103-T148 | PROFILESCAN |
| | | | | | G-PROTEIN COUPLED RECEPTORS: DM00013\|G45774\|18-309 : P20-L306 | BLAST-DOMO |
| | | | | | PUTATIVE G-PROTEIN COUPLED RECEPTOR, RA1C: PD170483: I251-I312 | BLAST-PRODOM |
| | | | | | Transmembrane domain: F207-F225, M36-R55 | HMMER |
| | | | | | 7 transmembrane receptor (rhodopsin family), 7tm_1: G43-Y295 | HMMER-PFAM |
| | | | | | G-protein coupled receptor: BL00237A: C92-P131 ;BL00237C: E235-S261; BL00237D: P287-R303 | BLIMPS-BLOCKS |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| 6 | | | | | Olfactory receptor signature: PR00245A: M61-K82; PR00245B: S180-D194; PR00245C: L279-L290 | BLIMPS-PRINTS |
| 7 | 7476775CD1 | 324 | T147 T301 | N12 | Transmembrane domain: I35-G51 | HMMER |
| | | | | | 7 transmembrane receptor (rhodopsin family): G51-Y300 | HMMER-PFAM |
| | | | | | G-protein coupled receptors signature: Y112-F157 | ProfileScan |
| | | | | | G-protein coupled receptor motif: T120-I136 | MOTIFS |
| | | | | | G-protein coupled receptor signatures: BL00237A: K100-P139 BL00237C: R245-S271 BL00237D: T292-K308 | BLIMPS-BLOCKS |
| | | | | | Olfactory receptor signatures: PR00245A: M69-N90 PR00245B: F187-P201 PR00245C: F248-G263 PR00245D: L284-F295 PR00245E: T301-L315 | BLIMPS-PRINTS |
| | | | | | G-protein coupled receptor: DM00013\|P23270\|18-311: F27-L315 | BLAST-DOMO |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | G-protein coupled receptor: DM00013\|P23267\|20-309: F27-L315 | BLAST-DOMO |
| | | | | | G-protein coupled receptor: DM00013\|P23266\|17-306: Q34-L315 | BLAST-DOMO |
| | | | | | G-protein coupled receptor: DM00013\|P30955\|18-305: F38-L315 | BLAST-DOMO |
| | | | | | Olfactory G-protein coupled receptor: PD000921: L176-V256 | BLAST-PRODOM |
| | | | | | Olfactory G-protein coupled receptor: PD149621: V257-L315 | BLAST-PRODOM |
| 8 | 7477937CD1 | 322 | S164 S232 S291 S316 S67 T237 T315 | N5 | G_Protein_Receptor: T110-I126 | MOTIFS |
| | | | | | G-protein coupled receptors signature: Y102-V147 | PROFILESCAN |
| 8 | | | | | Visual pigments (opsins) retinal binding site opsin.prf: S263-R318 | PROFILESCAN |
| | | | | | signal peptide: M136-T155 | HMMER |
| | | | | | transmembrane domains: A25-T47, I92-M118, V197-I221 | HMMER |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | 7 transmembrane receptor (rhodopsin family) 7tm_1: G41-Y290 | HMMER-PFAM |
| | | | | | G-protein coupled receptor domain: BL00237: Q90-P129, F200-F211, T195-I221, T282-K298 | BLIMPS-BLOCKS |
| | | | | | Olfactory receptor signature PR00245: M59-L80, F177-R191, F238-G253, I274-L285, S291-M305 | BLIMPS-PRINTS |
| | | | | | OLFACTORY RECEPTOR PROTEIN PD000921: L166-L245, PD149621: V247-K308 | BLAST-PRODOM |
| | | | | | G-PROTEIN COUPLED RECEPTORS DM00013\|P23274\|18-306: E22-M305 | BLAST-DOMO |
| 9 | 7476798CD1 | 312 | S136 S20 S263 S266 S290 S304 S309 S66 T7 | N41 N5 N88 | 7 transmembrane receptor (rhodopsin family) 7tm_1: G40-Y289 | HMMER-PFAM |
| | | | | | G-PROTEIN COUPLED RECEPTORS DM00013\|S29707\|18-306: V17-L300 | BLAST-DOMO |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | RECEPTOR OLFACTORY RECEPTORLIKE GPROTEIN COUPLED TRANSMEMBRANE GLYCOPROTEIN MULTIGENE FAMILY PD000921: I165-L244 | BLAST-PRODOM |
| | | | | | OLFACTORY RECEPTOR RECEPTORLIKE GPROTEIN COUPLED TRANSMEMBRANE GLYCOPROTEIN MULTIGENE FAMILY PD149621: T245-R306 | BLAST- PRODOM |
| | | | | | G-protein coupled receptor BL00237: K89-P128, T281-M297 | BLIMPS-BLOCKS |
| 9 | | | | | Rhodopsin-like GPCR superfamily PR00237: F25-F49, M58-K79, F103-I125, M139-V160, V198-L221, A236-R260, K271-M297 | BLIMPS-PRINTS |
| | | | | | Olfactory receptor signature PR00245: M58-K79, F176-D190, Y237-A252, L273-L284, S290-S304 | BLIMPS-PRINTS |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | G-protein coupled receptors signature: F101-T145 | PROFILESCAN |
| | | | | | transmem_domain: F25-F49, F199-G218 | HMMER |
| | | | | | G_Protein_Receptor: A109-I125 | MOTIFS |
| 10 | 7477889CD1 | 319 | S294 S71 | N9 | 7 transmembrane receptor (rhodopsin family) 7tm_1: G45-Y293 | HMMER-PFAM |
| | | | | | G-PROTEIN COUPLED RECEPTORS DM00013\|P23270\|18-311: L29-H309 | BLAST-DOMO |
| | | | | | RECEPTOR OLFACTORY RECEPTORLIKE GPROTEIN COUPLED TRANSMEMBRANE GLYCOPROTEIN MULTIGENE FAMILY PD000921: L170-L248 | BLAST-PRODOM |
| | | | | | G-protein coupled receptor BL00237: K94-P133, E235-M261, A285-K301 | BLIMPS-BLOCKS |
| | | | | | Rhodopsin-like GPCR superfamily PR00237: L63-K84, F108-I130, V202-1225, A240-R264, T275-K301 | BLIMPS-PRINTS |

(continued)

| SEQ ID NO: | Incyte Polypeptide ID | Amino Acid Residues | Potential Phosphorylation Sites | Potential Glycosylation Sites | Signature Sequences, Domains and Motifs | Analytical Methods and Databases |
|---|---|---|---|---|---|---|
| | | | | | Olfactory receptor signature PR00245: L63-K84, I181-D195, F241-G256, S294-F308 | BLIMPS-PRINTS |
| | | | | | G-protein coupled receptors signature: Y106-G156 | PROFILESCAN |
| | | | | | transmembrane domains: G28-I51, V211-M231 | HMMER |
| | | | | | G_Protein_Receptor: T114-I130 | MOTIFS |

Table 4

| Polynucleotide SEQ ID NO: | Incyte Polynucleotide ID | Sequence Length | Selected Fragments | Sequence Fragments | 5' Position | 3' vPosition |
|---|---|---|---|---|---|---|
| 11 | 7474927CB1 | 2245 | 755-1136, 1-643, 2090-2116, 1-1710 | 5805033T6 (BONRFET03) | 1704 | 2168 |
| | | | | 7673613H1 (FIBPFEC01) | 1465 | 1986 |
| | | | | 5805033F6 (BONRFET03) | 1711 | 2245 |
| | | | | 55012833H1 | 1 | 780 |
| | | | | 6939423H1 (FTUBTUR01) | 1129 | 1492 |
| | | | | FL7474927_g2822142_g4995709 | 394 | 1455 |
| 12 | 7475194CB1 | 2729 | 1276-1513, 1-1175, 1622-2182, 2454-2729 | 7669623H1 (NOSEDIC02) | 2123 | 2729 |
| | | | | FL7475194_g7523967_000013_g5809686 | 1 | 2592 |
| 13 | 7475203CB1 | 2759 | 1672-1979, 1-608, 2089-2197, 740-1464, 2738-2759 | 55002220H2 | 1409 | 2048 |
| | | | | 55002212H2 | 1 | 553 |
| | | | | 55002204H2 | 1319 | 1965 |
| | | | | GBI:g7669574_edit | 53 | 2578 |
| | | | | g5110689 | 2292 | 2759 |
| | | | | 55002204J2 | 2229 | 2753 |
| 14 | 7474987CB1 | 945 | 555-639, 918-945 | GNN.g7283250_000011_008 | 1 | 945 |
| 15 | 5617631CB1 | 1511 | 1115-1154, 1-663, 1478-1511 | 6036056F8 (PITUNOT06) | 389 | 1119 |
| | | | | FL5617631-g7157997_000060-g6691937 | 558 | 1511 |
| | | | | 71700159V1 | 1 | 528 |
| 16 | 7472098CB1 | 954 | 1-106, 496-656 | FL7472096CB1_00001 | 1 | 954 |
| 17 | 7476775CB1 | 975 | 1-51, 573-975 | GNN:g7838156_edit | 1 | 975 |

(continued)

| Polynucleotide SEQ ID NO: | Incyte Polynucleotide ID | Sequence Length | Selected Fragments | Sequence Fragments | 5' Position | 3' vPosition |
|---|---|---|---|---|---|---|
| 18 | 7477937CB1 | 969 | 920-969 | GNN.g8568403_000027_002 | 1 | 969 |
| 19 | 7476798CB1 | 939 | 1-838, 885-939 | GNN.g8052176_000007_002 | 1 | 939 |
| 20 | 7477889CB1 | 960 | 1-24, 594-655, 810-960 | GNN.g8570522_024.edit | 1 | 960 |

Table 5

| Polynucleotide SEQ ID NO: | Incyte Project ID | Representative Library |
|---|---|---|
| 11 | 7474927CB1 | BONRFET03 |
| 12 | 7475194CB1 | NOSEDIC02 |
| 15 | 5617631CB1 | THYMNOR02 |

Table 6

| Library | Vector | Library Description |
|---|---|---|
| BONRFET03 | pINCY | Library was constructed using RNA isolated from rib bone tissue removed from a Caucasian male fetus who died from Patau's syndrome (trisomy 13) at 20-weeks' gestation. |
| NOSEDIC02 | PSPORT1 | This large size fractionated library was constructed using RNA isolated from nasal polyp tissue. |
| THYMNOR02 | pINCY | The library was constructed using RNA isolated from thymus tissue removed from a 2-year-old Caucasian female during a thymectomy and patch closure of left atrioventricular fistula. Pathology indicated there was no gross abnormality of the thymus. The patient presented with congenital heart abnormalities. Patient history included double inlet left ventricle and a rudimentary right ventricle, pulmonary hypertension, cyanosis, subaortic stenosis, seizures, and a fracture of the skull base. Family history included reflux neuropathy. |

Table 7

| **Program** | **Description** | **Reference** | **Parameter Threshold** |
|---|---|---|---|
| ABI FACTURA | A program that removes vector sequences and masks ambiguous bases in nucleic acid sequences. | Applied Biosystems, Foster City, CA. | |
| ABI/ARACEL FDF | A Fast Data Finder useful in comparing and annotating amino acid or nucleic acid sequences. | Applied Biosystems, Foster City, CA; Paracel Inc., Pasadena, CA. | Mismatch <50% |
| ABI AutoAssembler | A program that assembles nucleic acid sequences. | Applied Biosystems, Foster City, CA. | |
| BLAST | A Basic Local Alignment Search Tool useful in sequence similarity search for amino acid and nucleic acid sequences. BLAST includes five functions: blastp, blastn, blastx, tblastn, and tblastx. | Altschul, S.F. et al. (1990) J. Mol. Biol. 215:403-410; Altschul, S.F. et al. (1997) Nucleic Acids Res. 25: 3389-3402. | *ESTs:* Probability value= 1.0E-8 or less *Full Length sequences*: Probability value= 1.0E-10 or less |

(continued)

| Program | Description | Reference | Parameter Threshold |
|---|---|---|---|
| FASTA | A Pearson and Lipman algorithm that searches for similarity between a query sequence and a group of sequences of the same type. FASTA comprises as least five functions: fasta, tfasta, fastx, tfastx, and ssearch. | Pearson, W.R. and D.J. Lipman (1988) Proc. Natl. Acad Sci. USA 85: 2444-2448; Pearson, W.R. (1990) Methods Enzymol. 183:63-98; and Smith, T.F. and M.S. Waterman (1981) Adv. Appl. Math. 2:482-489. | *ESTs:* fasta E value=1.06E-6 *Assembled ESTs*: fasta Identity= 95% or greater and Match length=200 bases or greater; fastx E value=1.0E-8 or less *Full Length sequences:* fastx score=100 or greater |
| BLIMPS | A BLocks IMProved Searcher that matches a sequence against those in BLOCKS, PRINTS, DOMO, PRODOM, and PFAM databases to search for gene families, sequence homology, and structural fingerprint regions. | Henikoff, S. and J.G. Henikoff (1991) Nucleic Acids Res. 19: 6565-6572; Henikoff, J.G. and S. Henikoff (1996) Methods Enzymol. 266: 88-105; and Attwood, T.K. et al. (1997) J. Chem. Inf. Comput. Sci. 37:417-424. | Probability value= 1.0E-3 or less |
| HMMER | An algorithm for searching a query sequence against hidden Markov model (HMM)-based databases of protein family consensus sequences, such as PFAM. | Krogh, A. et al. (1994) J. Mol. Biol. 235:1501-1531; Sonnhammer, E.L.L. et al. (1988) Nucleic Acids Res.26: 320-322; Durbin, R. et al. (1998) Our World View, in a Nutshell, Cambridge Univ. Press, pp. 1-350. | *PFAM hits:* Probability value= 1.0E-3 or less *Signal peptide hits:* Score= 0 or greater |
| ProfileScan | An algorithm that searches for structural and sequence motifs in protein sequences that match sequence patterns defined in Prosite. | Gribskov, M. et al. (1988) CABIOS 4:61-66; Gribskov, M. et al. (1989) Methods Enzymol. 183:146-159; Bairoch, A. et al. (1997) Nucleic Acids Res. 25: 217-221. | Normalized quality score≥GCG-specified "HIGH" value for that particular Prosite motif. Generally, score=1.4-2.1. |
| Phred | A base-calling algorithm that examines automated sequencer traces with high sensitivity and probability. | Ewing, B. et al. (1998) Genome Res. 8:175-185; Ewing, B. and P. Green (1998) Genome Res. 8: 186-194. | |
| Phrap | A Phils Revised Assembly Program including SWAT and CrossMatch, programs based on efficient implementation of the Smith-Waterman algorithm, useful in searching sequence homology and assembling DNA sequences. | Smith, T.F. and M.S. Waterman (1981) Adv. Appl. Math. 2:482-489; Smith, T.F. and M.S. Waterman (1981) J. Mol. Biol. 147:195-197; and Green, P., University of Washington, Seattle, WA. | Score=120 or greater; Match length= 56 or greater |

(continued)

| Program | Description | Reference | Parameter Threshold |
|---|---|---|---|
| Consed | A graphical tool for viewing and editing Phrap assemblies. | Gordon, D. et al. (1998) Genome Res. 8:195-202. | |
| SPScan | A weight matrix analysis program that scans protein sequences for the presence of secretory signal peptides. | Nielson, H. et al. (1997) Protein Engineering 10:1-6; Claverie, J.M. and S. Audic (1997) CABIOS 12:431-439. | Score=3.5 or greater |
| TMAP | A program that uses weight matrices to delineate transmembrane segments on protein sequences and determine orientation. | Persson, B. and P. Argos (1994) J. Mol. Biol. 237: 182-192; Persson, B. and P. Argos (1996) Protein Sci. 5: 363-371. | |
| TMHMMER | A program that uses a hidden Markov model (HMM) to delineate transmembrane segments on protein sequences and determine orientation. | Sonnhammer, E.L. et al. (1998) Proc. Sixth Intl. Conf. on Intelligent Systems for Mol. Biol., Glasgow et al., eds., The Am. Assoc. for Artificial Intelligence Press, Menlo Park, CA, pp. 175-182. | |
| Motifs | A program that searches amino acid sequences for patterns that matched those defined in Prosite. | Bairoch, A. et al. (1997) Nucleic Acids Res. 25: 217-221; Wisconsin Package Program Manual, version 9, page M51-59, Genetics Computer Group, Madison, WI. | |

Table 8

| Tissues | Polynucleotide SEQ ID NO: | |
|---|---|---|
| | 14 | 15 |
| Breast, Fat, Skin | + | - |
| Muscle, Bone, Synovium, Connective tissue | - | - |
| Pancreas, Liver, Gallbladder | - | + |
| Brain: Amygdala, Thalamus, Hippocampus, Entorhinal cortex, Archaecortex | - | - |
| Brain: Striatum, Caudate nucleus, Putamen, Dentate nucleus, Globus pallidus, Substantia innominata, Ralphe magnus | - | - |
| Kidney, Fetal colon, Small intestine, Ileum, Esophagus | - | - |
| Fetal heart, Aorta, Coronary artery | - | - |
| Fetal lung, Adult lung | - | - |
| Placenta, Prostate, Uterus | - | - |
| Olfactory bulb | - | - |

## Claims

**1.** An isolated polypeptide comprising:

a) any of SEQ ID NOs: 3, 1-2 or 4-10;
b) an amino acid sequence at least 90% identical to any of SEQ ID NOs: 3, 1-2 or 4-10; or
c) a biologically active or immunogenic fragment of any of SEQ ID NOs: 3,1-2 or 4-10.

**2.** An isolated polynucleotide which comprises:

a) a sequence encoding a polypeptide according to claim 1;
b) a recombinant polynucleotide comprising a promoter sequence operably linked to a polynucleotide of (a);
c) any of SEQ ID NOs: 13, 11-12 or 14-20;
d) a sequence at least 90% identical to any of SEQ ID NOs: 13, 11-12 or 14-20;
e) a sequence complementary to a polynucleotide of c) or d); or
f) an RNA equivalent of c), d) or e); or
g) at least 60 contiguous nucleotides of a polynucleotide of c), d), e) or f).

**3.** An expression or cloning vector comprising a polynucleotide according to claim 2.

**4.** A host cell comprising or transformed with a recombinant polynucleotide according to claim 2 or a vector according to claim 3.

**5.** A transgenic organism comprising a recombinant polynucleotide according to claim 2.

**6.** A method for production of a polypeptide according to claim 1, the method comprising:

a) culturing a cell according to claim 4 under conditions suitable for expression of the polypeptide; and
b) recovering any polypeptide so expressed.

**7.** An antibody which specifically binds to a polypeptide according to claim, such as:

a) a polyclonal antibody;
b) a monoclonal antibody;

c) a chimeric antibody;
d) a single chain antibody;
e) a Fab fragment;
f) a $F(ab')_2$ fragment;
g) a humanized antibody;
h) a labelled antibody;
i) produced by screening a Fab expression library; or
j) produced by screening a recombinant immunoglobulin library.

**8.** A method for detection of a target polynucleotide according to claim 2 in a sample, the method comprising:

a) hybridizing the sample with a probe comprising at least 20 contiguous nucleotides complementary to the target polynucleotide and which probe specifically hybridizes to the target polynucleotide, under conditions which allow the formation of a hybridization complex between the probe and the target polynucleotide or fragments thereof; and
b) detecting the presence or absence of such hybridization complex(es), and, optionally, if present, the amount thereof,

optionally wherein the probe comprises at least 60 contiguous nucleotides.

**9.** A method for detection of a target polynucleotide according to claim 2 in a sample, the method comprising:

a) amplifying the target polynucleotide or fragment thereof using polymerase chain reaction amplification; and
b) detecting the presence or absence of the amplified target polynucleotide or fragment thereof, and, optionally, if present, the amount thereof, or

a method for detecting a polypeptide according to claim 1 in a sample, the method comprising:

a) incubating an antibody according to claim 7 or 8 with a sample under conditions which allow specific binding of the antibody and the polypeptide; and
b) detecting, if present, any specific binding, wherein specific binding indicates the presence of a polypeptide according to claim 1 in the sample.

**10.** A method of purifying a polypeptide according to claim 1 from a sample, the method comprising:

a) incubating an antibody according to claim 7 with a sample under conditions which allow specific binding of the antibody and the polypeptide; and
b) separating the antibody from the sample, thereby obtaining a purified polypeptide according to claim 1.

**11.** A method for screening a compound for effectiveness as an agonist of a polypeptide according to claim 1, the method comprising:

a) exposing a sample comprising a polypeptide according to claim 1 to a compound; and
b) detecting , if present, any agonist activity in the sample, or

a method for screening a compound for effectiveness as an antagonist of a polypeptide according to claim 1, the method comprising:

a) exposing a sample comprising a polypeptide according to claim 1 to a compound; and
b) detecting, if present, any antagonist activity in the sample.

**12.** A method of screening for a compound that specifically binds to a polypeptide according to claim 1, the method comprising:

a) combining a polypeptide according to claim 1 with at least one test compound under suitable conditions; and
b) detecting, if present, any binding of the polypeptide according to claim 1 to the test compound, thereby identifying a compound that specifically binds to the polypeptide according to claim 1, or

a method of screening for a compound that modulates the activity of a polypeptide according to claim 1, the method comprising:

a) combining the polypeptide according to claim 1 with at least one test compound under conditions permissive for the activity of the polypeptide;
b) assessing, if present, any activity of the polypeptide in the presence of the test compound; and
c) comparing the activity of the polypeptide in the presence of the test compound with the activity of the polypeptide in the absence of the test compound, wherein a change in the activity of the polypeptide in the presence of the test compound is indicative of a compound that modulates the activity of a polypeptide according to claim 1, or

a method for screening a compound for effectiveness in altering expression of a target polynucleotide according to claim 2, the method comprising:

a) exposing a sample comprising the target polynucleotide to a compound, under conditions suitable for the expression of the target polynucleotide;
b) detecting, if present, any altered expression of the target polynucleotide; and
c) comparing the expression of the target polynucleotide in the presence of varying amounts of the compound and in the absence of the compound.

**13.** A method for assessing toxicity of a test compound, the method comprising:

a) treating a biological sample containing nucleic acids with the test compound;
b) hybridizing the nucleic acids of the treated biological sample with a probe comprising at least 20 contiguous nucleotides of a polynucleotide according to claim 2 under conditions which allow the formation of a specific hybridization complex between the probe and a target polynucleotide in the biological sample, said target polynucleotide comprising a polynucleotide sequence according to claim 2 or a fragment thereof;
c) quantifying the amount, if any, of hybridization complex; and
d) comparing the amount of hybridization complex in the treated biological sample with the amount of hybridization complex in the untreated biological sample, wherein a difference in the amount of hybridization complex in the treated biological sample is indicative of toxicity of the test compound.

**14.** A diagnostic test for a condition or disease associated with the expression of PP in a biological sample, the method comprising:

a) combining the biological sample with an antibody according to claim 7 under conditions suitable for the antibody to bind the polypeptide and form an antibody:polypeptide complex; and
b) detecting, if present, any complex, wherein the presence of the complex correlates with the presence of the polypeptide in the biological sample.

**15.** A composition comprising a polypeptide according to claim 1, a polynucleotide according to claim 2, a vector according to claim 3, an antibody according to claim 7, an agonist compound or an antagonist compound identified by a method of claim 11 and a pharmaceutically acceptable excipient.

**16.** A method of preparing a polyclonal antibody according to claim 7 comprising:

a) providing an animal that has been immunized with a polypeptide of claim 1, or an immunogenic fragment thereof, under conditions suitable to elicit an antibody response;
b) isolating antibodies from the animal; and
c) screening the isolated antibodies with the polypeptide, thereby identifying a polyclonal antibody; or

a method of making a monoclonal antibody according to claim 7 comprising:

a) providing an animal that has been immunized with a polypeptide of claim 1, or an immunogenic fragment thereof, under conditions suitable to elicit an antibody response;
b) isolating antibody producing cells from the animal;
c) fusing the antibody producing cells with immortalized cells to form monoclonal antibody-producing hybridoma cells;

d) culturing the hybridoma cells; and
e) isolating from the culture monoclonal antibody which binds specifically to a polypeptide of claim 1.

**17.** A peptide nucleic acid, ribozyme, hybridisation probe, dipstick, membrane, pin or microarray comprising a polynucleotide according to claim 2.

**18.** A polypeptide according to claim 1, a polynucleotide according to claim 2, a vector according to claim 3, an antibody according to claim 7, a composition according to claim 21, an agonist or an antagonist identified by a method of claim 11 for use in a method of treatment of the human or animal body by therapy or diagnosis.

**19.** Use of a polypeptide according to claim 1, a polynucleotide according to claim 2, a vector according to claim 3, an antibody according to claim 7, or an agonist identified by a method of claim 11 in the manufacture of a medicament for treating a disease or condition associated with decreased expression of functional GCREC; or
use of a polypeptide according to claim 1, a polynucleotide according to claim 2, a vector according to claim 3, an antibody according to claim 7, or an antagonist identified by a method of claim 11 in the manufacture of a medicament for treating a disease or condition associated with overexpression of functional GCREC; or
use of an antibody according to claim 7 in the manufacture of a medicament for diagnosing a condition or disease associated with the expression of GCREC in a subject.

**20.** A polypeptide according to claim 1, a polynucleotide according to claim 2, a vector according to claim 3, an antibody according to claim 7, or an agonist identified by a method of claim 11 for treating a disease or condition associated with decreased expression of functional GCREC; or
a polypeptide according to claim 1, a polynucleotide according to claim 2, a vector according to claim 3, an antibody according to claim 7, or an antagonist identified by a method of claim 11 for treating a disease or condition associated with overexpression of functional GCREC; or
use of an antibody according to claim 7 for diagnosing a condition or disease associated with the expression of GCREC in a subject.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5837458 A
- US 5175383 A
- US 5767337 A
- US 5910434 A, Rigg
- US 5707618 A, Armentano
- US 5804413 A, DeLuca
- US 5932435 A, Atkins, D.
- US 5686242 A, Bruice, T.W.
- US 6022691 A, Bruice, T.W.
- US 5997848 A, Patton, J.S.
- US 5840484 A
- US 5474796 A, Brennan, T.M.
- WO 95251116 A, Baldeschweiler
- WO 9535505 A, Shalon, D.
- US 5605662 A, Heller, M.J.
- WO 8403564 A, Geysen
- US 60212483 B
- US 60213950 B
- US 60214062 B
- US 60216595 B
- US 60218936 B
- US 60219154 B
- US 5807522 A
- US 6057101 A, Nandabalan, K.


### Non-patent literature cited in the description


- **Strosberg, A.D.** *Eur. J. Biochem.,* 1991, vol. 196, 1-10
- **Coughlin, S.R.** *Curr. Opin. Cell Biol.,* 1994, vol. 6, 191-197
- **Watson, S. ; S. Arkinstall.** The G-protein Linked Receptor Facts Book. Academic Press, 1994, 2-6
- **Bolander, F.F.** Molecular Endocrinology. Academic Press, 1994, 162-176
- **Baldwin, J.M.** *Curr. Opin. Cell Biol.,* 1994, vol. 6, 180-190
- **Kilpatrick, G.J. et al.** *Trends Pharmacol. Sci.,* 1999, vol. 20, 294-301
- **Watson, S. ; S. Arkinstall.** The G-Protein Linked Receptor Facts Book. Academic Press, 1994, 7-919-2232-35130-131214-216221-222
- **Habert-Ortoli, E. et al.** *Proc. Natl. Acad. Sci. USA,* 1994, vol. 91, 9780-9783
- **Kask, K. et al.** *Life Sci.,* 1997, vol. 60, 1523-1533
- **Chun, J. et al.** *Cell Biochem. Biophys.,* 1999, vol. 30, 213-242
- **Raming, K. et al.** *Receptors Channels,* 1998, vol. 6, 141-151
- **Thomas, M.B. et al.** *Gene,* 1996, vol. 178, 1-5
- **McKnight, A.J. ; S. Gordon.** *J. Leukoc. Biol.,* 1998, vol. 63, 271-280
- **Parma, J. et al.** *Nature,* 1993, vol. 365, 649-651
- **Wilson, S. et al.** *Br. J. Pharmocol.,* 1998, vol. 125, 1387-1392
- **Stadel, J.M. et al.** *Trends Pharmacol. Sci.,* 1997, vol. 18, 430-437
- **Horn, F. ; G. Vriend.** *J. Mol. Med.,* 1998, vol. 76, 464-468
- **Schöneberg, T. et al.** *EMBO J.,* 1996, vol. 15, 1283-1291
- **Ho, G. ; R.G. MacKenzie.** *J. Biol. Chem.,* 1999, vol. 274, 35816-35822
- **Mannstadt, M. et al.** *Am. J. Physiol.,* 1999, vol. 277, F665-F675
- **Locati, M. ; P.M. Murphy.** *Annu. Rev. Med.,* 1999, vol. 50, 425-440
- **Higgins, D.G. ; P.M. Sharp.** *CABIOS,* 1989, vol. 5, 151-153
- **Higgins, D.G. et al.** *CABIOS,* 1992, vol. 8, 189-191
- **Altschul, S.F. et al.** *J. Mol. Biol.,* 1990, vol. 215, 403-410
- **Sambrook, J. et al.** Molecular Cloning: A Laboratory Manual. Cold Spring Harbor Press, 1989, vol. 1-3
- **Ausubel, F.M. et al.** Current Protocols in Molecular Biology. Greene Publ. Assoc. & Wiley-Intersciences, 1987
- **Innis, M. et al.** PCR Protocols, A Guide to Methods and Applications. Academic Press, 1990
- **Wahl, G.M. ; S.L. Berger.** *Methods Enzymol.,* 1987, vol. 152, 399-407
- **Kimmel, A.R.** *Methods Enzymol.,* 1987, vol. 152, 507-511
- **Ausubel, F.M.** Short Protocols in Molecular Biology. John Wiley & Sons, 1997
- **Meyers, R.A.** Molecular Biology and Biotechnology. Wiley VCH, 1995, 856-853

- **Sarkar, G.** *PCR Methods Applic.,* 1993, vol. 2, 318-322
- **Triglia, T. et al.** *Nucleic Acids Res.,* 1988, vol. 16, 8186
- **Lagerstrom, M. et al.** *PCR Methods Applic.,* 1991, vol. 1, 111-119
- **Parker, J.D. et al.** *Nucleic Acids Res.,* 1991, vol. 19, 3055-3060
- **Chang, C.-C. et al.** *Nat. Biotechnol.,* 1999, vol. 17, 793-797
- **Christians, F.C. et al.** *Nat. Biotechnol.,* 1999, vol. 17, 259-264
- **Crameri, A. et al.** *Nat. Biotechnol.,* 1996, vol. 14, 315-319
- **Caruthers, M.H. et al.** *Nucleic Acids Symp. Ser.,* 1980, vol. 7, 215-223
- **Horn, T. et al.** *Nucleic Acids Symp. Ser.,* 1980, vol. 7, 225-232
- **Creighton, T.** Proteins, Structures and Molecular Properties. WH Freeman, 1984, 55-60
- **Roberge, J.Y. et al.** *Science,* 1995, vol. 269, 202-204
- **Chiez, R.M. ; F.Z. Regnier.** *Methods Enzymol.,* 1990, vol. 182, 392-421
- **Scharf, D. et al.** *Results Probl. Cell Differ.,* 1994, vol. 20, 125-162
- **Sambrook, J. et al.** Molecular Cloning, A Laboratory Manual. Cold Spring Harbor Press, 1989
- **Ausubel, F.M. et al.** Current Protocols in Molecular Biology. John Wiley & Sons, 1995
- **Van Heeke, G. ; S.M. Schuster.** *J. Biol. Chem.,* 1989, vol. 264, 5503-5509
- **Engelhard, E.K. et al.** *Proc. Natl. Acad. Sci. USA,* 1994, vol. 91, 3224-3227
- **Sandig, V. et al.** *Hum. Gene Ther.,* 1996, vol. 7, 1937-1945
- **Takamatsu, N.** *EMBO J.,* 1987, vol. 6, 307-311
- The McGraw Hill Yearbook of Science and Technology. McGraw Hill, 1992, 191-196
- **Logan, J. ; T. Shenk.** *Proc. Natl. Acad. Sci. USA,* 1984, vol. 81, 3655-3659
- **Harrington, J.J. et al.** *Nat. Genet.,* 1997, vol. 15, 345-355
- **Di Nicola, M. et al.** *Cancer Gen. Ther.,* 1998, vol. 5 (6), 350-356
- **Yu, M. et al.** *Proc. Natl. Acad. Sci. USA,* 1993, vol. 90 (13), 6340-6344
- **Buller, R.M. et al.** *Nature,* 1985, vol. 317 (6040), 813-815
- **McGregor, D.P. et al.** *Mol. Immunol.,* 1994, vol. 31 (3), 219-226
- **Verma, I.M. ; N. Somia.** *Nature,* 1997, vol. 389, 239-242
- **Bitter, G.A. et al.** *Methods Enzymol.,* 1987, vol. 153, 516-544
- **Scorer, C.A. et al.** *Bio/Technology,* 1994, vol. 12, 181-184
- **Coruzzi, G. et al.** *EMBO J.,* 1984, vol. 3, 1671-1680
- **Broglie, R. et al.** *Science,* 1984, vol. 224, 838-843
- **Winter, J. et al.** *Results Probl. Cell Differ.,* 1991, vol. 17, 85-105
- **Wigler, M. et al.** *Cell,* 1977, vol. 11, 223-232
- **Lowy, I. et al.** *Cell,* 1980, vol. 22, 817-823
- **Wigler, M. et al.** *Proc. Natl. Acad. Sci. USA,* 1980, vol. 77, 3567-3570
- **Colbere-Garapin, F. et al.** *J. Mol. Biol.,* 1981, vol. 150, 1-14
- **Hartman, S.C. ; R.C. Mulligan.** *Proc. Natl. Acad. Sci. USA,* 1988, vol. 85, 8047-8051
- **Rhodes, C.A.** *Methods Mol. Biol.,* 1995, vol. 55, 121-131
- **Hampton, R. et al.** Serological Methods, a Laboratory Manual. APS Press, 1990
- **Coligan, J.E. et al.** Current Protocols in Immunology. Greene Pub. Associates and Wiley-Interscience, 1997
- **Pound, J.D.** Immunochemical Protocols. Humana Press, 1998
- **Coligan, J.E. et al.** Current Protocols in Immunology. 1991, vol. 1
- **Capecchi, M.R.** *Science,* 1989, vol. 244, 1288-1292
- **Marth, J.D.** *Clin. Invest.,* 1996, vol. 97, 1999-2002
- **Wagner, K.U. et al.** *Nucleic Acids Res.,* 1997, vol. 25, 4323-4330
- **Thomson, J.A. et al.** *Science,* 1998, vol. 282, 1145-1147
- **Janne, J. et al.** *Biotechnol. Annu. Rev.,* 1998, vol. 4, 55-74
- **Kohler, G. et al.** *Nature,* 1975, vol. 256, 495-497
- **Kozbor, D. et al.** *J. Immunol. Methods,* 1985, vol. 81, 31-42
- **Cote, R.J. et al.** *Proc. Natl. Acad. Sci. USA,* 1983, vol. 80, 2026-2030
- **Cole, S.P. et al.** *Mol. Cell Biol.,* 1984, vol. 62, 109-120
- **Morrison, S.L. et al.** *Proc. Natl. Acad. Sci. USA,* 1984, vol. 81, 6851-6855
- **Neuberger, M.S. et al.** *Nature,* 1984, vol. 312, 604-608
- **Takeda, S. et al.** *Nature,* 1985, vol. 314, 452-454
- **Burton, D.R.** *Proc. Natl. Acad. Sci. USA,* 1991, vol. 88, 10134-10137
- **Orlandi, R. et al.** *Proc. Natl. Acad. Sci. USA,* 1989, vol. 86, 3833-3837
- **Winter, G. et al.** *Nature,* 1991, vol. 349, 293-299
- **Huse, W.D. et al.** *Science,* 1989, vol. 246, 1275-1281

- **Catty, D.** Antibodies, Volume I: A Practical Approach. IRL Press, 1988
- **Liddell, J.E. ; A. Cryer.** A Practical Guide to Monoclonal Antibodies. John Wiley & Sons, 1991
- Antisense Therapeutics. Humana Press Inc, 1996
- **Slater, J.E. et al.** *J. Allergy Clin. Immunol.,* 1998, vol. 102 (3), 469-475
- **Scanlon, K.J. et al.** *J. ALLERGY CLIN. IMMUNOL.,* 1995, vol. 9 (13), 1288-1296
- **Miller, A.D.** *Blood,* 1990, vol. 76, 271
- **Uckert, W. ; W. Walther.** *Pharmacol. Ther.,* vol. 63 (3), 323-347
- **Rossi, J.J.** *Br. Med. Bull.,* vol. 51 (1), 217-225
- **Boado, R.J. et al.** *J. Pharm. Sci.,* 1998, vol. 87 (11), 1308-1315
- **Morris, M.C. et al.** *Nucleic Acids Res.,* 1997, vol. 25 (14), 2730-2736
- **Cavazzana-Calvo, M. et al.** *Science,* 2000, vol. 288, 669-672
- **Blaese, R.M. et al.** *Science,* 1995, vol. 270, 475-480
- **Bordignon, C. et al.** *Science,* 1995, vol. 270, 470-475
- **Zabner, J. et al.** *Cell,* 1993, vol. 75, 207-216
- **Crystal, R.G. et al.** *Hum. Gene Therapy,* 1995, vol. 6, 643-666
- **Crystal, R.G. et al.** *Hum. Gene Therapy,* 1995, vol. 6, 667-703
- **Crystal, R.G.** *Science,* 1995, vol. 270, 404-410
- **Baltimore, D.** *Nature,* 1988, vol. 335, 395-396
- **Poeschla, E. et al.** *Proc. Natl. Acad. Sci. USA.,* 1996, vol. 93, 11395-11399
- **Morgan, R.A. ; W.F. Anderson.** *Annu. Rev. Biochem.,* 1993, vol. 62, 191-217
- **Ivics, Z.** *Cell,* 1997, vol. 91, 501-510
- **Boulay, J-L. ; H. Récipon.** *Curr. Opin. Biotechnol.,* 1998, vol. 9, 445-450
- **Gossen, M. ; H. Bujard.** *Proc. Natl. Acad. Sci. USA,* 1992, vol. 89, 5547-5551
- **Gossen, M. et al.** *Science,* 1995, vol. 268, 1766-1769
- **Rossi, F.M.V. ; H.M. Blau.** *Curr. Opin. Biotechnol.,* vol. 9, 451-456
- **Graham, F.L. ; A.J. Eb.** *Virology,* 1973, vol. 52, 456-467
- **Neumann, E. et al.** *EMBO J.,* 1982, vol. 1, 841-845
- **Riviere, I. et al.** *Proc. Natl. Acad. Sci. USA,* 1995, vol. 92, 6733-6737
- **Armentano, D. et al.** *J. Virol.,* 1987, vol. 61, 1647-1650
- **Bender, M.A. et al.** *J. Virol.,* 1987, vol. 61, 1639-1646
- **Adam, M.A. ; A.D. Miller.** *J. Virol.,* 1988, vol. 62, 3802-3806
- **Dull, T. et al.** *J. Virol.,* 1998, vol. 72, 8463-8471
- **Zufferey, R. et al.** *J. Virol.,* 1998, vol. 72, 9873-9880
- **Ranga, U. et al.** *J. Virol.,* 1997, vol. 71, 7020-7029
- **Bauer, G. et al.** *Blood,* 1997, vol. 89, 2259-2267
- **Bonyhadi, M.L.** *J. Virol.,* 1997, vol. 71, 4707-4716
- **Ranga, U. et al.** *Proc. Natl. Acad. Sci. USA,* 1998, vol. 95, 1201-1206
- **Su, L.** *Blood,* 1997, vol. 89, 2283-2290
- **Csete, M.E. et al.** *Transplantation,* 1995, vol. 27, 263-268
- **Antinozzi, P.A. et al.** *Annu. Rev. Nutr.,* 1999, vol. 19, 511-544
- **Verma, I.M. ; N. Somia.** *Nature,* 1997, vol. 18 (389), 239-242
- **Liu, X. et al.** *Exp. Eye Res.,* 1999, vol. 169, 385-395
- **Goins, W.F. et al.** *J. Virol.,* 1999, vol. 73, 519-532
- **Xu, H. et al.** *Dev. Biol.,* 1994, vol. 163, 152-161
- **Garoff, H. ; K.-J. Li.** *Curr. Opin. Biotechnol.,* 1998, vol. 9, 464-469
- **Dryga, S.A. et al.** *Virology,* 1997, vol. 228, 74-83
- **Gee, J.E. ; Huber, B.E. ; B.I. Carr et al.** Molecular and Immunologic Approaches. Futura Publishing, 1994, 163-177
- **Amdt, G.M. et al.** *Nucleic Acids Res.,* 2000, vol. 28, E15
- **Clarke, M.L. et al.** *Biochem. Biophys. Res. Commun.,* 2000, vol. 268, 8-13
- **Goldman, C.K. et al.** *Nat. Biotechnol.,* 1997, vol. 15, 462-466
- Remington's Pharmaceutical Sciences. Maack Publishing
- **Schwarze, S.R. et al.** *Science,* 1999, vol. 285, 1569-1572
- **Melby, P.C. et al.** *J. Immunol. Methods,* 1993, vol. 159, 235-244
- **Duplaa, C. et al.** *Anal. Biochem.,* 1993, vol. 212, 229-236
- **Nuwaysir, E.F. et al.** *Mol. Carcinog.,* 1999, vol. 24, 153-159
- **Steiner, S. ; N.L. Anderson.** *Toxicol. Lett.,* 2000, vol. 112-113, 467-471
- **Lueking, A. et al.** *Anal. Biochem.,* 1999, vol. 270, 103-111
- **Mendoze, L.G. et al.** *Biotechniques,* 1999, vol. 27, 778-788
- **Anderson, N.L. ; J. Seilhamer.** *Electrophoresis,* 1997, vol. 18, 533-537
- **Schena, M. et al.** *Proc. Natl. Acad. Sci. USA,* 1996, vol. 93, 10614-10619

- **Heller, R.A. et al.** *Proc. Natl. Acad. Sci. USA,* 1997, vol. 94, 2150-2155
- DNA Microarrays: A Practical Approach. Oxford University Press, 1999
- **Price, C.M.** *Blood Rev.,* 1993, vol. 7, 127-134
- **Trask, B.J.** *Trends Genet.,* 1991, vol. 7, 149-154
- **Lander, E.S. ; D. Botstein.** *Proc. Natl. Acad. Sci. USA,* 1986, vol. 83, 7353-7357
- **Gatti, R.A. et al.** *Nature,* 1988, vol. 336, 577-580
- **Rao, V.B.** *Anal. Biochem.,* 1994, vol. 216, 1-14
- **Eddy, S.R.** *Curr. Opin. Struct. Biol.,* 1996, vol. 6, 361-365
- **Burge, C. ; S. Karlin.** *J. Mol. Biol.,* 1997, vol. 268, 78-94
- **Burge, C. ; S. Karlin.** *Curr. Opin. Struct. Biol.,* 1998, vol. 8, 346-354
- **Schena, M. et al.** *Science,* 1995, vol. 270, 467-470
- **Shalon, D. et al.** *Genome Res.,* 1996, vol. 6, 639-645
- **Marshall, A. ; J. Hodgson.** *Nat. Biotechnol.,* 1998, vol. 16, 27-31
- **Ormerod, M.G.** *Flow Cytometry,* 1994
- **Harrington, M.G.** *Methods Enzymol.,* 1990, vol. 182, 488-495
- **Bolton A.E. ; W.M. Hunter.** *Biochem. J.,* 1973, vol. 133, 529-539
- **Fields, S. ; O. Song.** *Nature,* 1989, vol. 340, 245-246
- **Kobilka, B.K. et al.** *Science,* 1988, vol. 240, 1310-1316
- **Conklin, B.R. et al.** *Cell,* 1993, vol. 73, 631-641
- **de la Fuente, M.A. et al.** *Blood,* 1997, vol. 90, 2398-2405
- Growth Factors: A Practical Approach. Oxford University Press, 1993, 73
- **Gaudin, P. et al.** *J. Biol. Chem.,* 1998, vol. 273, 4990-4996
- **Milligan, G. et al.** *Trends Pharmacol. Sci.,* 1996, vol. 17, 235-237
- **Offermanns, S. ; M.I. Simon.** *J. Biol. Chem.,* 1995, vol. 270, 15175-15180
- **Broach, J.R. ; J. Thorner.** *Nature,* 1996, vol. 384, 14-16
- **Garrett, J.E. et al.** *J. Biol. Chem.,* 1995, vol. 270, 12919-12925
- **Freichel, M. et al.** *Endocrinology,* 1996, vol. 137, 3842-3848
- **Zheng, C. et al.** *Neuron,* 2000, vol. 26, 81-91
- **Bulger, M. et al.** *Proc. Natl. Acad. Sci. USA,* 1999, vol. 96, 5129-5134
- **Nef, S. ; P. Nef.** *Proc. Natl. Acad. Sci. USA,* 1997, vol. 94, 4766-4771
- **Altschul, S.F. et al.** ESTs: Probability value= 1.0E-8 sequence similarity search for amino acid. *J. Mol. Biol.,* 1990, vol. 215, 403-410
- **Altschul, S.F. et al.** less nucleic acid sequences. BLAST includes five. *Nucleic Acids Res.,* 1997, vol. 25, 3389-3402
- **Ewing, B. ; P. Green.** *Genome Res.,* 1998, vol. 8, 186-194
- **Gordon, D. et al.** *Genome Res.,* 1998, vol. 8, 195-202
- **Nielson, H. et al.** *Protein Engineering,* 1997, vol. 10, 1-6
- **Claverie, J.M. ; S. Audic.** *CABIOS,* 1997, vol. 12, 431-439
- **Persson, B. ; P. Argos.** *J. Mol. Biol.,* 1994, vol. 237, 182-192
- **Persson, B. ; P. Argos.** *Protein Sci.,* 1996, vol. 5, 363-371
- Proc. Sixth Intl. Conf. on Intelligent Systems for Mol. Biol. **Sonnhammer, E.L. et al.** The Am. Assoc. for Artificial. 1998, 175-182
- **Bairoch, A. et al.** *Nucleic Acids Res.,* 1997, vol. 25, 217-221

```
<110> INCYTE GENOMICS, INC.
      LAL, Preeti
      GRAUL, Richard
      HAFALIA, April J.A.
      WALIA, Narinder K.
      THORNTON, Michael
      NGUYEN, Danniel B.
      LU, Yan
      GANDHI, Ameena R.
      PATTERSON, Chandra
      KALLICK, Deborah A.
      BAUGHN, Mariah R.
      RAMKUMAR, Jayalaxmi
      TRIBOULEY, Catherine M.
      LEE, Ernestine A.
      DING, Li
      BURFORD, Neil
      YAO, Monique G.
      YANG, Junming
      GRIFFIN, Jennifer A.

<120> G-PROTEIN COUPLED RECEPTORS

<130> SF-0795 PCT

<140> To Be Assigned
<141> Herewith

<150> 60/212,483; 60/213,950; 60/214,062; 60/216,595; 60/218,936;
      60/219,154
<151> 2000-06-16; 2000-06-23; 2000-06-26; 2000-07-07; 2000-07-14;
      2000-07-19

<160> 20
<170> PERL Program

<210> 1
<211> 353
<212> PRT
<213> Homo sapiens

<220>
<221> misc_feature
<223> Incyte ID No: 7474927CD1

<400> 1
Met Val Gly Asp Thr Leu Lys Leu Leu Ser Pro Leu Met Thr Arg
 1               5                  10                  15
Tyr Phe Phe Leu Leu Phe Tyr Ser Thr Asp Ser Ser Asp Leu Asn
                20                  25                  30
Glu Asn Gln His Pro Leu Asp Phe Asp Glu Met Ala Phe Gly Lys
                35                  40                  45
Val Lys Ser Gly Ile Ser Phe Leu Ile Gln Thr Gly Val Gly Ile
                50                  55                  60
Leu Gly Asn Ser Phe Leu Leu Cys Phe Tyr Asn Leu Ile Leu Phe
                65                  70                  75
Thr Gly His Lys Leu Arg Pro Thr Asp Leu Ile Leu Ser Gln Leu
                80                  85                  90
Ala Leu Ala Asn Ser Met Val Leu Phe Phe Lys Gly Ile Pro Gln
                95                 100                 105
Thr Met Ala Ala Phe Gly Leu Lys Tyr Leu Leu Asn Asp Thr Gly
               110                 115                 120
```

Cys Lys Phe Val Phe Tyr Tyr His Arg Val Gly Thr Arg Val Ser
125 130 135
Leu Ser Thr Ile Cys Leu Leu Asn Gly Phe Gln Ala Ile Lys Leu
140 145 150
Asn Pro Ser Ile Cys Arg Trp Met Glu Ile Lys Ile Arg Ser Pro
155 160 165
Arg Phe Ile Asp Phe Cys Cys Leu Leu Cys Trp Ala Pro His Val
170 175 180
Leu Met Asn Ala Ser Val Leu Leu Leu Val Asn Gly Pro Leu Asn
185 190 195
Ser Lys Asn Ser Ser Ala Lys Asn Asn Tyr Gly Tyr Cys Ser Tyr
200 205 210
Lys Ala Ser Lys Arg Phe Ser Ser Leu His Ala Val Leu Tyr Phe
215 220 225
Ser Pro Asp Phe Met Ser Leu Gly Phe Met Val Trp Ala Ser Gly
230 235 240
Ser Met Val Phe Phe Leu Tyr Arg His Lys Gln Gln Val Gln His
245 250 255
Asn His Ser Asn Arg Leu Ser Cys Arg Pro Ser Gln Glu Ala Arg
260 265 270
Ala Thr His Thr Ile Met Val Leu Val Ser Ser Phe Phe Val Phe
275 280 285
Tyr Ser Val His Ser Phe Leu Thr Ile Trp Thr Thr Val Val Ala
290 295 300
Asn Pro Gly Gln Trp Ile Val Thr Asn Ser Val Leu Val Ala Ser
305 310 315
Cys Phe Pro Ala Arg Ser Pro Phe Val Leu Ile Met Ser Asp Thr
320 325 330
His Ile Ser Gln Phe Cys Phe Ala Cys Arg Thr Arg Lys Thr Leu
335 340 345
Phe Pro Asn Leu Val Val Met Pro
350

<210> 2
<211> 863
<212> PRT
<213> Homo sapiens

<220>
<221> misc_feature
<223> Incyte ID No: 7475194CD1

<400> 2
Met Leu Gly Pro Ala Val Leu Gly Leu Ser Leu Trp Ala Leu Leu
1 5 10 15
His Pro Gly Thr Gly Ala Pro Leu Cys Leu Ser Gln Gln Leu Arg
20 25 30
Met Lys Gly Asp Tyr Val Leu Gly Gly Leu Phe Pro Leu Gly Glu
35 40 45
Ala Glu Glu Ala Gly Leu Arg Ser Arg Thr Arg Pro Ser Ser Pro
50 55 60
Val Cys Thr Arg Phe Ser Ser Asn Gly Leu Leu Trp Ala Leu Ala
65 70 75
Met Lys Met Ala Val Glu Glu Ile Asn Asn Lys Ser Asp Leu Leu
80 85 90
Pro Gly Leu Arg Leu Gly Tyr Asp Leu Phe Asp Thr Cys Ser Glu
95 100 105
Pro Val Val Ala Met Lys Pro Ser Leu Met Phe Leu Ala Lys Ala
110 115 120
Gly Ser Arg Asp Ile Ala Ala Tyr Cys Asn Tyr Thr Gln Tyr Gln
125 130 135
Pro Arg Val Leu Ala Val Ile Gly Pro His Ser Ser Glu Leu Ala
140 145 150

Met Val Thr Gly Lys Phe Phe Ser Phe Phe Leu Met Pro Gln Val
155 160 165
Ser Tyr Gly Ala Ser Met Glu Leu Leu Ser Ala Arg Glu Thr Phe
170 175 180
Pro Ser Phe Phe Arg Thr Val Pro Ser Asp Arg Val Gln Leu Thr
185 190 195
Ala Ala Ala Glu Leu Leu Gln Glu Phe Gly Trp Asn Trp Val Ala
200 205 210
Ala Leu Gly Ser Asp Asp Glu Tyr Gly Arg Gln Gly Leu Ser Ile
215 220 225
Phe Ser Ala Leu Ala Ala Ala Arg Gly Ile Cys Ile Ala His Glu
230 235 240
Gly Leu Val Pro Leu Pro Arg Ala Asp Asp Ser Arg Leu Gly Lys
245 250 255
Val Gln Asp Val Leu His Gln Val Asn Gln Ser Ser Val Gln Val
260 265 270
Val Leu Leu Phe Ala Ser Val His Ala Ala His Ala Leu Phe Asn
275 280 285
Tyr Ser Ile Ser Ser Arg Leu Ser Pro Lys Val Trp Val Ala Ser
290 295 300
Glu Ala Trp Leu Thr Ser Asp Leu Val Met Gly Leu Pro Gly Met
305 310 315
Ala Gln Met Gly Thr Val Leu Gly Phe Leu Gln Arg Gly Ala Gln
320 325 330
Leu His Glu Phe Pro Gln Tyr Val Lys Thr His Leu Ala Leu Ala
335 340 345
Thr Asp Pro Ala Phe Cys Ser Ala Leu Gly Glu Arg Glu Gln Gly
350 355 360
Leu Glu Glu Asp Val Val Gly Gln Arg Cys Pro Gln Cys Asp Cys
365 370 375
Ile Thr Leu Gln Asn Val Ser Ala Gly Leu Asn His His Gln Thr
380 385 390
Phe Ser Val Tyr Ala Ala Val Tyr Ser Val Ala Gln Ala Leu His
395 400 405
Asn Thr Leu Gln Cys Asn Ala Ser Gly Cys Pro Ala Gln Asp Pro
410 415 420
Val Lys Pro Trp Gln Leu Leu Glu Asn Met Tyr Asn Leu Thr Phe
425 430 435
His Val Gly Gly Leu Pro Leu Arg Phe Asp Ser Ser Gly Asn Val
440 445 450
Asp Met Glu Tyr Asp Leu Lys Leu Trp Val Trp Gln Gly Ser Val
455 460 465
Pro Arg Leu His Asp Val Gly Arg Phe Asn Gly Ser Leu Arg Thr
470 475 480
Glu Arg Leu Lys Ile Arg Trp His Thr Ser Asp Asn Gln Pro Ser
485 490 495
Arg Ala Arg Pro Gln Ala Cys Ala Gln Lys Pro Val Ser Arg Cys
500 505 510
Ser Arg Gln Cys Gln Glu Gly Gln Val Arg Arg Val Lys Gly Phe
515 520 525
His Ser Cys Cys Tyr Asp Cys Val Asp Cys Glu Ala Gly Ser Tyr
530 535 540
Arg Gln Asn Pro Asp Asp Ile Ala Cys Thr Phe Cys Gly Gln Asp
545 550 555
Glu Trp Ser Pro Glu Arg Ser Thr Arg Cys Phe Arg Arg Arg Ser
560 565 570
Arg Phe Leu Ala Trp Gly Glu Pro Ala Val Leu Leu Leu Leu Leu
575 580 585
Leu Leu Ser Leu Ala Leu Gly Leu Val Leu Ala Ala Leu Gly Leu
590 595 600
Phe Val His His Arg Asp Ser Pro Leu Val Gln Ala Ser Gly Gly
605 610 615
Pro Leu Ala Cys Phe Gly Leu Val Cys Leu Gly Leu Val Cys Leu

```
                    620                 625                 630
Ser Val Leu Leu Phe Pro Gly Gln Pro Ser Pro Ala Arg Cys Leu
                    635                 640                 645
Ala Gln Gln Pro Leu Ser His Leu Pro Leu Thr Gly Cys Leu Ser
                    650                 655                 660
Thr Leu Phe Leu Gln Ala Ala Glu Ile Phe Val Glu Ser Glu Leu
                    665                 670                 675
Pro Leu Ser Trp Ala Asp Arg Leu Ser Gly Cys Leu Arg Gly Pro
                    680                 685                 690
Trp Ala Trp Leu Val Val Leu Leu Ala Met Leu Val Glu Val Ala
                    695                 700                 705
Leu Cys Thr Trp Tyr Leu Val Ala Phe Pro Pro Glu Val Val Thr
                    710                 715                 720
Asp Trp His Met Leu Pro Thr Glu Ala Leu Val His Cys Arg Thr
                    725                 730                 735
Arg Ser Trp Val Ser Phe Gly Leu Ala His Ala Thr Asn Ala Thr
                    740                 745                 750
Leu Ala Phe Leu Cys Phe Leu Gly Thr Phe Leu Val Arg Ser Gln
                    755                 760                 765
Pro Gly Arg Tyr Asn Arg Ala Arg Gly Leu Thr Phe Ala Met Leu
                    770                 775                 780
Ala Tyr Phe Ile Thr Trp Val Ser Phe Val Pro Leu Leu Ala Asn
                    785                 790                 795
Val Gln Val Val Leu Arg Pro Ala Val Gln Met Gly Ala Leu Leu
                    800                 805                 810
Leu Cys Val Leu Gly Ile Leu Ala Ala Phe His Leu Pro Arg Cys
                    815                 820                 825
Tyr Leu Leu Met Arg Gln Pro Gly Leu Asn Thr Pro Glu Phe Phe
                    830                 835                 840
Leu Gly Gly Gly Pro Gly Asp Ala Gln Gly Gln Asn Asp Gly Asn
                    845                 850                 855
Thr Gly Asn Gln Gly Lys His Glu
                    860

<210> 3
<211> 841
<212> PRT
<213> Homo sapiens

<220>
<221> misc_feature
<223> Incyte ID No: 7475203CD1

<400> 3
Met Leu Leu Cys Thr Ala Arg Leu Val Gly Leu Gln Leu Leu Ile
  1                 5                  10                  15
Ser Cys Cys Trp Ala Phe Ala Cys His Ser Thr Glu Ser Ser Pro
                    20                  25                  30
Asp Phe Thr Leu Pro Gly Asp Tyr Leu Leu Ala Gly Leu Phe Pro
                    35                  40                  45
Leu His Ser Gly Cys Leu Gln Val Arg His Arg Pro Glu Val Thr
                    50                  55                  60
Leu Cys Asp Arg Ser Cys Ser Phe Asn Glu His Gly Tyr His Leu
                    65                  70                  75
Phe Gln Ala Met Arg Leu Gly Val Glu Glu Ile Asn Asn Ser Thr
                    80                  85                  90
Ala Leu Leu Pro Asn Ile Thr Leu Gly Tyr Gln Leu Tyr Asp Val
                    95                 100                 105
Cys Ser Asp Ser Ala Asn Val Tyr Ala Thr Leu Arg Val Leu Ser
                    110                115                 120
Leu Pro Gly Gln His His Ile Glu Leu Gln Gly Asp Leu Leu His
                    125                130                 135
Tyr Ser Pro Thr Val Leu Ala Val Ile Gly Pro Asp Ser Thr Asn
```

```
                    140                     145                     150
Arg Ala Ala Thr Thr Ala Ala Leu Leu Ser Pro Phe Leu Val Pro
                    155                     160                     165
Met Ile Ser Tyr Ala Ala Ser Ser Glu Thr Leu Ser Val Lys Arg
                    170                     175                     180
Gln Tyr Pro Ser Phe Leu Arg Thr Ile Pro Asn Asp Lys Tyr Gln
                    185                     190                     195
Val Glu Thr Met Val Leu Leu Leu Gln Lys Phe Gly Trp Thr Trp
                    200                     205                     210
Ile Ser Leu Val Gly Ser Ser Asp Asp Tyr Gly Gln Leu Gly Val
                    215                     220                     225
Gln Ala Leu Glu Asn Gln Ala Thr Gly Gln Gly Ile Cys Ile Ala
                    230                     235                     240
Phe Lys Asp Ile Met Pro Phe Ser Ala Gln Val Gly Asp Glu Arg
                    245                     250                     255
Met Gln Cys Leu Met Arg His Leu Ala Gln Ala Gly Ala Thr Val
                    260                     265                     270
Val Val Val Phe Ser Ser Arg Gln Leu Ala Arg Val Phe Phe Glu
                    275                     280                     285
Ser Val Val Leu Thr Asn Leu Thr Gly Lys Val Trp Val Ala Ser
                    290                     295                     300
Glu Ala Trp Ala Leu Ser Arg His Ile Thr Gly Val Pro Gly Ile
                    305                     310                     315
Gln Arg Ile Gly Met Val Leu Gly Val Ala Ile Gln Lys Arg Ala
                    320                     325                     330
Val Pro Gly Leu Lys Ala Phe Glu Glu Ala Tyr Ala Arg Ala Asp
                    335                     340                     345
Lys Lys Ala Pro Arg Pro Cys His Lys Gly Ser Trp Cys Ser Ser
                    350                     355                     360
Asn Gln Leu Cys Arg Glu Cys Gln Ala Phe Met Ala His Thr Met
                    365                     370                     375
Pro Lys Leu Lys Ala Phe Ser Met Ser Ser Ala Tyr Asn Ala Tyr
                    380                     385                     390
Arg Ala Val Tyr Ala Val Ala His Gly Leu His Gln Leu Leu Gly
                    395                     400                     405
Cys Ala Ser Gly Ala Cys Ser Arg Gly Arg Val Tyr Pro Trp Gln
                    410                     415                     420
Leu Leu Glu Gln Ile His Lys Val His Phe Leu Leu His Lys Asp
                    425                     430                     435
Thr Val Ala Phe Asn Asp Asn Arg Asp Pro Leu Ser Ser Tyr Asn
                    440                     445                     450
Ile Ile Ala Trp Asp Trp Asn Gly Pro Lys Trp Thr Phe Thr Val
                    455                     460                     465
Leu Gly Ser Ser Thr Trp Ser Pro Val Gln Leu Asn Ile Asn Glu
                    470                     475                     480
Thr Lys Ile Gln Trp His Gly Lys Asp Asn Gln Val Pro Lys Ser
                    485                     490                     495
Val Cys Ser Ser Asp Cys Leu Glu Gly His Gln Arg Val Val Thr
                    500                     505                     510
Gly Phe His His Cys Cys Phe Glu Cys Val Pro Cys Gly Ala Gly
                    515                     520                     525
Thr Phe Leu Asn Lys Ser Asp Leu Tyr Arg Cys Gln Pro Cys Gly
                    530                     535                     540
Lys Glu Glu Trp Ala Pro Glu Gly Ser Gln Thr Cys Phe Pro Arg
                    545                     550                     555
Thr Val Val Phe Leu Ala Leu Arg Glu His Thr Ser Trp Val Leu
                    560                     565                     570
Leu Ala Ala Asn Thr Leu Leu Leu Leu Leu Leu Leu Gly Thr Ala
                    575                     580                     585
Gly Leu Phe Ala Trp His Leu Asp Thr Pro Val Val Arg Ser Ala
                    590                     595                     600
Gly Gly Arg Leu Cys Phe Leu Met Leu Gly Ser Leu Ala Ala Gly
                    605                     610                     615
```

```
Ser Gly Ser Leu Tyr Gly Phe Phe Gly Glu Pro Thr Arg Pro Ala
                620                 625                 630
Cys Leu Leu Arg Gln Ala Leu Phe Ala Leu Gly Phe Thr Ile Phe
                635                 640                 645
Leu Ser Cys Leu Thr Val Arg Ser Phe Gln Leu Ile Ile Ile Phe
                650                 655                 660
Lys Phe Ser Thr Lys Val Pro Thr Phe Tyr His Ala Trp Val Gln
                665                 670                 675
Asn His Gly Ala Gly Leu Phe Val Met Ile Ser Ser Ala Ala Gln
                680                 685                 690
Leu Leu Ile Cys Leu Thr Trp Leu Val Val Trp Thr Pro Leu Pro
                695                 700                 705
Ala Arg Glu Tyr Gln Arg Phe Pro His Leu Val Met Leu Glu Cys
                710                 715                 720
Thr Glu Thr Asn Ser Leu Gly Phe Ile Leu Ala Phe Leu Tyr Asn
                725                 730                 735
Gly Leu Leu Ser Ile Ser Ala Phe Ala Cys Ser Tyr Leu Gly Lys
                740                 745                 750
Asp Leu Pro Glu Asn Tyr Asn Glu Ala Lys Cys Val Thr Phe Ser
                755                 760                 765
Leu Leu Phe Asn Phe Val Ser Trp Ile Ala Phe Phe Thr Thr Ala
                770                 775                 780
Ser Val Tyr Asp Gly Lys Tyr Leu Pro Ala Ala Asn Met Met Ala
                785                 790                 795
Gly Leu Ser Ser Leu Ser Ser Gly Phe Gly Gly Tyr Phe Leu Pro
                800                 805                 810
Lys Cys Tyr Val Ile Leu Cys Arg Pro Asp Leu Asn Ser Thr Glu
                815                 820                 825
His Phe Gln Ala Ser Ile Gln Asp Tyr Thr Arg Arg Cys Gly Ser
                830                 835                 840
Thr


<210> 4
<211> 309
<212> PRT
<213> Homo sapiens

<220>
<221> misc_feature
<223> Incyte ID No: 7474987CD1

<400> 4
Met Ala Asn Leu Thr Ile Val Thr Glu Phe Ile Leu Met Gly Phe
 1               5                  10                  15
Ser Thr Asn Lys Asn Met Cys Ile Leu His Ser Ile Leu Phe Leu
                20                  25                  30
Leu Ile Tyr Leu Cys Ala Leu Met Gly Asn Val Leu Ile Ile Met
                35                  40                  45
Ile Thr Thr Leu Asp His His Leu His Thr Pro Val Tyr Phe Phe
                50                  55                  60
Leu Lys Asn Leu Ser Phe Leu Asp Leu Cys Leu Ile Ser Val Thr
                65                  70                  75
Ala Pro Lys Ser Ile Ala Asn Ser Leu Ile His Asn Asn Ser Ile
                80                  85                  90
Ser Phe Leu Gly Cys Val Ser Gln Val Phe Leu Leu Leu Ser Ser
                95                 100                 105
Ala Ser Ala Glu Leu Leu Leu Leu Thr Val Met Ser Phe Asp Arg
                110                 115                 120
Tyr Thr Ala Ile Cys His Pro Leu His Tyr Asp Val Ile Met Asp
                125                 130                 135
Arg Ser Thr Cys Val Gln Arg Ala Thr Val Ser Trp Leu Tyr Gly
                140                 145                 150
```

Gly Leu Ile Ala Val Met His Thr Ala Gly Thr Phe Ser Leu Ser
155 160 165
Tyr Cys Gly Ser Asn Met Val His Gln Phe Phe Cys Asp Ile Pro
170 175 180
Gln Leu Leu Ala Ile Ser Cys Ser Glu Asn Leu Ile Arg Glu Ile
185 190 195
Ala Leu Ile Leu Ile Asn Val Val Leu Asp Phe Cys Cys Phe Ile
200 205 210
Val Ile Ile Ile Thr Tyr Val His Val Phe Ser Thr Val Lys Lys
215 220 225
Ile Pro Ser Thr Glu Gly Gln Ser Lys Ala Tyr Ser Ile Cys Leu
230 235 240
Pro His Leu Leu Val Val Leu Phe Leu Ser Thr Gly Phe Ile Ala
245 250 255
Tyr Leu Lys Pro Ala Ser Glu Ser Pro Ser Ile Leu Asp Ala Val
260 265 270
Ile Ser Val Phe Tyr Thr Met Leu Pro Pro Thr Phe Asn Pro Ile
275 280 285
Ile Tyr Ser Leu Arg Asn Lys Ala Ile Lys Val Ala Leu Gly Met
290 295 300
Leu Ile Lys Gly Lys Leu Thr Lys Lys
305

<210> 5
<211> 317
<212> PRT
<213> Homo sapiens

<220>
<221> misc_feature
<223> Incyte ID No: 5617631CD1

<400> 5
Met Leu Arg Asn Gly Ser Ile Val Thr Glu Phe Ile Leu Val Gly
1 5 10 15
Phe Gln Gln Ser Ser Thr Ser Thr Arg Ala Leu Leu Phe Ala Leu
20 25 30
Phe Leu Ala Leu Tyr Ser Leu Thr Met Ala Met Asn Gly Leu Ile
35 40 45
Ile Phe Ile Thr Ser Trp Thr Asp Pro Lys Leu Asn Ser Pro Met
50 55 60
Tyr Phe Phe Leu Gly His Leu Ser Leu Leu Asp Val Cys Phe Ile
65 70 75
Thr Thr Thr Ile Pro Gln Met Leu Ile His Leu Val Val Arg Asp
80 85 90
His Ile Val Ser Phe Val Cys Cys Met Thr Gln Met Tyr Phe Val
95 100 105
Phe Cys Val Gly Val Ala Glu Cys Ile Leu Leu Ala Phe Met Ala
110 115 120
Tyr Asp Arg Tyr Val Ala Ile Cys Tyr Pro Leu Asn Tyr Val Pro
125 130 135
Ile Ile Ser Gln Lys Val Cys Val Arg Leu Val Gly Thr Ala Trp
140 145 150
Phe Phe Gly Leu Ile Asn Gly Ile Phe Leu Glu Tyr Ile Ser Phe
155 160 165
Arg Glu Pro Phe Arg Arg Asp Asn His Ile Glu Ser Phe Phe Cys
170 175 180
Glu Ala Pro Ile Val Ile Gly Leu Ser Cys Gly Asp Pro Gln Phe
185 190 195
Ser Leu Trp Ala Ile Phe Ala Asp Ala Ile Val Val Ile Leu Ser
200 205 210
Pro Met Val Leu Thr Val Thr Ser Tyr Val His Ile Leu Ala Thr
215 220 225

```
Ile Leu Ser Lys Ala Ser Ser Ser Gly Arg Gly Lys Thr Phe Ser
                230                 235                 240
Thr Cys Ala Ser His Leu Thr Val Val Ile Phe Leu Tyr Thr Ser
                245                 250                 255
Ala Met Phe Ser Tyr Met Asn Pro His Ser Thr His Gly Pro Asp
                260                 265                 270
Lys Asp Lys Pro Phe Ser Leu Leu Tyr Thr Ile Ile Thr Pro Met
                275                 280                 285
Cys Asn Pro Ile Ile Tyr Ser Phe Arg Asn Lys Glu Ile Lys Glu
                290                 295                 300
Ala Met Val Arg Ala Leu Gly Arg Thr Arg Leu Ala Gln Pro Gln
                305                 310                 315
Ser Val


<210> 6
<211> 317
<212> PRT
<213> Homo sapiens

<220>
<221> misc_feature
<223> Incyte ID No: 7472098CD1

<400> 6
Met Leu Thr Phe His Asn Val Cys Ser Val Pro Ser Ser Phe Trp
 1               5                  10                  15
Leu Thr Gly Ile Pro Gly Leu Glu Ser Leu His Val Trp Leu Ser
                20                  25                  30
Ile Pro Phe Gly Ser Met Tyr Leu Val Ala Val Val Gly Asn Val
                35                  40                  45
Thr Ile Leu Ala Val Val Lys Ile Glu Arg Ser Leu His Gln Pro
                50                  55                  60
Met Tyr Phe Phe Leu Cys Met Leu Ala Ala Ile Asp Leu Val Leu
                65                  70                  75
Ser Thr Ser Thr Ile Pro Lys Leu Leu Gly Ile Phe Trp Phe Gly
                80                  85                  90
Ala Cys Asp Ile Gly Leu Asp Ala Cys Leu Gly Gln Met Phe Leu
                95                  100                 105
Ile His Cys Phe Ala Thr Val Glu Ser Gly Ile Phe Leu Ala Met
                110                 115                 120
Ala Phe Asp Arg Tyr Val Ala Ile Cys Asn Pro Leu Arg His Ser
                125                 130                 135
Met Val Leu Thr Tyr Thr Val Val Gly Arg Leu Gly Leu Val Ser
                140                 145                 150
Leu Leu Arg Gly Val Leu Tyr Ile Gly Pro Leu Pro Leu Met Ile
                155                 160                 165
Arg Leu Arg Leu Pro Leu Tyr Lys Thr His Val Ile Ser His Ser
                170                 175                 180
Tyr Cys Glu His Met Ala Val Val Ala Leu Thr Cys Gly Asp Ser
                185                 190                 195
Arg Val Asn Asn Val Tyr Gly Leu Ser Ile Gly Phe Leu Val Leu
                200                 205                 210
Ile Leu Asp Ser Val Ala Ile Ala Ala Ser Tyr Val Met Ile Phe
                215                 220                 225
Arg Ala Val Met Gly Leu Ala Thr Pro Glu Ala Arg Leu Lys Thr
                230                 235                 240
Leu Gly Thr Cys Ala Ser His Leu Cys Ala Ile Leu Ile Phe Tyr
                245                 250                 255
Val Pro Ile Ala Val Ser Ser Leu Ile His Arg Phe Gly Gln Cys
                260                 265                 270
Val Pro Pro Pro Val His Thr Leu Leu Ala Asn Phe Tyr Leu Leu
                275                 280                 285
```

```
Ile Pro Pro Ile Leu Asn Pro Ile Val Tyr Ala Val Arg Thr Lys
                290                 295                 300
Gln Ile Arg Glu Ser Leu Leu Gln Ile Pro Arg Ile Glu Met Lys
                305                 310                 315
Ile Arg


<210> 7
<211> 324
<212> PRT
<213> Homo sapiens

<220>
<221> misc_feature
<223> Incyte ID No: 7476775CD1

<400> 7
Met Ser Phe Phe Phe Val Asp Leu Arg Pro Met Asn Arg Ser Ala
  1               5                  10                  15
Thr His Ile Val Thr Glu Phe Ile Leu Leu Gly Phe Pro Gly Cys
                 20                  25                  30
Trp Lys Ile Gln Ile Phe Leu Phe Ser Leu Phe Leu Val Ile Tyr
                 35                  40                  45
Val Leu Thr Leu Leu Gly Asn Gly Ala Ile Ile Tyr Ala Val Arg
                 50                  55                  60
Cys Asn Pro Leu Leu His Thr Pro Met Tyr Phe Leu Leu Gly Asn
                 65                  70                  75
Phe Ala Phe Leu Glu Ile Trp Tyr Val Ser Ser Thr Ile Pro Asn
                 80                  85                  90
Met Leu Val Asn Ile Leu Ser Lys Thr Lys Ala Ile Ser Phe Ser
                 95                 100                 105
Gly Cys Phe Leu Gln Phe Tyr Phe Phe Phe Ser Leu Gly Thr Thr
                110                 115                 120
Glu Cys Leu Phe Leu Ala Val Met Ala Tyr Asp Arg Tyr Leu Ala
                125                 130                 135
Ile Cys His Pro Leu Gln Tyr Pro Ala Ile Met Thr Val Arg Phe
                140                 145                 150
Cys Gly Lys Leu Val Ser Phe Cys Trp Leu Ile Gly Phe Leu Gly
                155                 160                 165
Tyr Pro Ile Pro Ile Phe Tyr Ile Ser Gln Leu Pro Phe Cys Gly
                170                 175                 180
Pro Asn Ile Ile Asp His Phe Leu Cys Asp Met Asp Pro Leu Met
                185                 190                 195
Ala Leu Ser Cys Ala Pro Ala Pro Ile Thr Glu Cys Ile Phe Tyr
                200                 205                 210
Thr Gln Ser Ser Leu Val Leu Phe Phe Thr Ser Met Tyr Ile Leu
                215                 220                 225
Arg Ser Tyr Ile Leu Leu Leu Thr Ala Val Phe Gln Val Pro Ser
                230                 235                 240
Ala Ala Gly Arg Arg Lys Ala Phe Ser Thr Cys Gly Ser His Leu
                245                 250                 255
Val Val Val Ser Leu Phe Tyr Gly Thr Val Met Val Met Tyr Val
                260                 265                 270
Ser Pro Thr Tyr Gly Ile Pro Thr Leu Leu Gln Lys Ile Leu Thr
                275                 280                 285
Leu Val Tyr Ser Val Thr Thr Pro Leu Phe Asn Pro Leu Ile Tyr
                290                 295                 300
Thr Leu Arg Asn Lys Asp Met Lys Leu Ala Leu Arg Asn Val Leu
                305                 310                 315
Phe Gly Met Arg Ile Arg Gln Asn Ser
                320

<210> 8
```

<211> 322
<212> PRT
<213> Homo sapiens

<220>
<221> misc_feature
<223> Incyte ID No: 7477937CD1

<400> 8

```
Met Glu Pro Gln Asn Thr Ser Thr Val Thr Asn Phe Gln Leu Leu
 1               5                  10                  15
Gly Phe Gln Asn Leu Leu Glu Trp Gln Ala Leu Leu Phe Val Ile
                20                  25                  30
Phe Leu Leu Ile Tyr Cys Leu Thr Ile Ile Gly Asn Val Val Ile
                35                  40                  45
Ile Thr Val Val Ser Gln Gly Leu Arg Leu His Ser Pro Met Tyr
                50                  55                  60
Met Phe Leu Gln His Leu Ser Phe Leu Glu Val Trp Tyr Thr Ser
                65                  70                  75
Thr Thr Val Pro Leu Leu Leu Ala Asn Leu Leu Ser Trp Gly Gln
                80                  85                  90
Ala Ile Ser Phe Ser Ala Cys Met Ala Gln Leu Tyr Phe Phe Val
                95                 100                 105
Phe Leu Gly Ala Thr Glu Cys Phe Leu Leu Ala Phe Met Ala Tyr
               110                 115                 120
Asp Arg Tyr Leu Ala Ile Cys Ser Pro Leu Arg Tyr Pro Phe Leu
               125                 130                 135
Met His Arg Gly Leu Cys Ala Arg Leu Val Val Val Ser Trp Cys
               140                 145                 150
Thr Gly Val Ser Thr Gly Phe Leu His Ser Met Met Ile Ser Arg
               155                 160                 165
Leu Asp Phe Cys Gly Arg Asn Gln Ile Asn His Phe Phe Cys Asp
               170                 175                 180
Leu Pro Pro Leu Met Gln Leu Ser Cys Ser Arg Val Tyr Ile Thr
               185                 190                 195
Glu Val Thr Ile Phe Ile Leu Ser Ile Ala Val Leu Cys Ile Cys
               200                 205                 210
Phe Phe Leu Thr Leu Gly Pro Tyr Val Phe Ile Val Ser Ser Ile
               215                 220                 225
Leu Arg Ile Pro Ser Thr Ser Gly Arg Arg Lys Thr Phe Ser Thr
               230                 235                 240
Cys Gly Ser His Leu Ala Val Val Thr Leu Tyr Tyr Gly Thr Met
               245                 250                 255
Ile Ser Met Tyr Val Cys Pro Ser Pro His Leu Leu Pro Glu Ile
               260                 265                 270
Asn Lys Ile Ile Ser Val Phe Tyr Thr Val Val Thr Pro Leu Leu
               275                 280                 285
Asn Pro Val Ile Tyr Ser Leu Arg Asn Lys Asp Phe Lys Glu Ala
               290                 295                 300
Val Arg Lys Val Met Arg Arg Lys Cys Gly Ile Leu Trp Ser Thr
               305                 310                 315
Ser Lys Arg Lys Phe Leu Tyr
               320
```

<210> 9
<211> 312
<212> PRT
<213> Homo sapiens

<220>
<221> misc_feature
<223> Incyte ID No: 7476798CD1

<400> 9
Met Glu Asn Tyr Asn Gln Thr Ser Thr Asp Phe Ile Leu Leu Gly
1 5 10 15
Leu Val Pro Pro Ser Arg Ile Asp Leu Phe Leu Phe Ile Leu Ile
20 25 30
Val Phe Ile Phe Leu Met Ala Leu Ile Gly Asn Leu Ser Met Ile
35 40 45
Leu Leu Ile Phe Leu Asp Thr His Leu His Thr Pro Met Tyr Phe
50 55 60
Leu Leu Ser Gln Leu Ser Leu Ile Asp Leu Asn Tyr Ile Ser Thr
65 70 75
Ile Val Pro Lys Met Ala Ser Asp Phe Leu Ser Gly Asn Lys Ser
80 85 90
Ile Ser Phe Thr Gly Cys Gly Ile Gln Ser Phe Phe Phe Ser Ala
95 100 105
Leu Gly Gly Ala Glu Ala Leu Leu Leu Ala Ser Met Ala Tyr Asp
110 115 120
Arg Tyr Ile Ala Ile Cys Phe Pro Leu His Tyr Pro Ile Arg Met
125 130 135
Ser Lys Arg Met Cys Val Leu Met Ile Thr Gly Ser Trp Ile Ile
140 145 150
Gly Ser Ile Asn Ala Cys Ala His Thr Val Tyr Val Leu His Ile
155 160 165
Pro Tyr Cys Gln Ser Arg Ala Ile Asn His Phe Phe Cys Asp Val
170 175 180
Pro Ala Met Val Thr Leu Ala Cys Met Asp Thr Trp Val Tyr Glu
185 190 195
Gly Thr Val Phe Leu Ser Thr Thr Ile Phe Leu Val Phe Pro Phe
200 205 210
Ile Ala Ile Ser Cys Ser Tyr Gly Arg Val Leu Leu Ala Val Tyr
215 220 225
His Met Lys Ser Ala Glu Gly Arg Lys Lys Ala Tyr Leu Thr Cys
230 235 240
Ser Thr His Leu Thr Val Val Thr Phe Tyr Tyr Ala Pro Phe Val
245 250 255
Tyr Thr Tyr Leu Arg Pro Arg Ser Leu Arg Ser Pro Thr Glu Asp
260 265 270
Lys Val Leu Ala Val Phe Tyr Thr Ile Leu Thr Pro Met Leu Asn
275 280 285
Pro Ile Ile Tyr Ser Leu Arg Asn Lys Glu Val Met Gly Ala Leu
290 295 300
Thr Arg Val Ser Gln Arg Ile Cys Ser Val Lys Met
305 310

<210> 10
<211> 319
<212> PRT
<213> Homo sapiens

<220>
<221> misc_feature
<223> Incyte ID No: 7477889CD1

<400> 10
Met Thr Gln Leu Thr Ala Ser Gly Asn Gln Thr Met Val Thr Glu
1 5 10 15
Phe Leu Phe Ser Met Phe Pro His Ala His Arg Gly Gly Leu Leu
20 25 30
Phe Phe Ile Pro Leu Leu Leu Ile Tyr Gly Phe Ile Leu Thr Gly
35 40 45
Asn Leu Ile Met Phe Ile Val Ile Gln Val Gly Met Ala Leu His
50 55 60
Thr Pro Leu Tyr Phe Phe Ile Ser Val Leu Ser Phe Leu Glu Ile

```
                    65                  70                  75
Cys Tyr Thr Thr Thr Thr Ile Pro Lys Met Leu Ser Cys Leu Ile
                    80                  85                  90
Ser Glu Gln Lys Ser Ile Ser Val Ala Gly Cys Leu Leu Gln Met
                    95                  100                 105
Tyr Phe Phe His Ser Leu Gly Ile Thr Glu Ser Cys Val Leu Thr
                    110                 115                 120
Ala Met Ala Ile Asp Arg Tyr Ile Ala Ile Cys Asn Pro Leu Arg
                    125                 130                 135
Tyr Pro Thr Ile Met Ile Pro Lys Leu Cys Ile Gln Leu Thr Val
                    140                 145                 150
Gly Ser Cys Phe Cys Gly Phe Leu Leu Val Leu Pro Glu Ile Ala
                    155                 160                 165
Trp Ile Ser Thr Leu Pro Phe Cys Gly Ser Asn Gln Ile His Gln
                    170                 175                 180
Ile Phe Cys Asp Phe Thr Pro Val Leu Ser Leu Ala Cys Thr Asp
                    185                 190                 195
Thr Phe Leu Val Val Ile Val Asp Ala Ile His Ala Ala Glu Ile
                    200                 205                 210
Val Ala Ser Phe Leu Val Ile Ala Leu Ser Tyr Ile Arg Ile Ile
                    215                 220                 225
Ile Val Ile Leu Gly Met His Ser Ala Glu Gly His His Lys Ala
                    230                 235                 240
Phe Ser Thr Cys Ala Ala His Leu Ala Val Phe Leu Leu Phe Phe
                    245                 250                 255
Gly Ser Val Ala Val Met Tyr Leu Arg Phe Ser Ala Thr Tyr Ser
                    260                 265                 270
Val Phe Trp Asp Thr Ala Ile Ala Val Thr Phe Val Ile Leu Ala
                    275                 280                 285
Pro Phe Phe Asn Pro Ile Ile Tyr Ser Leu Lys Asn Lys Asp Met
                    290                 295                 300
Lys Glu Ala Ile Gly Arg Leu Phe His Tyr Gln Lys Arg Ala Gly
                    305                 310                 315
Trp Ala Gly Lys
```

```
<210> 11
<211> 2245
<212> DNA
<213> Homo sapiens

<220>
<221> misc_feature
<223> Incyte ID No: 7474927CB1

<400> 11
atggtgtatt atttttgctt ttcatatctc caggctactt aattgaaggt tttattgaaa 60
taagtgtaga ctcacaagca gtcataagaa ataatacaga aaaagccctg tccatattac 120
ccagggttcc gtcatgatta cattttgcaa actatagtat aatatcactc taatgatatt 180
gactcttctg tgttctgttt ttatatattt ccctagtttt gcttgtattt acttgttgca 240
tgtgtgtaag ccctgagttt tataggtttg atttgtggat ctgttgtgcg gttgagccct 300
cattcattca cctgcttctc tgcagttgga cacacaagca attgcctttg cacgaacagg 360
gacaatctta atttctgttt aagatgagaa aatatggttg gagacacatt aaaacttctg 420
tctccactga tgacaagata cttctttctg ctttttttatt ctactgattc ttcagacctc 480
aatgaaaatc aacatcccct agattttgat gaaatggctt ttggaaaagt aaaatcaggg 540
attagcttcc tcattcagac tggagttggg atcctgggaa attcctttct cctttgtttt 600
tataacttaa ttttgttcac tggacacaag ctgagaccca cggacttgat tctcagccaa 660
ctggccttgg ctaactccat ggtccttttc tttaaaggga tacctcagac aatggcagct 720
tttggattga aatatttgct gaatgacact ggatgtaagt ttgtctttta ttatcacagg 780
gtgggcacaa gagtttccct cagcaccatc tgccttctca atggattcca agccattaag 840
ctcaacccca gtatatgcag gtggatggag atcaagatta gatccccaag gtttattgac 900
ttctgttgtc tcctctgctg ggcccccat gtcttgatga atgcatctgt tcttctatta 960
gtgaatggcc cactgaatag caaaaacagt agtgcaaaaa acaattatgg atactgttct 1020
```

tacaaagcat caaagagatt tagctcatta catgcagtct tatatttttc ccctgatttt 1080
atgagtttgg gcttcatggt ctgggccagt ggctccatgg tcttcttcct ctacagacac 1140
aagcagcaag tccaacacaa tcacagcaac agactctcct gcagaccttc ccaggaagcc 1200
agagccacac acaccatcat ggtcctggtg agctcctttt ttgttttcta ttcagtccat 1260
agttttctga caatttggac aactgtagtt gcaaacccag gccagtggat agtgaccaac 1320
tctgtgttgg tcgcctcatg tttcccagca cgcagccctt ttgtcctcat catgagtgat 1380
actcatatct ctcagttctg ttttgcctgc aggacaagga aaacactctt tcctaatctg 1440
gttgtcatgc catgagtctt ttctcttcat ggaattcagc tatttatcat aactctgcta 1500
agatttagga aatattaact actagttatt tgtgatagca acatacacat ggccagtaat 1560
gctcttgtcc aggaagatct aataccagag ctaaaaatga aagtcatgga tactgttaca 1620
caaagaacac tctatataac tgtgttaagt ccttcagaca agttcaggaa atcaaaaagt 1680
ttaaaaaggg aattctttag agatttaggg gagatttctc atttttgtac tatgaagaat 1740
catggaatgt tttaaaaata tttttaaagc acagtttgat tcaggtgctt cttgaacagc 1800
ataaatcccc tggagagtcc acatgtaaga aagacatgtg caggccgggc acagtggctc 1860
acgcctgtaa tcccaccgtt ttgggaggct gaggcactcc caaatgcctc aagtgatcca 1920
ctcaagatga cttgaggcca ggagttcgag accagcctgg ccaacatggc aaaaaccctg 1980
tctcaaatac aaaaattagc caaccatgtg gcacacacct gtagtcccag ctactccaga 2040
gggtgaagca cgagaattac ttaccagctt gggtgacgga gggagactca aaataaaaat 2100
aaaaataact aaaagtggct gggcaccgtg ggtcacgccg gtaaccccag cactttgaga 2160
ggctgatgtg ggcagatcac ttgaagtcag gagttcaaga ccagcctggc caacatggtg 2220
aaaccccatc tctattaaaa aaaaa 2245

<210> 12
<211> 2729
<212> DNA
<213> Homo sapiens

<220>
<221> misc_feature
<223> Incyte ID No: 7475194CB1

<400> 12
atgctgggcc ctgctgtcct gggcctcagc ctctgggctc tcctgcaccc tgggacgggg 60
gccccattgt gcctgtcaca gcaacttagg atgaaggggg actacgtgct gggggggctg 120
ttccccctgg gcgaggccga ggaggctggc ctccgcagcc ggacacggcc cagcagccct 180
gtgtgcacca ggttctcctc aaacggcctg ctctgggcac tggccatgaa aatggccgtg 240
gaggagatca acaacaagtc ggatctgctg cccgggctgc gcctgggcta cgacctcttt 300
gatacgtgct cggagcctgt ggtggccatg aagcccagcc tcatgttcct ggccaaggca 360
ggcagccgcg acatcgccgc ctactgcaac tacacgcagt accagccccg tgtgctggct 420
gtcatcgggc cccactcgtc agagctcgcc atggtcaccg gcaagttctt cagcttcttc 480
ctcatgcccc aggtcagcta cggtgctagc atggagctgc tgagcgcccg ggagaccttc 540
ccctccttct tccgcaccgt gcccagcgac cgtgtgcagc tgacggccgc cgcggagctg 600
ctgcaggagt tcggctggaa ctgggtggcc gccctgggca gcgacgacga gtacggccgg 660
cagggcctga gcatcttctc ggccctggcc gcggcacgcg gcatctgcat cgcgcacgag 720
ggcctggtgc cgctgccccg tgccgatgac tcgcggctgg ggaaggtgca ggacgtcctg 780
caccaggtga accagagcag cgtgcaggtg gtgctgctgt tcgcctccgt gcacgccgcc 840
cacgccctct tcaactacag catcagcagc aggctctcgc ccaaggtgtg ggtggccagc 900
gaggcctggc tgacctctga cctggtcatg gggctgcccg gcatggccca gatgggcacg 960
gtgcttggct tcctccagag ggtgcccag ctgcacgagt tcccccagta cgtgaagacg 1020
cacctggccc tggccaccga cccggccttc tgctctgccc tgggcgagag ggagcagggt 1080
ctggaggagg acgtggtggg ccagcgctgc ccgcagtgtg actgcatcac gctgcagaac 1140
gtgagcgcag ggctaaatca ccaccagacg ttctctgtct acgcagctgt gtatagcgtg 1200
gcccaggccc tgcacaacac tcttcagtgc aacgcctcag gctgccccgc gcaggacccc 1260
gtgaagccct ggcagctcct ggagaacatg tacaacctga ccttccacgt gggcgggctg 1320
ccgctgcggt tcgacagcag cggaaacgtg gacatggagt acgacctgaa gctgtgggtg 1380
tggcagggct cagtgcccag gctccacgac gtgggcaggt tcaacggcag cctcaggaca 1440
gagcgcctga agatccgctg gcacacgtct gacaaccagc cgagcagagc cagaccccag 1500
gcctgtgcgc agaagcccgt gtcccggtgc tcgcggcagt gccaggaggg ccaggtgcgc 1560
cgggtcaagg ggttccactc ctgctgctac gactgtgtgg actgcgaggc gggcagctac 1620
cggcaaaacc cagacgacat cgcctgcacc ttttgtggcc aggatgagtg gtccccggag 1680
cgaagcacac gctgcttccg ccgcaggtct cggttcctgg catggggcga gccggctgtg 1740
ctgctgctgc tcctgctgct gagcctggcg ctgggccttg tgctggctgc tttggggctg 1800
ttcgttcacc atcgggacag cccactggtt caggcctcgg gggggcccct ggcctgcttt 1860

```
ggcctggtgt gcctgggcct ggtctgcctc agcgtcctcc tgttccctgg ccagcccagc 1920
cctgccccgat gcctggccca gcagcccttg tcccacctcc cgctcacggg ctgcctgagc 1980
acactcttcc tgcaggcggc cgagatcttc gtggagtcag aactgcctct gagctgggca 2040
gaccggctga gtggctgcct gcgggggccc tgggcctggc tggtggtgct gctggccatg 2100
ctggtggagg tcgcactgtg cacctggtac ctggtggcct tcccgccgga ggtggtgacg 2160
gactggcaca tgctgcccac ggaggcgctg gtgcactgcc gcacacgctc ctgggtcagc 2220
ttcggcctag cgcacgccac caatgccacg ctggcctttc tctgcttcct gggcactttc 2280
ctggtgcgga gccagccggg ccgctacaac cgtgcccgtg gcctcacctt tgccatgctg 2340
gcctacttca tcacctgggt ctcctttgtg cccctcctgg ccaatgtgca ggtggtcctc 2400
aggcccgccg tgcagatggg cgccctcctg ctctgtgtcc tgggcatcct ggctgccttc 2460
cacctgccca ggtgttacct gctcatgcgg cagccagggc tcaacacccc cgagttcttc 2520
ctgggagggg gccctgggga tgcccaaggc cagaatgacg ggaacacagg aaatcagggg 2580
aaacatgagt gacccaacca ctgtgatctc agccccggtg aacccagact tagctgcgat 2640
cccccccaag ccagcaatga cccgtgtctc gctacagaga ccctcccgct ctaggttctg 2700
accccaggtt gtctcctgac ctgacccccc 2729
```

<210> 13
<211> 2759
<212> DNA
<213> Homo sapiens

<220>
<221> misc_feature
<223> Incyte ID No: 7475203CB1

<400> 13

```
cacgcgtacg taagctcgga agttcggaat cgagcgcggg catctggcca gcatgctgct 60
ctgcacggct cgcctggtcg gcctgcagct tctcatttcc tgctgctggg cctttgcctg 120
ccatagcacg gagtcttctc ctgacttcac cctccccgga gattacctcc tggcaggcct 180
gttccctctc cattctggct gtctgcaggt gaggcacaga cccgaggtga ccctgtgtga 240
caggtcttgt agcttcaatg agcatggcta ccacctcttc caggctatgc ggcttggggt 300
tgaggagata aacaactcca cggccctgct gcccaacatc accctggggt accagctgta 360
tgatgtgtgt tctgactctg ccaatgtgta tgccacgctg agagtgctct ccctgccagg 420
gcaacaccac atagagctcc aaggagacct tctccactat tcccctacgg tgctggcagt 480
gattgggcct gacagcacca accgtgctgc caccacagcc gccctgctga gccctttcct 540
ggtgcccatg attagctatg cggccagcag cgagacgctc agcgtgaagc ggcagtatcc 600
ctctttcctg cgcaccatcc ccaatgacaa gtaccaggtg gagaccatgg tgctgctgct 660
gcagaagttc gggtggacct ggatctctct ggttggcagc agtgacgact atgggcagct 720
aggggtgcag gcactggaga accaggccac tggtcagggg atctgcattg ctttcaagga 780
catcatgccc ttctctgccc aggtgggcga tgagaggatg cagtgcctca tgcgccacct 840
ggcccaggcc ggggccaccg tcgtggttgt tttttccagc cggcagttgg ccagggtgtt 900
tttcgagtcc gtggtgctga ccaacctgac tggcaaggtg tgggtcgcct cagaagcctg 960
ggccctctcc aggcacatca ctggggtgcc cgggatccag cgcattggga tggtgctggg 1020
cgtggccatc cagaagaggg ctgtccctgg cctgaaggcg tttgaagaag cctatgcccg 1080
ggcagacaag aaggccccta ggccttgcca caagggctcc tggtgcagca gcaatcagct 1140
ctgcagagaa tgccaagctt tcatggcaca cacgatgccc aagctcaaag ccttctccat 1200
gagttctgcc tacaacgcat accgggctgt gtatgcggtg gcccatggcc tccaccagct 1260
cctgggctgt gcctctggag cttgttccag gggccgagtc taccccctggc agcttttgga 1320
gcagatccac aaggtgcatt tccttctaca caaggacact gtggcgttta atgacaacag 1380
agatcccctc agtagctata acataattgc ctgggactgg aatggaccca agtggacctt 1440
cacggtcctc ggttcctcca catggtctcc agttcagcta aacataaatg agaccaaaat 1500
ccagtggcac ggaaaggaca accaggtgcc taagtctgtg tgttccagcg actgtcttga 1560
agggcaccag cgagtggtta cgggtttcca tcactgctgc tttgagtgtg tgccctgtgg 1620
ggctgggacc ttcctcaaca agagtgacct ctacagatgc cagccttgtg ggaaagaaga 1680
gtgggcacct gagggaagcc agacctgctt cccgcgcact gtggtgtttt tggctttgcg 1740
tgagcacacc tcttgggtgc tgctggcagc taacacgctg ctgctgctgc tgctgcttgg 1800
gactgctggc ctgtttgcct ggcacctaga cacccctgtg gtgaggtcag caggggggccg 1860
cctgtgcttt cttatgctgg gctccctggc agcaggtagt ggcagcctct atggcttctt 1920
tggggaaccc acaaggcctg cgtgcttgct acgccaggcc ctctttgccc ttggtttcac 1980
catcttcctg tcctgcctga cagttcgctc attccaacta atcatcatct tcaagttttc 2040
caccaaggta cctacattct accacgcctg ggtccaaaac cacggtgctg gcctgtttgt 2100
gatgatcagc tcagcggccc agctgcttat ctgtctaact tggctggtgg tgtggacccc 2160
actgcctgct agggaatacc agcgcttccc ccatctggtg atgcttgagt gcacagagac 2220
```

caactccctg ggcttcatac tggccttcct ctacaatggc ctcctctcca tcagtgcctt 2280
tgcctgcagc tacctgggta aggacttgcc agagaactac aacgaggcca aatgtgtcac 2340
cttcagcctg ctcttcaact tcgtgtcctg gatcgccttc ttcaccacgg ccagcgtcta 2400
cgacggcaag tacctgcctg cggccaacat gatggctggg ctgagcagcc tgagcagcgg 2460
cttcggtggg tattttctgc ctaagtgcta cgtgatcctc tgccgcccag acctcaacag 2520
cacagagcac ttccaggcct ccattcagga ctacacgagg cgctgcggct ccacctgacc 2580
agtgggtcag caggcacggc tggcagcctt ctctgccctg agggtcgaag gtcgagcagg 2640
ccgggggtgt ccgggaggtc tttgggcatc gcggtctggg gttgggacgt gtaagcgcct 2700
gggagagcct agaccaggct ccgggctgcc aataaagaaa aaaaatgcgt aaaaaaaaa 2759

<210> 14
<211> 945
<212> DNA
<213> Homo sapiens

<220>
<221> misc_feature
<223> Incyte ID No: 7474987CB1

<400> 14
attactcctg caataatggc aaatctcaca atcgtgactg aatttatcct tatggggttt 60
tctaccaata aaaatatgtg cattttgcat tcgattctct tcttgttgat ttatttgtgt 120
gccctgatgg ggaatgtcct cattatcatg atcacaactt tggaccatca tctccacacc 180
cccgtgtatt tcttcttgaa gaatctatct ttcttggatc tctgccttat ttcagtcacg 240
gctcccaaat ctatcgccaa ttctttgata cacaacaact ccatttcatt ccttggctgt 300
gtttcccagg tctttttgtt gctttcttca gcatctgcag agctgctcct cctcacggtg 360
atgtcctttg accgctatac tgctatatgt caccctctgc actatgatgt catcatggac 420
aggagcacct gtgtccaaag agccactgtg tcttggctgt atgggggtct gattgctgtg 480
atgcacacag ctggcacctt ctccttatcc tactgtgggt ccaacatggt ccatcagttc 540
ttctgtgaca ttccccagtt attagctatt tcttgctcag aaaatttaat aagagaaatt 600
gcactcatcc ttattaatgt agttttggat ttctgctgtt ttattgtcat catcattacc 660
tatgtccacg tcttctctac agtcaagaag atcccttcca cagaaggcca gtcaaaagcc 720
tactctattt gccttccaca cttgctggtt gtgttatttc tttccactgg attcattgct 780
tatctgaagc cagcttcaga gtctccttct attttggatg ctgtaatttc tgtgttctac 840
actatgctgc cccaaccttt taatcccatt atatacagtt tgagaaacaa ggccataaag 900
gtggctctgg ggatgttgat aaagggaaag ctcaccaaaa agtaa 945

<210> 15
<211> 1511
<212> DNA
<213> Homo sapiens

<220>
<221> misc_feature
<223> Incyte ID No: 5617631CB1

<400> 15
gaacccattc atcattttaa attaggtaca cagaggttga gcatttttgc ttctagaaaa 60
atacaccttc aaatatctca agtgcttcct aaatacaatc ttctataacc tgatagccac 120
cagccctttc cctcacacac tccattcttc aattcctaaa actttcctc agttggcatg 180
gtcccagacc caattatctt atttttcac ctctgaatac atcctggtta atggctaatc 240
cttcaatatt gagggctctg gagcagatgc aatacttcac ttgatgcctg atgaaaccag 300
agtaaagaac attacaacca gcactttctt catctgttga tattatgatt ctatgtatgc 360
agcctgtgat gaccctggca ttttcaccac cccgtaacac tgccaaatta ctagtttact 420
aagttccaaa aggttgacca ccattcatcc atcctgtttt ataattgggc ttctgggacc 480
aagtgggtac cttctattac cccacagcta tacccttgtg cttttcccat catctttcct 540
cctcaaacag gccccagatg ctaaggaatg gcagcatagt gacggaattt atcctcgtgg 600
gctttcagca gagctccact tccacacgag cattgctctt tgccctcttc ttggccctct 660
acagcctcac catggccatg aatggcctca tcatctttat cacctcctgg acagacccca 720
agctcaacag ccccatgtac ttcttcctcg gccatctgtc tctcctggat gtctgcttca 780
tcaccactac catcccacag atgttgatcc acctcgtggt cagggaccac attgtctcct 840
ttgtatgttg catgacccag atgtactttg tcttctgtgt tggtgtggcc gagtgcatcc 900
tcttggcttt catggcctat gaccgttatg ttgctatctg ctacccactt aactatgtcc 960

cgatcataag ccagaaggtc tgtgtcaggc ttgtgggaac tgcctggttc tttgggctga 1020
tcaatggcat ctttctcgag tatatttcat tccgagagcc cttccgcaga gacaaccaca 1080
tagaaagctt cttctgtgag gcccccatag tgattggcct ctcttgtggg gaccctcagt 1140
ttagtctgtg ggcaatcttt gccgatgcca tcgtggtaat tctcagcccc atggtgctca 1200
ctgtcacttc ctatgtgcac atcctggcca ccatcctcag caaagcctcc tcctcaggtc 1260
gggggaagac tttctctact tgtgcctctc acctgactgt ggtcatcttt ctctacactt 1320
cagctatgtt ctcttacatg aaccccaca gcacacatgg gcctgacaaa gacaaacctt 1380
tctccctcct gtacaccatc attaccccca tgtgcaaccc catcatttat agtttccgca 1440
acaaggaaat taaggaggcc atggtgaggg cacttggaag aaccaggctg gcccagccac 1500
agtctgtcta g 1511

<210> 16
<211> 954
<212> DNA
<213> Homo sapiens

<220>
<221> misc_feature
<223> Incyte ID No: 7472098CB1

<400> 16
atgctcactt ttcataatgt ctgctcagta cccagctcct tctggctcac tggcatccca 60
gggctggagt ccctacacgt ctggctctcc atcccctttg gctccatgta cctggtggct 120
gtggtgggga atgtgaccat cctggctgtg gtaaagatag aacgcagcct gcaccagccc 180
atgtactttt tcttgtgcat gttggctgcc attgacctgg ttctgtctac ttccactata 240
cccaaacttc tgggaatctt ctggttcggt gcttgtgaca ttggcctgga cgcctgcttg 300
ggccaaatgt tccttatcca ctgctttgcc actgttgagt caggcatctt ccttgccatg 360
gcttttgatc gctacgtggc catctgcaac ccactacgtc atagcatggt gctcacttat 420
acagtggtgg gtcgtttggg gcttgtttct ctcctccggg gtgttctcta cattggacct 480
ctgcctctga tgatccgcct gcggctgccc ctttataaaa cccatgttat ctcccactcc 540
tactgtgagc acatggctgt agttgccttg acatgtggcg acagcagggt caataatgtc 600
tatgggctga gcatcggctt tctggtgttg atcctggact cagtggctat tgctgcatcc 660
tatgtgatga ttttcagggc cgtgatgggg ttagccactc ctgaggctag gcttaaaacc 720
ctggggacat gcgcttctca cctctgtgcc atcctgatct tttatgttcc cattgctgtt 780
tcttccctga ttcaccgatt tggtcagtgt gtgcctcctc cagtccacac tctgctggcc 840
aacttctatc tcctcattcc tccaatcctc aatcccattg tctatgctgt tcgcaccaag 900
cagatccgag agagccttct ccaaatacca aggatagaaa tgaagattag atga 954

<210> 17
<211> 975
<212> DNA
<213> Homo sapiens

<220>
<221> misc_feature
<223> Incyte ID No: 7476775CB1

<400> 17
atgtctttct tctttgtaga cttaagaccc atgaacaggt cagcaacaca catcgtgaca 60
gagtttattc tcctgggatt ccctggttgc tggaagattc agattttcct cttctcattg 120
tttttggtga tttatgtctt gaccttgctg ggaaatggag ccatcatcta tgcagtgaga 180
tgcaacccac tactacacac ccccatgtac tttctgctgg gaaattttgc cttccttgag 240
atctggtatg tgtcctccac tattcctaac atgctagtca acattctctc caagaccaag 300
gccatctcat tttctgggtg cttcctccag ttctatttct tcttttcact gggaacaact 360
gaatgtctct ttctggcagt aatggcttat gatcgatacc tggccatctg ccacccactg 420
cagtaccctg ccatcatgac tgtaaggttc tgtggtaagc tggtgtcttt ctgttggctt 480
attggattcc ttggataccc aattcccatt ttctacatct cccaactccc cttctgtggt 540
cctaatatca ttgatcactt cctgtgtgac atggacccat tgatggctct atcctgtgcc 600
ccagctccca taactgaatg tattttctat actcagagct cccttgtcct ctttttcact 660
agtatgtaca ttcttcgatc ctatatcctg ttactaacag ctgtttttca ggtcccttct 720
gcagctggtc ggagaaaagc cttctctacc tgtggttctc atttggttgt ggtatctctt 780
ttctatggga cagtcatggt aatgtatgta agtcctacat atgggatccc aactttattg 840
cagaagatcc tcacactggt atattcagta acgactcctc tttttaatcc tctgatctat 900

actcttcgta ataaggacat gaaactcgct ctgagaaatg tcctgtttgg aatgagaatt 960
cgtcaaaatt cgtga 975

<210> 18
<211> 969
<212> DNA
<213> Homo sapiens

<220>
<221> misc_feature
<223> Incyte ID No: 7477937CB1

<400> 18
atggagcccc aaaatacctc cactgtgact aactttcagc tgttaggatt ccagaacctt 60
cttgaatggc aggccctgct ctttgtcatt ttcctgctca tctactgcct gaccattata 120
gggaatgttg tcatcatcac cgtggtgagc cagggcctgc gactgcactc ccctatgtac 180
atgttcctcc agcatctctc ctttctggag gtctggtaca cgtccaccac tgtgcccctt 240
ctcctagcca acctgctgtc ctggggccaa gccatctcct tctctgcctg catggcacag 300
ctctacttct tcgtattcct cggcgccacc gagtgctttc tcctggcctt catggcctat 360
gaccgttacc tggccatctg cagcccactc cgctacccct ttctcatgca tcgtgggcta 420
tgtgccaggt tggtggtggt ctcatggtgc acaggggtca gcacaggctt tctgcattcc 480
atgatgattt ccaggttgga cttctgtggg cgcaatcaga ttaaccattt cttctgcgac 540
ctcccgccac tcatgcagct ctcctgttcc agagtttata tcaccgaggt gaccatcttc 600
atcctgtcaa ttgccgtgct gtgcatttgt ttttttctga cactggggcc ctatgttttc 660
attgtgtcct ccatattgag aatcccttcc acctctggcc ggagaaagac cttttccaca 720
tgtggctccc acctggctgt tgtcactctc tactacggga ccatgatctc catgtatgtg 780
tgtcccagtc cccacctgtt gcctgaaatc aacaagatca tttctgtctt ctacactgtg 840
gtcacaccac tgctgaaccc agttatctac agcttgagga acaaagactt caaagaagct 900
gttagaaagg tcatgagaag gaaatgtggt attctatgga gtacaagtaa aaggaagttc 960
ctttattag 969

<210> 19
<211> 939
<212> DNA
<213> Homo sapiens

<220>
<221> misc_feature
<223> Incyte ID No: 7476798CB1

<400> 19
atggaaaatt acaatcaaac atcaactgat ttcatcttat tggggctggt tccaccatca 60
agaattgacc ttttcctctt catcctcatt gttttcattt tcctaatggc tctaattgga 120
aacctatcca tgattcttct catcttcttg gacacccatc tccacacacc catgtatttc 180
ctacttagtc agctctccct cattgaccta aattacatct ccaccattgt tcctaagatg 240
gcatctgatt ttctgtctgg taacaagtct atctccttca ctgggtgtgg gattcagagt 300
ttcttcttct cggcattagg aggtgcagaa gcactacttt tggcatctat ggcctatgat 360
cgttacattg ctatttgctt tcctcttcac tatcccatcc gcatgagcaa aagaatgtgt 420
gtgctgatga taacagggtc ttggatcata ggctcgatca atgcttgtgc tcacactgta 480
tatgtactcc atattcctta ttgccaatcc agggccatca atcatttctt ctgtgatgtc 540
ccagcaatgg tgactctggc ctgcatggac acctgggtct atgagggcac agtgtttttg 600
agcaccacca tctttctcgt gtttcccttc attgctattt catgttccta tggccgggtt 660
ctccttgctg tctaccacat gaaatctgca gaagggagga agaaagccta cctgacctgc 720
agcacccacc tcactgtagt aactttctac tatgcacctt ttgtctacac ttatctacgt 780
ccaagatccc tgcgatctcc aacagaggac aaggttctgg ctgtcttcta caccatcctc 840
accccaatgc tcaaccccat catctatagc ctgaggaaca aggaggtgat gggggccctg 900
acacgagtga gtcagagaat ctgctctgtg aaaatgtag 939

<210> 20
<211> 960
<212> DNA
<213> Homo sapiens

```
<220>
<221> misc_feature
<223> Incyte ID No: 7477889CB1

<400> 20
atgacacagt tgacggccag tgggaatcag acaatggtga ctgagttcct cttctctatg 60
ttcccgcatg cgcacagagg tggcctctta ttctttattc ccttgcttct catctacgga 120
tttatcctaa ctggaaacct aataatgttc attgtcatcc aggtgggcat ggccctgcac 180
acccctttgt atttctttat cagtgtcctc tccttcctgg agatctgcta taccacaacc 240
accatcccca agatgctgtc ctgcctaatc agtgagcaga agagcatttc cgtggctggc 300
tgcctcctgc agatgtactt tttccactca cttggtatca cagaaagctg tgtcctgaca 360
gcaatggcca ttgacaggta catagctatc tgcaatccac tccgttaccc aaccatcatg 420
attcccaaac tttgtatcca gctgacagtt ggatcctgct tttgtggctt cctccttgtg 480
cttcctgaga ttgcatggat ttccaccttg cctttctgtg gctccaacca gatccaccag 540
atattctgtg atttcacacc tgtgctgagc ttggcctgca cagatacatt cctagtggtc 600
attgtggatg ccatccatgc agcggaaatt gtagcctcct tcctggtcat tgctctatcc 660
tacatccgga ttattatagt gattctggga atgcactcag ctgaaggtca tcacaaggcc 720
ttttccacct gtgctgctca ccttgctgtg ttcttgctat tttttggcag tgtggctgtc 780
atgtatttga gattctcagc cacctactca gtgttttggg acacagcaat tgctgtcact 840
tttgttatcc ttgctccctt tttcaacccc atcatctata gcctgaaaaa caaggacatg 900
aaagaggcta ttggaaggct tttccactat cagaagaggg ctggttgggc tgggaaatag 960
```